# EUROPÄISCHE PATENTSCHRIFT

(11) **EP 2 621 919 B1**
(45) Veröffentlichungstag und Bekanntmachung des Hinweises auf die Patenterteilung: **13.01.2016**
(21) Anmeldenummer: 11760695.4
(22) Anmeldetag: 31.08.2011
(51) Int. Cl.: C07D 401/14, C07D 405/14, C07D 403/06, C07D 403/14, A61K 31/517, A61K 31/675, A61K 45/06, C07F 9/6558

(54) **PHENYLCHINAZOLINDERIVATE**
PHENYLQUINAZOLINE DERIVATIVES
DÉRIVÉS DE PHÉNYLQUINAZOLINE

(30) Priorität: 29.09.2010 DE 102010046837
(43) Veröffentlichungstag der Anmeldung: 07.08.2013
(73) Patentinhaber: Merck Patent GmbH, 64293 Darmstadt (DE)
(72) Erfinder: EGGENWEILER, Hans-Michael, 64291 Darmstadt (DE); SIRRENBERG, Christian, 64285 Darmstadt (DE); BUCHSTALLER, Hans-Peter, 64347 Griesheim (DE)
(86) Internationale Anmeldenummer: PCT/EP2011/004397
(87) Internationale Veröffentlichungsnummer: WO 2012/041435

(56) Entgegenhaltungen:
- WO-A1-2010/066324
- WO-A2-2009/010139

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation von HSP90 eine Rolle spielt, ferner pharmazeutische Zusammensetzungen, die diese Verbindungen enthalten, sowie die Verwendung der Verbindungen zur Behandlung von Krankheiten, bei denen HSP90 eine Rolle spielt.

Die korrekte Faltung und Konformation von Proteinen in Zellen wird durch molekulare Chaperone gewährleistet und ist kritisch für die Regulation des Gleichgewichts zwischen Protein Synthese und Degradation. Chaperone sind wichtig für die Regulation vieler zentraler Funktionen von Zellen wie z.B. Zellproliferation und Apoptose (Jolly and Morimoto, 2000; Smith et al., 1998; Smith, 2001).

### Hitzeschock-Proteine (heat shock proteins, HSPs)

Die Zellen eines Gewebes reagieren auf äußerlichen Stress wie z.B. Hitze, Hypoxie, oxidativem Stress, oder Giftstoffen wie Schwermetallen oder Alkoholen mit der Aktivierung einer Reihe von Chaperonen, welche unter der Bezeichnung "heat shock proteins" (HSPs) bekannt sind.

Die Aktivierung von HSPs schützt die Zelle gegen Verletzungen, die durch solche Stressfaktoren ausgelöst werden, beschleunigt die Wiederherstellung des physiologischen Zustands und führt zu einem stresstoleranten Zustand der Zelle.

Neben diesem ursprünglich entdeckten durch HSPs vermittelten Schutzmechanismus bei äußerlichem Stress wurden im Laufe der Zeit weitere wichtige Chaperon-Funktionen für einzelne HSPs auch unter normalen stressfreien Bedingungen beschrieben. So regulieren verschiedene HSPs beispielsweise die korrekte Faltung, die intrazelluläre Lokalisierung und Funktion oder den geregelten Abbau einer Reihe biologisch wichtiger Proteine von Zellen.

HSPs bilden eine Genfamilie mit individuellen Genprodukten, deren Zellulärexpression, Funktion und Lokalisierung in verschiedenen Zellen sich unterscheidet. Die Benennung und Einteilung innerhalb der Familie erfolgt aufgrund ihres Molekulargewichts z.B. HSP27, HSP70, and HSP90.

Einigen menschlichen Krankheiten liegt eine falsche Proteinfaltung zugrunde (siehe Review z.B. Tytell et al., 2001; Smith et al., 1998). Die Entwicklung von Therapien, welche in den Mechanismus der Chaperon abhängigen Proteinfaltung eingreift, könnte daher in solchen Fällen nützlich sein. Beispielsweise führen bei der Alzheimer-Erkrankung, Prionenerkrankungen oder dem Huntington Syndrom falsch gefaltete Proteine zu einer Aggregation von Protein mit neurodegenerativem Verlauf. Durch falsche Proteinfaltung kann auch ein Verlust der Wildtyp-Funktion entstehen, der eine fehlregulierte molekulare und physiologische Funktion zur Folge haben kann.

HSPs wird auch eine grosse Bedeutung bei Tumorerkrankungen beigemessen. Es gibt z.B. Hinweise, dass die Expression bestimmter HSPs im Zusammenhang mit dem Stadium der Progression von Tumoren steht (Martin et al., 2000; Conroy et al., 1996; Kawanishi et al., 1999;
Jameel et al., 1992; Hoang et al., 2000; Lebeau et al., 1991).

Die Tatsache, dass HSP90 bei mehreren zentralen onkogenen Signalwegen in der Zelle eine Rolle spielt und gewisse Naturstoffe mit krebshemmender Aktivität HSP90 targetieren, führte zu dem Konzept, dass eine Hemmung der Funktion von HSP90 bei der Behandlung von Tumorerkrankungen sinnvoll wäre.

Ein HSP90 Inhibitor, 17-Allylamino-17-demethoxygeldanamycin (17AAG), ein Derivat von Geldanamycin, befindet sich gegenwärtig in klinischer Prüfung.

### HSP90

HSP90 repräsentiert ungefähr 1-2% der gesamten zellulären Proteinmasse. Es liegt in der Zelle gewöhnlich als Dimer vor und ist mit einer Vielzahl von Proteinen, sogenannten Co-chaperonen assoziiert (siehe z.B. Pratt, 1997). HSP90 ist essentiell für die Vitalität von Zellen (Young et al., 2001) und spielt eine Schlüsselrolle in der Antwort auf zellulären Stress durch Interaktion mit vielen Proteinen, deren native Faltung durch äußerlichen Stress, wie z.B. Hitzeschock, verändert wurde, um die ursprüngliche Faltung wiederherzustellen oder die Aggregation der Proteine zu verhindern (Smith et al., 1998).

Es gibt auch Hinweise, dass HSP90 als Puffer gegen die Auswirkungen von Mutationen eine Bedeutung hat, vermutlich durch die Korrektur falscher Proteinfaltung, die durch die Mutation hervorgerufen wurde (Rutherford and Lindquist, 1998).

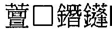 ber hinaus hat HSP90 auch eine regulatorische Bedeutung. Unter physiologischen Bedingungen spielt HSP90, zusammen mit seinem Homolog im Endoplasmatischen Retikulum, GRP94, eine Rolle im Zellhaushalt, um die Stabilität der Konformation und Reifung verschiedener "client" Schlüsselproteine zu gewährleisten. Diese können in drei Gruppen unterteilt werden: Rezeptoren für Steroidhormone, Ser/Thr or Tyrosinkinasen (z.B. ERBB2, RAF-1, CDK4 und LCK) und einer Sammlung unterschiedlicher Proteine wie z.B. mutiertes p53 oder die katalytische Untereinheit der Telomerase hTERT. Jedes dieser Proteine nimmt eine Schlüsselrolle in der Regulation physiologischer und biochemischer Prozesse von Zellen ein.

Die konservierte HSP90-Familie des Menschen besteht aus vier Genen, dem zytosolischen HSP90α, der induzierbaren HSP90β Isoform (Hickey et al., 1989), dem GRP94 im Endoplasmatischen Retikulum (Argon et al., 1999) und dem HSP75/TRAP1 in der mitochondrialen Matrix (Felts et al., 2000). Es wird angenommen, dass alle Mitglieder der Familie eine ähnliche Wirkweise haben, aber, je nach ihrer Lokalisierung in der Zelle, an unterschiedliche "client" Proteine binden. Beispielsweise ist ERBB2 ein spezifisches "client" Protein von GRP94 (Argon et al., 1999), während der Typ1 Rezeptor des Tumornekrosefaktors (TNFR1) oder das Retinoblastom Protein (Rb) als "clients" von TRAP1 nachgewiesen wurden (Song et al., 1995; Chen et al., 1996).

HSP90 ist an einer Reihe von komplexen Interaktionen mit einer grossen Zahl von "client" Proteinen und regulatorischen Proteinen beteiligt (Smith, 2001). Obwohl präzise molekulare Details noch nicht geklärt sind, haben biochemische Experimente und Untersuchungen mit Hilfe der Röntgenkristallographie in den letzten Jahren zunehmend Details der Chaperonfunktion von HSP90 entschlüsseln können (Prodromou et al., 1997; Stebbins et al., 1997). Danach ist HSP90 ein ATP-abhängiges molekulares Chaperon (Prodromou et al, 1997), wobei die Dimerisierung wichtig für die ATP Hydrolyse ist. Die Bindung von ATP resultiert in der Formation einer toroidalen Dimerstruktur, bei der die beiden N-terminalen Domainen in engem Kontakt zueinander kommen und einen "switch" in der Konformation bewirken. (Prodromou and Pearl, 2000).

### Bekannte HSP90 Inhibitoren

Die erste Klasse von HSP90 Inhibitoren, die entdeckt wurde, waren Benzochinon-Ansamycine mit den Verbindungen Herbimycin A und Geldanamycin. Ursprünglich wurde mit ihnen die Reversion des malignen Phänotyps bei Fibroblasten nachgewiesen, die durch Transformation mit dem v-Src Onkogen induziert worden war (Uehara et al., 1985).

Später wurde eine starke antitumorale Aktivität in vitro (Schulte et al., 1998) und in vivo in Tiermodellen gezeigt (Supko et al., 1995).

Immunpräzipitation und Untersuchungen an Affinitätsmatrices zeigten dann, dass der Hauptwirkmechanismus von Geldanamycin eine Bindung an HSP90 involviert (Whitesell et al., 1994; Schulte and Neckers, 1998). Darüber hinaus wurde durch röntgenkristallographische Untersuchungen gezeigt, dass Geldanamycin um die ATP-Bindestelle kompetitiert und die intrinsische ATPase Aktivität von HSP90 hemmt (Prodromou et al., 1997; Panaretou et al., 1998). Dadurch wird die Entstehung des multimeren HSP90 Komplexes, mit seiner Eigenschaft als Chaperon für "client" Proteine zu fungieren, verhindert. Als Konsequenz werden "client" Proteine über den Ubiquitin-Proteasom-Weg abgebaut.

Das Geldanamycin Derivat 17- Allylamino-17-demethoxygeldanamycin (17AAG) zeigte unveränderte Eigenschaft bei der Hemmung von HSP90, der Degradation von "client" Proteinen und antitumoraler Aktivität in Zellkulturen und in Xenograft Tumormodellen (Schulte et al, 1998; Kelland et al, 1999), hatte aber eine deutlich geringere Leberzytotoxizität als Geldanamycin (Page et all 1997). 17AAG wird gegenwärtig in PhaseI/II klinischen Studien geprüft.

Radicicol, ein makrozyklisches Antibiotikum, zeigte ebenfalls Revision des v-Src und v-Ha-Ras induzierten malignen Phänotyps von Fibroblasten (Kwon et all 1992; Zhao et al, 1995). Radicicol degradiert eine Vielzahl von Signalproteinen als Konsequenz der HSP90 Hemmung (Schulte et al., 1998). Röntgenkristallographische Untersuchungen zeigten, dass Radicicol ebenfalls an die N-terminale Domäne von HSP90 bindet und die intrinsische ATPase Aktivität hemmt (Roe et al., 1998).

Antibiotika vom Coumarin Typ binden bekannterweise an die ATP Bindestelle des HSP90 Homologs DNA Gyrase in Bakterien. Das Coumarin, Novobiocin, bindet an das Carboxy-terminale Ende von HSP90, also an eine andere Stelle bei HSP90 als die Benzochinon-Ansamycine und Radicicol, welche an das N-terminale Ende von HSP90 binden.(Marcu et al., 2000b).

Die Hemmung von HSP90 durch Novobiocin resultiert in der Degradation einer großen Zahl von HSP90-abhängigen Signalproteinen (Marcu et al., 2000a).

Mit PU3, einem von Purinen abgeleiteten HSP90 Inhibitor konnte die Degradation von Signalproteinen z.B. ERBB2, gezeigt werden. PU3 verursacht Zellzyklus-Arrest und Differenzierung in Brustkrebs-Zelllinien (Chiosis et al., 2001).

### HSP90 als therapeutisches Target

Durch die Beteiligung von HSP90 an der Regulation einer großen Zahl von Signalwegen, die entscheidende Bedeutung am Phänotyp eines Tumors haben, und der Entdeckung, dass gewisse Naturstoffe ihren biologischen Effekt durch Hemmung der Aktivität von HSP90 ausüben, wird HSP90 gegenwärtig als neues Target für die Entwicklung eines Tumortherapeutikum geprüft (Neckers et al., 1999).

Der Hauptmechanismus der Wirkweise von Geldanamycin, 17AAG, und Radicicol beinhaltet die Hemmung der Bindung von ATP an die ATP-Bindestelle am N-terminalen Ende des Proteins und die daraus resultierende Hemmung der intrinsischen ATPase-Aktivität von HSP90 (siehe z.B. Prodromou et al., 1997; Stebbins et al., 1997; Panaretou et al., 1998). Die Hemmung der ATPase-Aktivität von HSP90 verhindert die Rekrutierung von Co-chaperonen und favorisiert die Bildung eines HSP90 Heterokomplexes, der "client" Proteine über den Ubiquitin-Proteasom-Weg der Degradation zuführt (siehe, z.B. Neckers et al., 1999; Kelland et al., 1999). Die Behandlung von Tumorzellen mit HSP90 Inhibitoren führt zur selektiven Degradation wichtiger Proteine mit fundamentaler Bedeutung für Prozesse wie Zellproliferation, Regulation des Zellzyklus und Apoptose. Diese Prozesse sind häufig in Tumoren dereguliert (siehe z.B. Hostein et al., 2001).

Eine attraktive Rationale für die Entwicklung eines Inhibitors von HSP90 ist, dass durch gleichzeitige Degradation mehrerer Proteine, die mit dem transformierten Phänotyp im Zusammenhang stehen, eine starke tumortherapeutische Wirkung erreicht werden kann.

Im einzelnen betrifft die vorliegende Erfindung Verbindungen, die HSP90 hemmen, regulieren und/oder modulieren, Zusammensetzungen, die diese Verbindungen enthalten, sowie Verfahren zu ihrer Verwendung zur Behandlung von HSP90-bedingten Krankheiten, wie Tumorerkrankungen, virale Erkrankungen wie z.B. Hepatitis B (Waxman, 2002);

Immunsuppression bei Transplantationen (Bijlmakers, 2000 and Yorgin, 2000); Entzündungsbedingte Erkrankungen (Bucci, 2000) wie Rheumatoide Arthritis, Asthma, Multiple Sklerose, Typ 1 Diabetes, Lupus Erythematodes, Psoriasis und Inflammatory Bowel Disease; Zystische Fibrose (Fuller, 2000); Erkrankungen im Zusammenhang mit Angiogenese (Hur, 2002 and Kurebayashi, 2001) wie z.B. diabetische Retinopathie, Hämangiome, Endometriose und Tumorangiogenese; infektiöse Erkrankungen; Autoimmunerkrankungen; Ischämie; Förderung der Nervenregeneration (Rosen et al., WO 02/09696; Degranco et al., WO 99/51223; Gold, US 6,210,974 B1); fibrogenetische Erkrankungen, wie z.B. Sklerodermie, Polymyositis, systemischer Lupus, Leberzirrhose, Keloidbildung, interstitielle Nephritis und pulmonare Fibrose (Strehlow, WO 02/02123).

Die Erfindung betrifft auch die Verwendung der erfindungsgemäßen Verbindungen zum Schutz normaler Zellen gegen Toxizität, die durch Chemotherapie verursacht ist, sowie die Verwendung bei Krankheiten, wobei Proteinfehlfaltung oder Aggregation ein Hauptkausalfaktor ist, wie z.B. Skrapie, Creutzfeldt-Jakob-Krankheit, Huntington oder Alzheimer (Sittler, Hum. Mol. Genet., 10, 1307, 2001; Tratzelt et al., Proc. Nat. Acad. Sci., 92, 2944, 1995; Winklhofer et al., J. Biol. Chem., 276, 45160, 2001).

In der WO 01/72779 sind Purinverbindungen beschrieben, sowie deren Verwendung zur Behandlung von GRP94 (Homolog oder Paralog zu HSP90)-bedingten Krankheiten, wie Tumorerkrankungen, wobei das Krebsgewebe ein Sarkom oder Karzinom umfasst, ausgewählt aus der Gruppe, bestehend aus Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung.

In der WO 01/72779 ist weiterhin die Verwendung der dort genannten Verbindungen zur Behandlung von viralen Erkrankungen offenbart, wobei das virale Pathogen ausgewählt ist aus der Gruppe, bestehend aus Hepatitis Typ A, Hepatitis Typ B, Hepatitis Typ C, Influenza, Varicella, Adenovirus, Herpes-Simplex Typ I (HSV-I), Herpes Simplex Typ II (HSV-II), Rinderpest, Rhinovirus, Echovirus, Rotavirus, respiratorischem Synzytialvirus (RSV), Papillomvirus, Papovavirus, Cytomegalievirus, Echinovirus, Arbovirus, Huntavirus, Coxsackievirus, Mumpsvirus, Masernvirus, Rötelnvirus, Poliovirus, menschliches Immunschwächevirus Typ I (HIV-I) und menschliches Immunschwächevirus Typ II (HIV-II).

In der WO 01/72779 ist ferner die Verwendung der dort genannten Verbindungen zur GRP94-Modulation beschrieben, wobei die modulierte biologische GRP94-Aktivität eine Immunreaktion in einem Individuum, Proteintransport vom endoplasmatischen Retikulum, Genesung vom hypoxischen/anoxischen Stress, Genesung von Unterernährung, Genesung von Hitzestress, oder Kombinationen davon, hervorruft, und/oder wobei die Störung eine Art Krebs ist, eine Infektionserkrankung, eine Störung, die mit einem gestörten Proteintransport vom endoplasmatischen Retikulum, einer Störung, die mit Ischämie / Reperfusion einhergeht, oder Kombinationen davon, wobei die die mit Ischämie / Reperfusion einhergehende Störung eine Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist.

In der WO 01/72779 ist schließlich die Verwendung einer wirksamen Menge eines GRP94-Proteinmodulators zur Herstellung eines Medikamentes bechrieben, zum Verändern einer anschließenden zellulären Reaktion auf einen ischämischen Zustand bei einer Gewebestelle in einem Individuum, durch Behandlung der Zellen an der Gewebestelle mit dem GRP94-Protein-modulator, damit die GRP94-Aktivität in Zellen dermaßen verstärkt wird, dass eine anschließende zelluläre Reaktion auf einen ischämischen Zustand verändert wird, wobei die anschließende ischämische Bedingung vorzugsweise die Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist, oder wobei die Gewebestelle das Donatorgewebe für eine Transplantation ist.

A. Kamal et al. beschreiben in Trends in Molecular Medicine, Vol. 10 No. 6 June 2004, therapeutische und diagnostische Anwendungen der HSP90 Aktivierung, u.a. zur Behandlung von Krankheiten des Zentralnervensystems und von Herzkreislauferkrankungen.

Die Identifikation von kleinen Verbindungen, die HSP90 spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Insbesondere zeigen sie inhibierende Eigenschaften des HSP90.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

### STAND DER TECHNIK

Andere Chinazolinderivate sind als HSP90 Inhibitoren in EP 2 164 833 und in der WO 2010/066324 beschrieben.

In der WO 00/53169 wird die HSP90-Inhibierung mit Coumarin oder einem Coumarinderivat beschrieben.

In der WO 03/041643 A2 sind HSP90-inhibierende Zearalanol-Derivate offenbart.

HSP90-inhibierende Indazolderivate kennt man aus WO 06/010595 und WO 02/083648.

### Weitere Literatur:

Argon Y and Simen BB. 1999 "Grp94, an ER chaperone with protein and peptide binding properties", Semin. Cell Dev. Biol., Vol. 10, pp. 495-505.

Bijlmakers M-JJE, Marsh M. 2000 "Hsp90 is essential for the synthesis and subsequent membrane association, but not the maintenance, of the Srckinase p56Ick", Mol. Biol. Cell, Vol. 11(5), pp. 1585-1595.

Bucci M; Roviezzo F; Cicala C; Sessa WC, Cirino G. 2000 "Geldanamycin, an inhibitor of heat shock protein 90 (Hsp90) mediated signal transduction has anti-inflammatory effects and interacts with glucocorticoid receptor in vivo", Brit. J. Pharmacol., Vol 131(1), pp. 13-16.

Carreras CW, Schirmer A, Zhong Z, Santi VS. 2003 "Filter binding assay for the geldanamycin-heat shock protein 90 interaction", Analytical Biochem., Vol 317, pp 40-46.

Chen C-F, Chen Y, Dai KD, Chen P-L, Riley DJ and Lee W-H. 1996 "A new member of the hsp90 family of molecular chaperones interacts with the retinoblastoma protein during mitosis and after heat shock", Mol. Cell. Biol., Vol. 16, pp. 4691-4699.

Chiosis G, Timaul MN, Lucas B, Munster PN, Zheng FF, Sepp-Lozenzino L and Rosen N. 2001 "A small molecule designed to bind to the adenine nucleotide pocket of HSP90 causes Her2 degradation and the growth arrest and differentiation of breast cancer cells", Chem. Biol., Vol. 8, pp. 289-299.

Chiosis G, Lucas B, Shtil A, Huezo H, Rosen N 2002 "Development of a purine-scaffold novel class of HSP90 binders that inhibit the proliferation of cancer cells and induce the degradation of her2 tyrosine kinase". Bioorganic Med. Chem., Vol 10, pp 3555-3564.

Conroy SE and Latchman DS. 1996 "Do heat shock proteins have a role in breast cancer?", Brit. J. Cancer, Vol. 74, pp. 717-721.

Felts SJ, Owen BAL, Nguyen P, Trepel J, Donner DB and Toft DO. 2000 "The HSP90-related protein TRAP1 is a mitochondrial protein with distinct functional properties", J. Biol. Chem., Vol. 5, pp. 3305-331 2.

Fuller W, Cuthbert AW. 2000 "Post-translational disruption of the delta F508 cystic fibrosis transmembrane conductance regulator (CFTR)-molecular Chaperone complex with geldanamycin stabilizes delta F508 CFTR in the rabbit reticulocyte lysate", J. Biol. Chem., Vol. 275(48), pp. 37462-37468.

Hickey E, Brandon SE, Smale G, Lloyd D and Weber LA. 1999 "Sequence and regulation of a gene encoding a human 89-kilodalton heat shock protein", Mol. Cell. Biol., Vol. 9, pp. 2615-2626.

Hoang AT, Huang J, Rudra-Gonguly N, Zheng J, Powell WC, Rabindron SK, Wu C and Roy-Burman P. 2000 "A novel association between the human heat shock transcription factor 1 (HSF1) and prostate adenocarcinoma, Am. J. Pathol., Vol. 156, pp. 857-864.

Hostein I, Robertson D, Di Stefano F, Workman P and Clarke PA. 2001 "Inhibition of signal transduction by the HSP90 inhibitor 17-allylamino-1 7-demethoxygeldanamycin results in cytostasis and apoptosis", Cancer Res., Vol. 61, pp. 4003-4009.

Hur E, Kim H-H, Choi SM, Kim JH, Yim S, Kwon HJ, Choi Y, Kim DK, Lee M-0, Park H. 2002 "Reduction of hypoxia-induced transcription through the repression of hypoxia-inducible factor-1α/aryl hydrocarbon receptor nuclear translocator DNA binding by the 90-kDa heat-shock protein inhibitor radicicol", Mol. Pharmacol., Vol 62(5), pp. 975-982.

Jameel A, Skilton RA, Campbell TA, Chander SK, Coombes RC and Luqmani YA. 1992 "Clinical Jolly C and Morimoto RI. 2000 "Role of the heat shock response and molecular chaperones in oncogenesis and cell death", J. Natl. Cancer Inst., Vol. 92, pp. 1564-1572.

Kawanishi K, Shiozaki H, Doki Y, Sakita I, Inoue M, Yano M, Tsujinata T, Shamma A and Monden M. 1999 "Prognostic significance of heat shock proteins 27 and 70 in patients with squamous cell carcinoma of the esophagus", Cancer, Vol. 85, pp. 1649-1657.

Kelland LR, Abel G, McKeage MJ, Jones M, Goddard PM, Valenti M, Murrer BA, and Harrap KR. 1993 "Preclinical antitumour evaluation of bis-acetalo-amino-dichloro-cyclohexylamine platinum (IV): an orally active platinum drug", Cancer Research, Vol. 53, pp. 2581 - 2586.

Kelland LR, Sharp SY, Rogers PM, Myers TG and Workman P. 1999 "DTdiaphorase expression and tumor cell sensitivity to 17-allylamino,17-demethoxygeldanamycin, an inhibitor of heat shock protein 90", J. Natl. Cancer Inst., Vol. 91, pp. 1940-1949.

Kurebayashi J, Otsuki T, Kurosumi M, Soga S, Akinaga S, Sonoo, H. 2001 "A radicicol derivative, KF58333, inhibits expression of hypoxia-inducible factor-1α and vascular endothelial growth factor, angiogenesis and growth of human breast cancer xenografts", Jap. J. Cancer Res.,Vol. 92(12), 1342-1351.

Kwon HJ, Yoshida M, Abe K, Horinouchi S and Bepple T. 1992 "Radicicol, an agent inducing the reversal of transformed phentoype of src-transformed fibroblasts, Biosci., Biotechnol., Biochem., Vol. 56, pp. 538-539.

Lebeau J, Le Cholony C, Prosperi MT and Goubin G. 1991 "Constitutive overexpression of 89 kDa heat shock protein gene in the HBL100 mammary cell line converted to a tumorigenic phenotype by the EJE24 Harvey-ras oncogene", Oncogene, Vol. 6, pp. 1125-1132.

Marcu MG, Chadli A, Bouhouche I, Catelli M and Neckers L. 2000a "The heat shock protein 90 antagonist novobiocin interacts with a previously unrecognized ATP-binding domain in the carboxyl terminus of the chaperone", J. Biol. Chem., Vol. 275, pp. 37181-37186.

Marcu MG, Schulte TW and Neckers L. 2000b "Novobiocin and related coumarins and depletion of heat shock protein 90-dependent signaling proteins", J. Natl. Cancer Inst., Vol. 92, pp. 242-248.

Martin KJ, Kritzman BM, Price LM, Koh B, Kwan CP, Zhang X, MacKay A, O'Hare MJ, Kaelin CM, Mutter GL, Pardee AB and Sager R. 2000 "Linking gene expression patterns to therapeutic groups in breast cancer", Cancer Res., Vol. 60, pp. 2232-2238.

Neckers L, Schulte TW and Momnaaugh E. 1999 "Geldanamycin as a potential anti-cancer agent: its molecular target and biochemical activity", Invest. New Druqs, Vol. 17, pp. 361-373.

Page J, Heath J, Fulton R, Yalkowsky E, Tabibi E, Tomaszewski J, Smith A and Rodman L. 1997 "Comparison of geldanamycin (NSC-122750) and 17-allylaminogeldanamycin (NSC-330507D) toxicity in rats", Proc. Am. Assoc. Cancer Res., Vol. 38, pp. 308.

Panaretou B, Prodromou C, Roe SM, OBrien R, Ladbury JE, Piper PW and Pearl LH. 1998 "ATP binding and hydrolysis are essential to the function of the HSP90 molecular chaperone in vivo", EMBO J., Vol. 17, pp. 4829-4836.

Pratt WB. 1997 "The role of the HSP90-based chaperone system in signal transduction by nuclear receptors and receptors signalling via MAP kinase", Annu. Rev. Pharmacol. Toxicol., Vol. 37, pp. 297-326.

Prodromou C, Roe SM, O'Brien R, Ladbury JE, Piper PWand Pearl LH. 1997 "Identification and structural characterization of the ATP/ADP-binding site in the HSP90 molecular chaperone", Cell, Vol. 90, pp. 65-75.

Prodromou C, Panaretou B, Chohan S, Siligardi G, O'Brien R, Ladbury JE, Roe SM, Piper PW and Pearl LH. 2000 "The ATPase cycle of HSP90 drives a molecular "clamp" via transient dimerization of the N-terminal domains", EMBO J., Vol. 19, pp. 4383-4392.

Roe SM, Prodromou C, O'Brien R, Ladbury JE, Piper PW and Pearl LH. 1999 "Structural basis for inhibition of the HSP90 molecular chaperone by the antitumour antibiotics radicicol and geldanamycin", J. Med. Chem., Vol. 42, pp. 260-266.

Rutherford SL and Lindquist S. 1998 "HSP90 as a capacitor for morphological evolution. Nature, Vol. 396, pp. 336-342.

Schulte TW, Akinaga S, Murakata T, Agatsuma T, Sugimoto S, Nakano H, Lee YS, Simen BB, Argon Y, Felts S, Toft DO, Neckers LM and Sharma SV. 1999 "Interaction of radicicol with members of the heat shock protein 90 family of molecular chaperones", Mol. Endocrinoloqy, Vol. 13, pp. 1435-1448. Schulte TW, Akinaga S, Soga S, Sullivan W, Sensgard B, Toft D and Neckers LM. 1998 "Antibiotic radicicol binds to the N-terminal domain of HSP90 and shares important biologic activities with geldanamcyin", Cell Stress and Chaperones, Vol. 3, pp. 100-108.

Schulte TW and Neckers LM. 1998 "The benzoquinone ansamycin 17-allylamino-17-demethoxygeldanamcyin binds to HSP90 and shares important biologic activities with geldanamycin", Cancer Chemother. Pharmacol., Vol. 42, pp. 273-279.

Smith DF. 2001 "Chaperones in signal transduction", in: Molecular chaperones in the cell (P Lund, ed.; Oxford University Press, Oxford and NY), pp. 165-178.

Smith DF, Whitesell L and Katsanis E. 1998 "Molecular chaperones: Biology and prospects for pharmacological intervention", Pharmacological Reviews, Vol. 50, pp. 493-513.

Song HY, Dunbar JD, Zhang YX, Guo D and Donner DB. 1995 "Identification of a protein with homology to hsp90 that binds the type 1 tumour necrosis factor receptor", J. Biol. Chem., Vol. 270, pp. 3574-3581.

Stebbins CE, Russo A, Schneider C, Rosen N, Hartl FU and Pavletich NP. 1997 "Crystal structure of an HSP90-geldanamcyin complex: targeting of a protein chaperone by an antitumor agent", Cell, Vol. 89, pp. 239-250.

Supko JG, Hickman RL, Grever MR and Malspeis L. 1995 "Preclinical pharmacologic evaluation of geldanamycin as an antitumour agent", Cancer Chemother. Pharmacol., Vol. 36, pp. 305-315.

Tytell M and Hooper PL. 2001 "Heat shock proteins: new keys to the development of cytoprotective therapies", Emerging Therapeutic Tarqets, Vol. 5, pp. 267-287.

Uehara U, Hori M, Takeuchi T and Umezawa H. 1986 "Phenotypic change from transformed to normal induced by benzoquinoid ansamycins accompanies inactivation of p6Osrc in rat kidney cells infected with Rous sarcoma virus", Mol. Cell. Biol., Vol. 6, pp. 21 98-2206.

Waxman, Lloyd H. Inhibiting hepatitis C virus processing and replication. (Merck & Co., Inc., USA). PCT Int. Appl. (2002), WO 0207761 Whitesell L, Mimnaugh EG, De Costa B, Myers CE and Neckers LM. 1994 "Inhibition of heat shock protein HSP90-pp60v-src heteroprotein complex formation by benzoquinone ansamycins: essential role for stress proteins in oncogenic transformation", Proc. Natl. Acad. Sci. USA., Vol. 91, pp. 8324-8328.

Yorgin et al. 2000 "Effects of geldanamycin, a heat-shock protein 90-binding agent, on T cell function and T cell nonreceptor protein tyrosine kinases", J. Immunol., Vol 164(6), pp. 2915-2923.

Young JC, Moarefi I and Hartl FU. 2001 "HSP90: a specialized but essential protein-folding tool", J. Cell. Biol., Vol. 154, pp. 267-273.

Zhao JF, Nakano H and Sharma S. 1995 "Suppression of RAS and MOS transformation by radicicol", Oncoqene, Vol. 11, pp. 161 -173.

### ZUSAMMENFASSUNG DER ERFINDUNG

Die Erfindung betrifft einzelne Verbindungen gemäß Anspruch 1 umfaßt von der Formel I 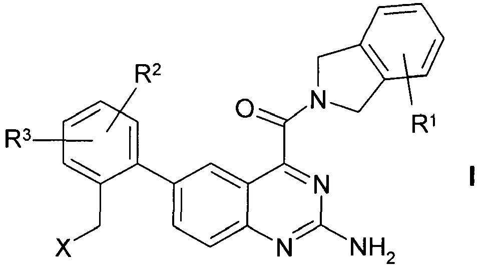 worin
- R¹: H, Hal oder A",
- R², R³: jeweils unabhängig voneinander H, Hal, OH oder OA,
- R² und R³: zusammen auch OCH₂O,
- X: OR⁴, O(CO)R⁴, O(CO)OR⁴, O(CO)NR⁴R⁵, ONR⁴R⁵, OP(=O)(OH)₂, OP(=O)(OA")₂, NR⁴OR⁵, NR⁴R⁵, NR⁴(CO)R⁵, NR⁴(CO)NR⁵ oder NR⁴(CO)OR⁵,
- R⁴, R⁵, R⁶: jeweils unabhängig voneinander H, A, Y-Het oder Y-Ar,
- R⁴ und R⁵ oder R⁵ und R⁶: zusammen mit dem Heteroatom an das sie gebunden sind, auch einen unsubstituierten oder ein-, zwei- oder dreifach durch Hal, A, (CH₂)ₙOH, (CH₂)ₙOA, (CH₂)ₙNH₂, (CH₂)ₙCOOH, (CH₂)ₙCOOA, NHCOA, NA'COA, CONH₂, CONHA, CONAA', OC(=O)(CH₂)ₙNH₂ und/oder =O (Carbonylsauerstoff) substituierten gesättigten, ungesättigten oder aromatischen mono- oder bicyclischen Heterocyclus, der weitere 1 bis 3 N-, O- und/oder S-Atome enthalten kann, und worin auch ein N-Atom oxidiert sein kann,
- Ar: unsubstituiertes oder ein-, zwei-, drei-, vier- oder fünffach durch A, Hal, (CH₂)ₙOA, (CH₂)ₙOH, (CH₂)ₙCN, SA, SOA, SO₂A, NO₂, C≡CH, (CH₂)ₙCOOH, CHO, (CH₂)ₙCOOA, CONH₂, CONHA, CONAA', NHCOA, CH(OH)A, (CH₂)ₙNH₂, (CH₂)ₙNHA, (CH₂)ₙNAA', (CH₂)ₙNHSO₂A, SO₂NH(CH₂)ₙNH₂, SO₂NH₂, SO₂NHA, SO₂NAA', CONH(CH₂)ₙCOOA, CONH(CH₂)ₙCOOH, NHCO(CH₂)ₙCOOA, NHCO(CH₂)ₙCOOH, CONH(CH₂)ₙNH₂, CONH(CH₂)ₙNHA, CONH(CH₂)ₙNAA', CONH(CH₂)ₙCN und/oder (CH₂)ₙCH(NH₂)COOH, substituiertes Phenyl, Naphthyl, Tetrahydronaphthyl oder Biphenyl,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch A, OA, OH, Phenyl, SH, S(O)ₘA, Hal, NO₂, CN, COA, COOA, COOBenzyl, CONH₂, CONHA, CONAA', SO₂NH₂, NH₂, NHA, NAA', NHCOA, NHSO₂A und/oder =O (Carbonylsauerstoff) substituiert sein kann,
- A, A': jeweils unabhängig voneinander unverzweigtes oder verzweigtes Alkyl mit 1-16 C-Atomen, worin 1-6 nicht-benachbarte CH₂-Gruppen durch O, NH, NMe, NEt, NZ, S, SO, SO₂, NHCO, CONH, NHCOO, NA'COO, NHSO₂, SO₂NH und/oder CH-Gruppen durch N und/oder auch 1-7 H-Atome durch F, Cl, OH, OZ, NH₂, SH und/oder SZ ersetzt sein können, oder cyclisches Alkyl mit 3-8 C-Atomen,
- A": unverzweigtes oder verzweigtes Alkyl mit 1-6 C-Atomen,
- Y: unverzweigtes oder verzweigtes Alkylen mit 1-10 C-Atomen, worin 1-3 nicht-benachbarte CH₂-Gruppen durch O, NH, NMe, NEt, S, SO, SO₂ und/oder CH-Gruppen durch N und/oder auch 1-5 H-Atome durch F, Cl, OH und/oder NH₂ ersetzt sein können,
- W, W': unverzweigtes oder verzweigtes Alkyl mit 1-10 C-Atomen, worin 1-3 nicht-benachbarte CH₂-Gruppen durch O, NH, NMe, NEt, S, SO, SO₂ und/oder CH-Gruppen durch N und/oder auch 1-5 H-Atome durch Ar, Het, F, Cl, OH und/oder NH₂ ersetzt sein können,
oder cyclisches Alkyl mit 3-8 C-Atomen,
- Z: -COW, -COOW, -CONWW', -SOW oder -SO₂W,
- Hal: F, Cl, Br oder I,
- m: 0, 1 oder 2,
- n: 0, 1, 2, 3 oder 4,
bedeuten,
sowie ihre pharmazeutisch verwendbaren Salze, Tautomere und Stereoisomere, einschließlich deren Mischungen in allen Verhältnissen.

Der Anmeldungsgegenstand bezieht sich auf die Definition der Ansprüche; jede Offenbarung, die über den Umfang der Ansprüche hinausgeht, dient nur zu Informationszwecken.

Gegenstand der Erfindung sind die Verbindungen der Formel I gemäß Anspruch 1 und ihre Salze sowie ein Verfahren zur Herstellung von Verbindungen der Formel I sowie ihrer pharmazeutisch verwendbaren Salze, Tautomere und Stereoisomere, dadurch gekennzeichnet, daß man
a)
   eine Verbindung der Formel II 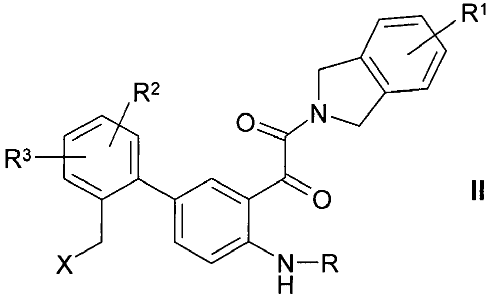 worin
   R¹, R², R³ und X die in Anspruch 1 angegebenen Bedeutungen haben,
   R eine Aminoschutzgruppe,
   bedeutet und
   A" die in Anspruch 1 angegebene Bedeutung hat,
   mit einer Verbindung der Formel III

   Y₃Si-N=C=N-SiY₃ **III**

   worin
   Y Alkyl mit 1-4 C-Atomen bedeutet,
   umsetzt,
   oder
b)
   eine Verbindung der Formel IV 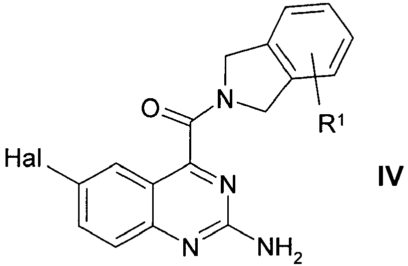 worin R¹ die in Anspruch 1 angegebene Bedeutung hat, und Hal Brom oder Iod bedeutet,
   mit einer Verbindung der Formel V 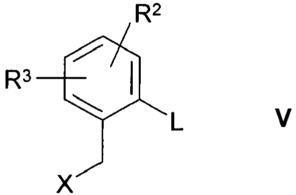 worin X, R² und R³ die in Anspruch 1 angegebenen Bedeutungen haben,
   und L einen Boronsäure- oder Boronsäureesterrest bedeutet,
umsetzt,
und/oder eine Base oder Säure der Formel **I** in eines ihrer Salze umwandelt.

Unter Verbindungen der Formel I versteht man auch die Hydrate und Solvate dieser Verbindungen, ferner pharmazeutisch verwendbare Derivate. Gegenstand der Erfindung sind auch die Stereoisomeren (E, Z-Isomeren) sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Selbstverständlich versteht man unter den Verbindungen der Formel I auch die Solvate der Salze.

Unter pharmazeutisch verwendbaren Derivaten versteht man z.B. die Salze der erfindungsgemäßen Verbindungen.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Für alle Reste, die mehrfach auftreten, gilt, daß deren Bedeutungen unabhängig voneinander sind.

Vor- und nachstehend haben die Reste bzw. Parameter R¹, R², R³ und X die bei der Formel I angegebenen Bedeutungen, falls nicht ausdrücklich etwas anderes angegeben ist.

Carbamoyl bedeutet Aminocarbonyl.

BOC oder Boc bedeutet tert.-Butyloxycarbonyl.

A bzw. A' bedeutet vorzugsweise Alkyl, ist unverzweigt (linear) oder verzweigt, und hat 1, 2, 3, 4, 5, 6, 7, 8, 9, 10, 11, 12, 13, 14, 15 oder 16 C-Atome. A bzw. A' bedeutet besonders bevorzugt Methyl, Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1-oder 2-Ethylbutyl, 1-Ethyl-1-methylpropyl, 1-Ethyl-2-methylpropyl, 1,1,2- oder 1,2,2-Trimethylpropyl.

A bzw. A' bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

A, A' bedeuten auch jeweils unabhängig voneinander unverzweigtes oder verzweigtes Alkyl mit 1-16 C-Atomen, worin 1-6 nicht-benachbarte CH₂-Gruppen durch O, NH, NMe, NEt, NHCO, CONH, NHCOO, NA'COO und/oder CH-Gruppen durch N und/oder auch 1-7 H-Atome durch F, Cl, OH und/oder NH₂ ersetzt sein können, wie z.B. 2-Methoxy-ethyl oder 3-Methylamino-propyl. A bzw. A' bedeutet auch cyclisches Alkyl (Cycloalkyl). Cycloalkyl oder cyclisches Alkyl bedeutet vorzugsweise Cyclopropyl, Cyclobutyl, Cylopentyl, Cyclohexyl oder Cycloheptyl.

A" bedeutet vorzugsweise Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

R⁴, R⁵, R⁶ bedeuten vorzugsweise, jeweils unabhängig voneinander, H oder A.

R⁴ und R⁵ oder
R⁵ und R⁶ bedeuten zusammen mit dem Heteroatom an das sie gebunden sind, auch einen unsubstituierten oder ein-, zwei- oder dreifach durch Hal, A, (CH₂)ₙOH, (CH₂)ₙOA, (CH₂)ₙNH₂, (CH₂)ₙCOOH, (CH₂)ₙCOOA, NHCOA, NA'COA, CONH₂, CONHA, CONAA', OC(=O)(CH₂)ₙNH₂ und/oder =O (Carbonylsauerstoff) substituierten gesättigten, ungesättigten oder aromatischen mono- oder bicyclischen Heterocyclus, der weitere 1 bis 3 N-, O- und/oder S-Atome enthalten kann, und worin auch ein N-Atom oxidiert sein kann.

Der Heterocyclus ist vorzugsweise ausgewählt aus der Gruppe Piperidin, Pyrrolidin, Piperazin, [1,2]Oxazinan, [1,2,5]Oxadiazinan, [1,3]Oxazinan, Hexahydropyrimidinyl, Morpholinyl, Imidazolidin, Oxazolidin, Dihydroindol, Isoindolin, Tetrahydrochinolin, Tetrahydroisochinolin, Tetrahydrochinoxalin.

X bedeutet vorzugsweise OR⁴, O(CO)R⁴, O(CO)OR⁴, O(CO)NR⁴R⁵, ONR⁴R⁵, OP(=O)(OH)₂, OP(=O)(OA")₂ NR⁴OR⁵, NR⁴R⁵, NR⁴(CO)R⁵, NR⁴(CO)NR⁵, NR⁴(CO)OR⁵, 1-A-Piperazin-4-yl-COO,
unsubstituiertes oder ein-, zwei- oder dreifach durch Hal, A, (CH₂)ₙOH, (CH₂)ₙOA, (CH₂)ₙNH₂, (CH₂)ₙCOOH, (CH₂)ₙCOOA, NHCOA, NA'COA, CONH₂, CONHA, CONAA' und/oder =O substituiertes Piperidin-1-yl, Pyrrolidin-1-yl, Piperazin-1-yl, [1,2]Oxazinan-2-yl, [1,2,5]Oxadiazinan-2-yl, [1,3]Oxazinan-3-yl, Hexahydropyrimidinyl oder Morpholinyl.

Y bedeutet vorzugsweise Methylen, Ethylen oder Propylen.

W, W' bedeuten vorzugsweise, jeweils unabhängig voneinader, Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Z bedeutet vorzugsweise Acetyl, Propionyl, Methoxycarbonyl, Ethoxycarbonyl, Dimethylaminocarbonyl, Diethylaminocarbonyl, SOCH₃ oder SO₂CH₃.

Ar bedeutet z.B. Phenyl, o-, m- oder p-Tolyl, o-, m- oder p-Ethylphenyl, o-, m-oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m-oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m-oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, o-, m- oder p-Cyanphenyl, o-, m- oder p-Acetylphenyl, o-, m- oder p-Aminosulfonylphenyl, o-, m- oder p-Carboxyphenyl, o-, m- oder p-Carboxymethyl-phenyl, o-, m- oder p-Carboxymethoxy-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-Iodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet besonders bevorzugt unsubstituiertes oder ein-, zwei-, drei-, vier-oder fünffach durch A, Hal und/oder OA substituiertes Phenyl.

Het bedeutet, ungeachtetet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3-oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7-oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Die heterocyclischen Reste können auch teilweise oder vollständig hydriert sein.

Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, Isoindolinyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet vorzugsweise unsubstituiertes oder ein-, zwei- oder dreifach durch A, OA, OH, Hal, CN und/oder =O (Carbonylsauerstoff) substituiertes Pyridyl, Pyrimidinyl, Furyl, Thienyl, Pyrrolyl, Imidazolyl, Pyrazolyl, Triazolyl, Oxazolyl, Isoxazolyl, Thiazolyl, Benzothiazolyl, Benzo[1,4]dioxanyl, Chinolyl, Isochinolyl, Chinazolinyl, Benzimidazolyl, Indazolyl, Indolyl, 1,3-Dihydro-isoindolyl, Benzofuranyl, Dihydro-benzofuranyl, Benzo[1,3]dioxolyl, Piperazinyl, Pyrazinyl, Pyridazinyl, Morpholinyl, Azepanyl, Azetidinyl, Pyrrolidinyl oder Piperidinyl.

n bedeutet vorzugsweise 0, 1 oder 2.

Die Verbindungen der Formel I können ein oder mehrere chirale Zentren besitzen und daher in verschiedenen stereoisomeren Formen vorkommen. Die Formel I umschließt alle diese Formen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Verbindungen der Formel I können vorzugsweise erhalten werden, indem man eine Verbindung der Formel II mit einer Verbindung der Formel III umsetzt.

In den Verbindungen der Formel II bedeutet R eine Aminoschutzgruppe, vorzugsweise tert.-Butyloxycarbonyl (BOC).

In den 1,3-Bis(trialkylsilyl)carbodiimiden der Formel III bedeutet Alkyl vorzugsweise C1, C2, C3 oder C4 Alkyl, wie z.B. N,N'-Bis(trimethylsilyl)-carbodiimid.

Als Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chlorform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl-oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Umsetzung erfolgt in einem geeigneten Lösungsmittel, vorzugsweise THF oder Acetonitril, und bei Temperaturen zwischen 10 und 50°C.

Die Reaktionszeit liegt je nach den angewendeten Bedingungen zwischen einigen Minuten und 14 Tagen, die Reaktionstemperatur zwischen etwa 0° und 150°, normalerweise zwischen 15° und 120°, besonders bevorzugt zwischen 20° und 60°C.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammoniumformiat (anstelle von Wasserstoff) an Pd/C in Methanol/DMF bei 20-30°.

Verbindungen der Formel I können weiterhin vorzugsweise erhalten werden, indem man eine Verbindung der Formel IV mit einer Verbindung der Formel V umsetzt.

Die Umsetzung erfolgt unter Bedingungen wie sie dem Fachmann für eine Suzuki-Reaktion bekannt sind.

Die Ausgangsverbindungen der Formeln IV und V sind in der Regel bekannt. Sind sie neu, so können sie aber nach an sich bekannten Methoden hergestellt werden.

In den Verbindungen der Formel V bedeutet L vorzugsweise

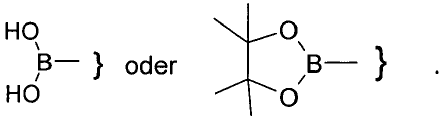

Die Umsetzung erfolgt unter Standardbedingungen einer Suzuki-Kopplung. Die Reaktionszeit liegt je nach den angewendeten Bedingungen zwischen einigen Minuten und 14 Tagen, die Reaktionstemperatur zwischen etwa -30° und 140°, normalerweise zwischen 0° und 100°, insbesondere zwischen etwa 60° und etwa 90°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl-oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Besonders bevorzugt ist Ethanol, Toluol, Dimethoxyethan und/oder Wasser.

Es ist ferner möglich, eine Verbindung der Formel I in eine andere Verbindung der Formel I umzuwandeln, indem man z.B. Nitrogruppen, beispielsweise durch Hydrierung an Raney-Nickel oder Pd-Kohle in einem inerten Lösungsmittel wie Methanol oder Ethanol, zu Aminogruppen reduziert und/oder
eine Estergruppe in eine Carboxygruppe umwandelt oder Carboxygruppen durch Umsetzung mit Alkoholen verestert und/oder Carboxygruppen oder Säurechloride durch Umsetzung mit einem Amin in ein Säureamid überführt.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Amino- und /oder Hydroxygruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Etherspaltungen erfolgen nach Methoden, die dem Fachmann bekannt sind.

Die Reaktion erfolgt in einem geeigneten Lösungsmittel, wie oben angegeben, vorzugsweise durch Zugabe von Bortribromid.

Die Reaktion erfolgt besonders bevorzugt in Dichlormethan bei einer Reaktionstemperatur zwischen etwa -30° und 50°, normalerweise zwischen - 20° und 20°, insbesondere zwischen etwa -15° und etwa 0°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der erfindungsgemäßen Verbindungen werden größtenteils konventionell hergestellt. Sofern die erfindungsgemäße Verbindung eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I gemäß Anspruch 1 zählen ebenfalls dazu. Bei bestimmten Verbindungen der Formel I gemäß Anspruch 1 lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I gemäß Anspruch 1 die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Palmoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der erfindungsgemäßen Verbindungen, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer erfindungsgemäßer Verbindungen werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der erfindungsgemäßen Verbindungen mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine erfindungsgemäße Verbindung in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Salze, Tautomere und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,1 mg bis 3 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akäzia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine erfindungsgemäße Verbindung und/oder ihre pharmazeutisch verwendbaren Salze, Tautomere und Stereoisomere, einschließlich deren Mischungen in allen Verhältnissen, und mindestens einen weiteren Arzneimittelwirkstoff.

Als weitere Arzneimittelwirkstoffe sind Chemotherapeutika bevorzugt, insbesondere solche, die Angiogenese hemmen und dadurch das Wachstum und die Verbreitung von Tumorzellen inhibieren; bevorzugt sind dabei VEGF-Rezeptorinhibitoren, beinhaltend Robozyme und Antisense, die auf VEGF-Rezeptoren gerichtet sind, sowie Angiostatin und Endostatin.

Beispiele antineoplastischer Agenzien, die in Kombination mit den erfindungsgemäßen Verbindungen verwendet werden können, beinhalten im allgemeinen alkylierende Agenzien, Antimetaboliten; Epidophyllotoxin; ein antineoplastisches Enzym; einen Topoisomerase-Inhibitor; Procarbazin; Mitoxantron oder Platin-Koordinationskomplexe.

Antineoplastische Agenzien sind vorzugsweise ausgewählt aus den folgenden Klassen:
Anthracycline, Vinca-Arzneistoffe, Mitomycine, Bleomycine, cytotoxische Nukleoside, Epothilone, Discodermolide, Pteridine, Diynene und Podophyllotoxine.

Besonders bevorzugt sind in den genanten Klassen z.B. Carminomycin, Daunorubicin, Aminopterin, Methotrexat, Methopterin, Dichlormethotrexat, Mitomycin C, Porfiromycin, 5-Fluoruracil, 6-Mercaptopurin, Gemcitabine, Cytosinarabinosid, Podophyllotoxin oder Podophyllotoxinderivate, wie z.B. Etoposide, Etoposide Phosphat oder Teniposide, Melphalan, Vinblastine, Vincristine, Leurosidine, Vindesine, Leurosine und Paclitaxel. Andere bevorzugte antineoplastische Agenzien sind ausgewählt aus der Gruppe Estramustine, Carboplatin, Cyclophosphamid, Bleomycin, Gemcitabine, Ifosamide, Melphalan, Hexamethylmelamin, Thiotepa, Cytarabin, Idatrexate, Trimetrexate, Dacarbazine, L-Asparaginase, Camptothecin, CPT-11, Topotecan, Arabinosyl-Cytosin, Bicalutamide, Flutamide, Leuprolide, Pyridobenzoindolderivate, Interferone und Interleukine.

Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren Salze, Tautomere und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Salze, Tautomere und Stereoisomere, einschließlich deren Mischungen in allen Verhältnissen,
und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von Krankheiten, bei denen HSP90 eine Rolle spielt.

Die vorliegende Erfindung umfasst die Verwendung der erfindungsgemäßen Verbindungen und/oder ihre physiologisch unbedenklichen Salze und Solvate zur Herstellung eines Arzneimittels zur Behandlung von Tumorerkrankungen, wie z.B. Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung; viralen Erkrankungen, wobei das virale Pathogen ausgewählt ist aus der Gruppe, bestehend aus Hepatitis Typ A, Hepatitis Typ B, Hepatitis Typ C, Influenza, Varicella, Adenovirus, Herpes-Simplex Typ I (HSV-I), Herpes Simplex Typ II (HSV-II), Rinderpest, Rhinovirus, Echovirus, Rotavirus, respiratorischem Synzytialvirus (RSV), Papillomvirus, Papovavirus, Cytomegalievirus, Echinovirus, Arbovirus, Huntavirus, Coxsackievirus, Mumpsvirus, Masernvirus, Rötelnvirus, Poliovirus, menschliches Immunschwächevirus Typ I (HIV-I) und menschliches Immunschwächevirus Typ II (HIV-II);
zur Immunsuppression bei Transplantationen; entzündungsbedingten Erkrankungen, wie Rheumatoide Arthritis, Asthma, Sepsis, Multiple Sklerose, Typ 1 Diabetes, Lupus Erythematodes, Psoriasis und Inflammatory Bowel Disease; Zystische Fibrose; Erkrankungen im Zusammenhang mit Angiogenese wie z.B. diabetische Retinopathie, Hämangiome, Endometriose, Tumorangiogenese; infektiösen Erkrankungen; Autoimmunerkrankungen; Ischämie; Förderung der Nervenregeneration; fibrogenetischen Erkrankungen, wie z.B. Sklerodermie, Polymyositis, systemischer Lupus, Leberzirrhose, Keloidbildung, interstitielle Nephritis und pulmonare Fibrose;

Die erfindungsgemäßen Verbindungen können insbesondere das Wachstum von Krebs, Tumorzellen und Tumormetastasen hemmen und sind deshalb für die Tumortherapie geeignet.

Die vorliegende Erfindung umfasst weiterhin die Verwendung der erfindungsgemäßen Verbindungen und/oder ihre physiologisch unbedenklichen Salze, Tautomere und Stereoisomere zur Herstellung eines Arzneimittels zum Schutz normaler Zellen gegen Toxizität, die durch Chemotherapie verursacht ist, sowie zur Behandlung von Krankheiten, wobei Proteinfehlfaltung oder Aggregation ein Hauptkausalfaktor ist, wie z.B. Skrapie, Creutzfeldt-Jakob-Krankheit, Huntington oder Alzheimer.

Die Erfindung betrifft auch die Verwendung der erfindungsgemäßen Verbindungen und/oder ihre physiologisch unbedenklichen Salze, Tautomere und Stereoisomere zur Herstellung eines Arzneimittels zur Behandlung von Krankheiten des Zentralnervensystems, von Herzkreislauferkrankungen und Kachexie.

Die Erfindung betrifft in einer weiteren Ausführungsform auch die Verwendung der erfindungsgemäßen Verbindungen und/oder ihre physiologisch unbedenklichen Salze, Tautomere und Stereoisomere zur Herstellung eines Arzneimittels zur HSP90-Modulation, wobei die modulierte biologische HSP90-Aktivität eine Immunreaktion in einem Individuum, Proteintransport vom endoplasmatischen Retikulum, Genesung vom hypoxischen / anoxischen Stress, Genesung von Unterernährung, Genesung von Hitzestress, oder Kombinationen davon, hervorruft, und/oder wobei die Störung eine Art Krebs ist, eine Infektionserkrankung, eine Störung, die mit einem gestörten Proteintransport vom endoplasmatischen Retikulum, einer Störung, die mit Ischämie / Reperfusion einhergeht, oder Kombinationen davon, wobei die die mit Ischämie / Reperfusion einhergehende Störung eine Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist.

Die Erfindung betrifft in einer weiteren Ausführungsform auch die Verwendung der erfindungsgemäßen Verbindungen und/oder ihre physiologisch unbedenklichen Salze, Tautomere und Stereoisomere zur Herstellung eines Arzneimittels zur Behandeln von Ischämie als Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist.

Gegenstand der Erfindung sind die Verbindungen der Formel I gemäß Anspruch 1 sowie ihrer pharmazeutisch verwendbaren Salze, Tautomere und Stereoisomere, einschließlich deren Mischungen in allen Verhältnissen, zur Verwendung zur Behandlung oder Vorbeugung von Tumorerkrankungen, viralen Erkrankungen, zur Immunsuppression bei Transplantationen, entzündungsbedingten Erkrankungen, Zystische Fibrose, Erkrankungen im Zusammenhang mit Angiogenese, infektiösen Erkrankungen, Autoimmunerkrankungen, Ischämie, fibrogenetischen Erkrankungen,
zur Förderung der Nervenregeneration,
zur Hemmung des Wachstums von Krebs, Tumorzellen und Tumormetastasen,
zum Schutz normaler Zellen gegen Toxizität, die durch Chemotherapie verursacht ist,
zur Behandlung von Krankheiten, wobei Proteinfehlfaltung oder Aggregation ein Hauptkausalfaktor ist.

Gegenstand der Erfindung sind weiterhin die Verbindungen der Formel I gemäß Anspruch 1 sowie ihrer pharmazeutisch verwendbaren Salze, Tautomere und Stereoisomere, einschließlich deren Mischungen in allen Verhältnissen, zur Verwendung zur Behandlung oder Vorbeugung von Tumoren, viralen Infekten, Malaria, Abstossungsreaktionen von Transplantaten, inflammatorischen Erkrankungen, multipler Sklerose, Alzheimer, Rheumatoide Arthritis, Asthma, COPD, Typ 1 Diabetes, Lupus, Psoriasis, cystische Fibrose, diabetische Retinopathie, Hämangion, Endometritis, angiogenese-bedingte Erkrankungen, sowie zur Behandlung von Herzkreislauferkrankungen oder Kachexie.

### Testverfahren zur Messung von HSP90 Inhibitoren

Die Bindung von Geldanamycin oder 17- Allylamino-17-demethoxygeldanamycin (17AAG) und deren kompetitive Hemmung an HSP90 kann benutzt werden, um die inhibitorische Aktivität der erfindungsgemäßen Verbindungen zu bestimmen (Carreras et al. 2003, Chiosis et al. 2002).

Im speziellen Fall wird ein Radioligand-Filterbindungstest verwendet. Als Radioligand wird dabei mit Tritium markiertes 17-Allylamino-geldanamycin, [3H]17AAG, verwendet. Dieser Filter-Bindungstest erlaubt eine gezielte Suche nach Inhibitoren, die mit der ATP-Bindestelle interferieren.

### Material

Rekombinantes humanes HSP90α (E. coli exprimiert, 95% Reinheit); [3H]17AAG (17-Allylamino-geldanamycin, [allylamino-2,3-³H. Spezifische Aktivität: 1,11x10¹² Bq/mmol (Moravek, MT-1717);
HEPES Filterpuffer (50 mM HEPES, pH 7,0, 5mM MgCl2, BSA 0.01 %) Multiscreen-FB (1µm) Filterplatte (Millipore, MAFBNOB 50).

### Methode

Die 96 well Mikrotiter-Filterplatten werden zunächst gewässert und mit 0,1% Polyethylenimin beschichtet.

Der Test wird unter folgenden Bedingungen durchgeführt:
Reaktionstemperatur 22 °C
Reaktionszeit: 30 min., Schütteln bei 800 upm
Testvolumen: 50 µl
Endkonzentrationen:
50 mM HEPES-HCl, pH7,0, 5 mM MgCl2, 0,01 % (w/v) BSA
HSP90: 1,5 µg/assay
[3H]17AAG: 0,08 µM.

Am Ende der Reaktion wird der Überstand in der Filterplatte mit Hilfe eines Vakuum-Manifolds (Multiscreen Separation System, Millipore) abgesaugt und der Filter zweimal gewaschen.

Die Filterplatten werden dann in einem Beta-counter (Microbeta, Wallac) mit Szintillator (Microscint 20, Packard) gemessen.

Aus den "counts per minutes"-Werten wird "% der Kontrolle" ermittelt und daraus der IC-50 Wert einer Verbindung kalkuliert.

**Tabelle I**

| HSP90-Inhibierung durch erfindungsgemäße Verbindungen | |
|---|---|
| Verbindung Nr. | IC₅₀ |
| {2-Amino-6-[2-(tert.-butylamino-methyl)-4,5-difluor-phenyl]-chinazolin-4-yl}-(1,3-dihydro-isoindol-2-yl)-methanon | A |
| 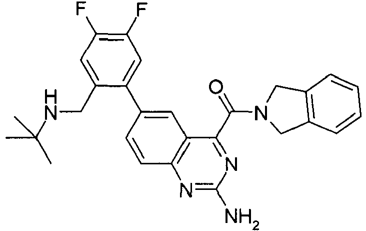 | |
| (2-Amino-6-{2-[(ethyl-methyl-amino)-methyl]-4,5-difluor-phenyl}-chinazolin-4-yl)-(1,3-dihydro-isoindol-2-yl)-methanon | A |
| 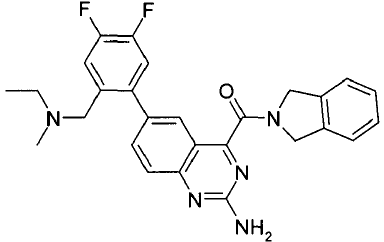 | |
| [2-Amino-6-(2-diethylaminomethyl-4,5-difluor-phenyl)-chinazolin-4-yl]-(1,3-dihydro-isoindol-2-yl)-methanon | A |
| 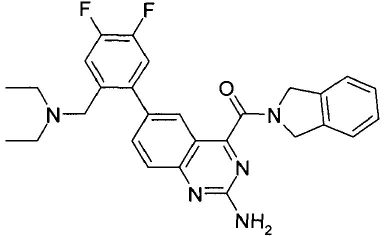 | |
| [2-Amino-6-(4,5-difluor-2-pyrrolidin-1-ylmethyl-phenyl)-chinazolin-4-yl]-(1,3-dihydro-isoindol-2-yl)-methanon | A |
| 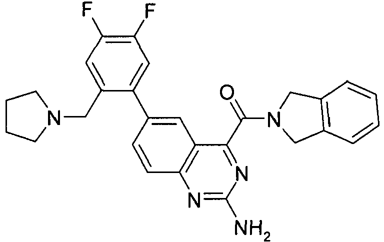 | |
| {2-Amino-6-[2-(2,5-dimethyl-pyrrolidin-1-ylmethyl)-4,5-difluor-phenyl]-chinazolin-4-yl}-(1,3-dihydro-isoindol-2-yl)-methanon | A |
| 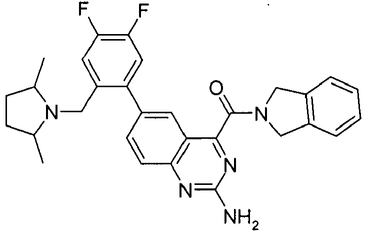 | |
| {2-Amino-6-[4,5-difluor-2-(2-methyl-pyrrolidin-1-ylmethyl)-phenyl]-chinazolin-4-yl}-(1,3-dihydro-isoindol-2-yl)-methanon | A |
| 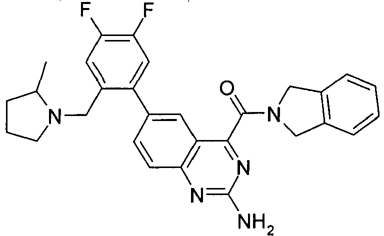 | |
| (2-Amino-6-{2-[(tert.-butyl-methyl-amino)-methyl]-4,5-difluor-phenyl}-chinazolin-4-yl)-(1,3-dihydro-isoindol-2-yl)-methanon | A |
| 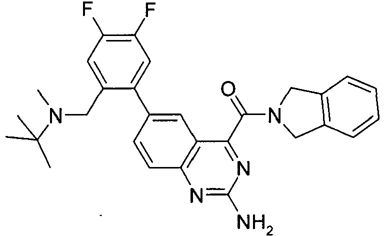 | |
| {2-Amino-6-[4,5-difluor-2-(4-methyl-piperazin-1-ylmethyl)-phenyl]-chinazolin-4-yl}-(1,3-dihydro-isoindol-2-yl)-methanon | A |
| 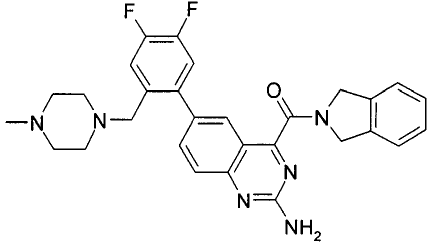 | |
| [2-Amino-6-(2-aminomethyl-4,5-difluor-phenyl)-chinazolin-4-yl]-(1,3-dihydro-isoindol-2-yl)-methanon | A |
| 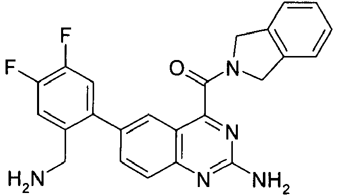 | |
| 3-Amino-N-{2-[2-amino-4-(1,3-dihydro-isoindol-2-carbonyl)-chinazolin-6-yl]-4,5-difluor-benzyl}-propionamid | A |
| 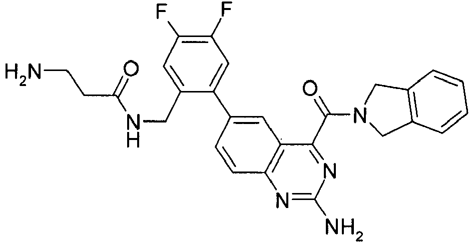 | |
| N-{2-[2-Amino-4-(1,3-dihydro-isoindol-2-carbonyl)-chinazolin-6-yl]-4,5-difluor-benzyl}-3-dimethylamino-propionamid | A |
| 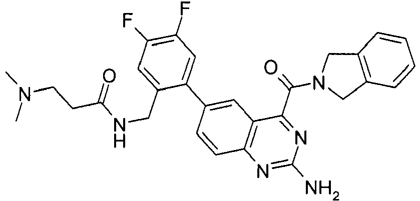 | |
| 3-Amino-propionsäure-2-[2-amino-4-(1,3-dihydro-isoindol-2-carbonyl)-chinazolin-6-yl]-4,5-difluor-benzylester | A |
| 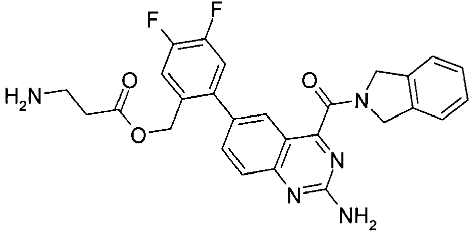 | |
| {2-Amino-6-[2-(3-dimethylamino-propoxymethyl)-4,5-difluor-phenyl]-chinazolin-4-yl}-(1,3-dihydro-isoindol-2-yl)-methanon | A |
| 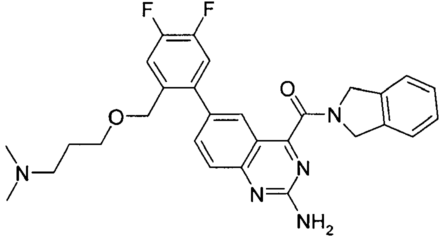 | |
| {2-Amino-6-[2-(2-dimethylamino-ethoxymethyl)-4,5-difluor-phenyl]-chinazolin-4-yl}-(1,3-dihydro-isoindol-2-yl)-methanon | A |
| 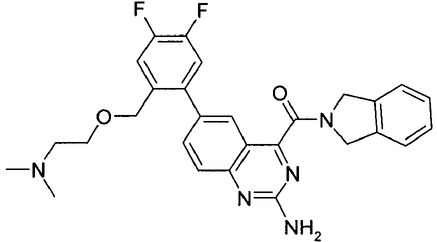 | |
| {2-Amino-6-[2-(3-amino-propoxymethyl)-4,5-difluor-phenyl]-chinazolin-4-yl}-(1,3-dihydro-isoindol-2-yl)-methanon | A |
| 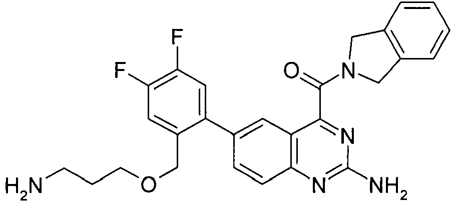 | |
| {2-Amino-6-[4,5-difluor-2-(2-hydroxy-ethoxymethyl)-phenyl]-chinazolin-4-yl}-(1,3-dihydro-isoindol-2-yl)-methanon | A |
| 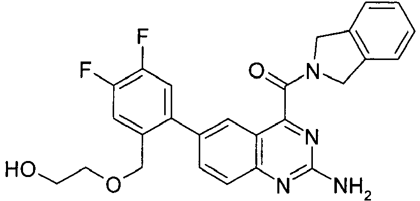 | |
| [2-Amino-6-(4,5-difluor-2-{2-[2-(2-hydroxy-ethoxy)-ethoxy]-ethoxymethyl}-phenyl)-chinazolin-4-yl]-(1,3-dihydro-isoindol-2-yl)-methanon | A |
| 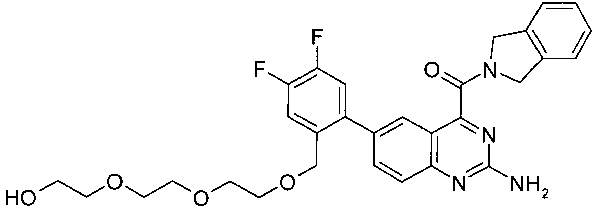 | |
| (S)-2,5-Diamino-pentansäure-2-[2-amino-4-(1,3-dihydro-isoindol-2-carbonyl)-chinazolin-6-yl]-4,5-difluor-benzylester | A |
| 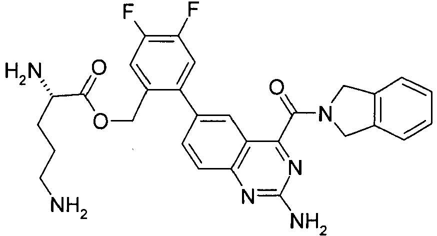 | |
| 3-Dimethylamino-propionsäure-2-[2-amino-4-(1,3-dihydro-isoindol-2-carbonyl)-chinazolin-6-yl]-4,5-difluor-benzylester | A |
| 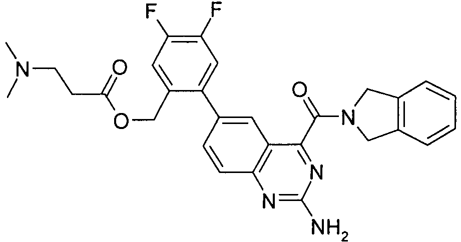 | |
| 2,6-Diamino-hexansäure-2-[2-amino-4-(1,3-dihydro-isoindol-2-carbonyl)-chinazolin-6-yl]-4,5-difluor-benzylester | A |
| 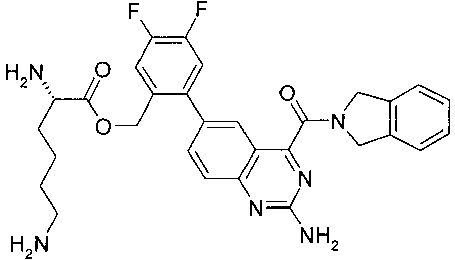 | |
| [2-Amino-6-(2-aminooxymethyl-4,5-difluor-phenyl)-chinazolin-4-yl]-(1,3-dihydro-isoindol-2-yl)-methanon | A |
| 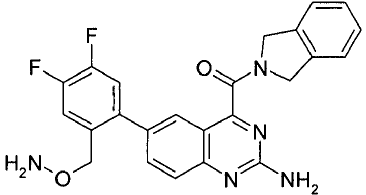 | |
| Phosphorsäure-mono-{2-[2-amino-4-(1,3-dihydro-isoindol-2-carbonyl)-chinazolin-6-yl]-4,5-difluor-benzyl}-ester | A |
| 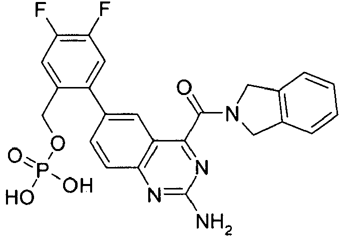 | |
| Phosphorsäure-2-[2-amino-4-(1,3-dihydro-isoindol-2-carbonyl)-chinazolin-6-yl]-4,5-difluor-benzylester-di-tert.-butylester | A |
| 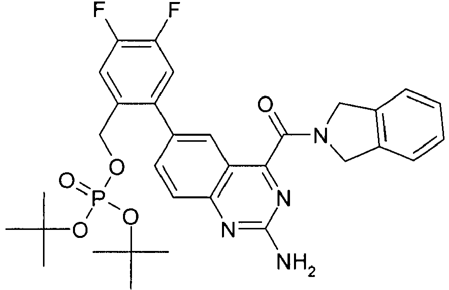 | |
| 2-Amino-4-methyl-pentansäure-2-[2-amino-4-(1,3-dihydro-isoindol-2-carbonyl)-chinazolin-6-yl]-4,5-difluor-benzylester | A |
| 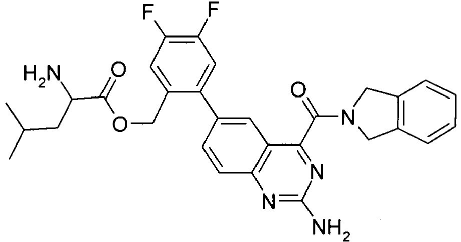 | |
| 2,2-Dimethyl-propionsäure-2-[2-amino-4-(1,3-dihydro-isoindol-2-carbonyl)-chinazolin-6-yl]-4,5-difluor-benzylester | A |
| 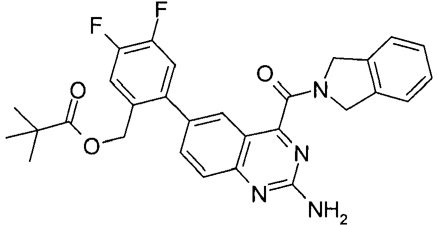 | |
| 6-tert.-Butoxycarbonylamino-hexansäure-2-[2-amino-4-(1,3-dihydro-isoindol-2-carbonyl)-chinazolin-6-yl]-4,5-difluor-benzylester | A |
| 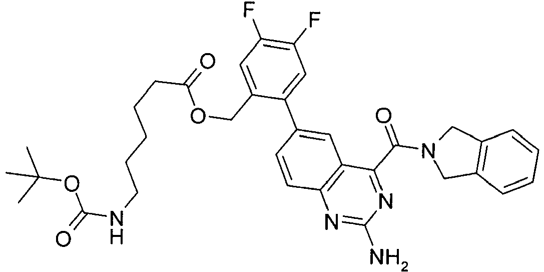 | |
| (S)-Pyrrolidin-1,2-dicarbonsäure-2-{2-[2-amino-4-(1,3-dihydro-isoindol-2-carbonyl)-chinazolin-6-yl]-4,5-difluor-benzyl}-ester-1-tert.-butylester | A |
| 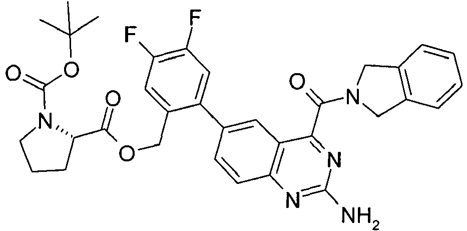 | |
| (S)-Pyrrolidin-2-carbonsäure-2-[2-amino-4-(1,3-dihydro-isoindol-2-carbonyl)-chinazolin-6-yl]-4,5-difluor-benzylester | A |
| 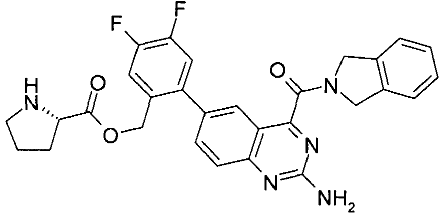 | |

| | |
|---|---|
| IC₅₀: 10nM - 1 µM = A 1 µM - 10 µM = B > 10 µM = C | |

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### LC-MS Bedingungen

Die LC/MS Messungen erfolgen mit einem Hewlett Packard System der HP 1200 Serie mit den folgenden Merkmalen: Ionenquelle: Elektrospray (positive mode); Scan: 100-1000 m/z; Fragmentier-Spannung: 60 V; GasTemperatur: 300°C, UV: 220 nm.
Flussrate: 2.4 ml/min.
Säule: Chromolith SpeedROD RP-18e 50-4.6
Lösungsmittel: LiChrosolv-Qualität der Fa. Merck KGaA
Lösungsmittel A: H₂O (0.05% Ameisensäure)
Lösungsmittel B: ACN (0.04% Ameisensäure)
Gradient "Standard":
   4% B → 100% B: 0 min bis 2.8 min
   100% B: 2.8 min bis 3.3 min
   100%B → 4%B: 3.3 min bis 3.4 min
Gradient "Polar":
   0% B: 0 min bis 0.5 min
   0% B → 100% B: 0.5 min bis 2.6 min
   100% B: 2.6 min bis 3 min
   100%B → 10%B: 3 min bis 3.1 min
Gradient "Unpolar":
   20% B → 100% B: 0 min bis 2.8 min
   100% B: 2.8 min bis 3.3 min
   100%B → 20%B: 3.3 min bis 3.4 min

Wenn keine weiteren Angaben zur Retentionszeit gemacht werden, wird Gradient "Standard" verwendet.

### Allgemeine Syntheseschemata:

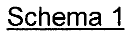

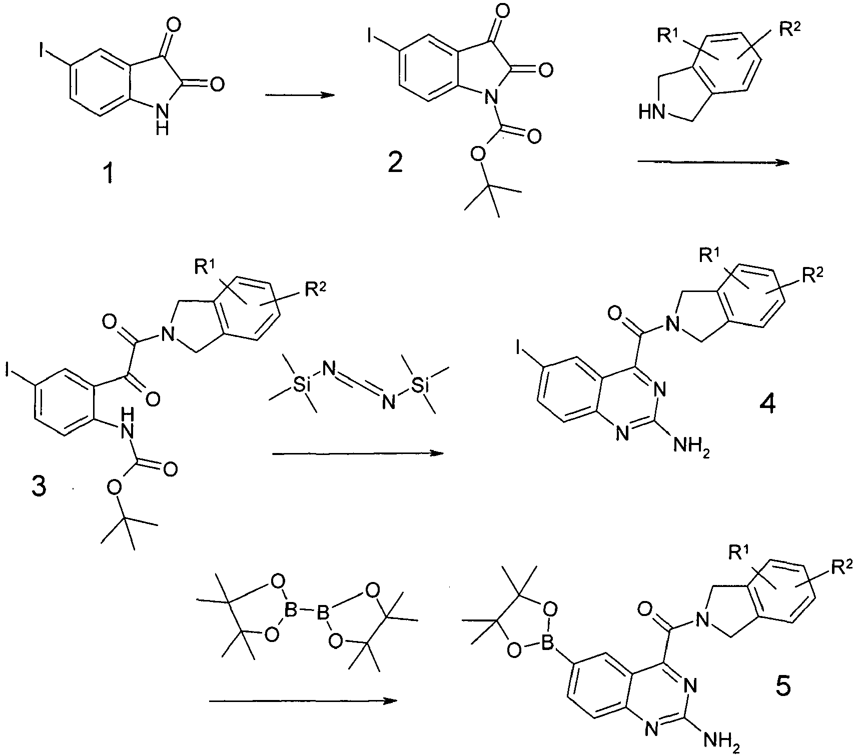

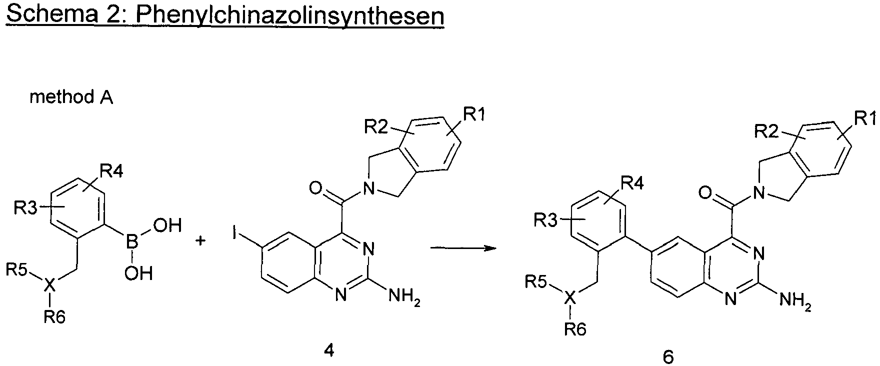

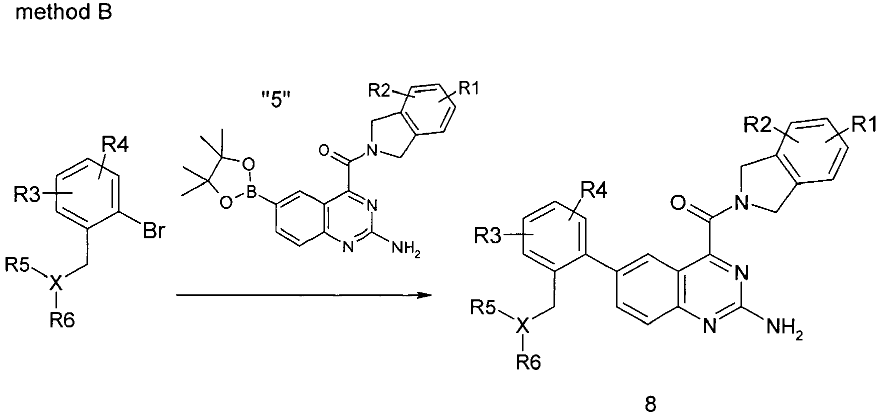

### Synthese nach Schema 1

### 5-Iod-2,3-dioxo-2,3-dihydro-indol-1-carbonsäure-tert. -butylester ("2")

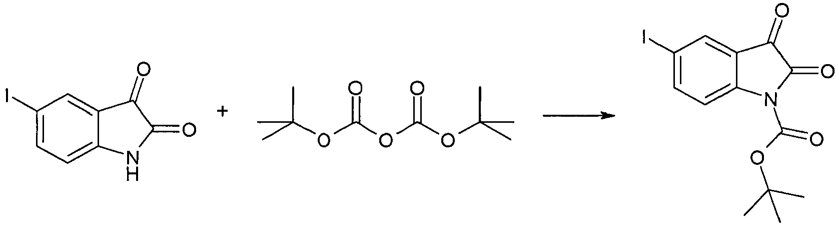

50 g 5-Iod-1H-indol-2,3-dion werden in 500 ml THF gelöst, auf 10°C abgekühlt und mit 43.97 g Di-tert.-butyldicarbonat versetzt. Es wird bei 23°C 16 h gerührt und das Gemisch anschließend im Vakuum zur Trockne eingeengt. Man nimmt in Petrolether und THF auf und kristallisiert bei -20 °C. Der so erhaltene gelbe Feststoff wird filtriert und bei 30°C im Trockenschrank getrocknet. Ausbeute: 62.41 g 5-Iod-2,3-dioxo-2,3-dihydro-indol-1-carbonsäure-tert.-butylester; Retentionszeit LC-MS: 2.11 min.

### {2-[2-(1,3-Dihydro-isoindol-2-yl)-2-oxo-acetyl]-4-iodo-phenyl}-carbamoylsäure-tert.-butylester ("3")

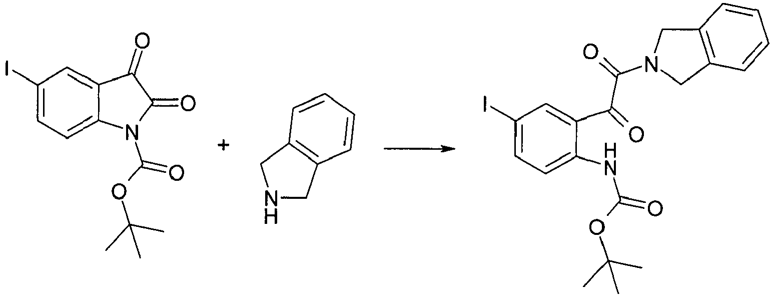

62.41 g 5-Iod-2,3-dioxo-2,3-dihydro-indol-1-carbonsäure-tert.-butylester werden in getrocknetem THF gelöst und mit 18,98 ml 2,3-Dihydro-1H-isoindol versetzt. Es wird 30 min bei 25°C gerührt, im Vakuum zur Trockne eingeengt und der Rückstand mit Petrolether verrieben, Filtration liefert 82.3 g {2-[2-(1,3-Dihydro-isoindol-2-yl)-2-oxo-acetyl]-4-iodo-phenyl}-carbamoylsäure-tert.-butylester (beiger Feststoff); Retentionszeit LC-MS: 2.63 min;
¹H NMR (500 MHz, DMSO-d₆/TFA-d₁): δ [ppm] 8.014 (d, 1H), 7.962 (dd, 1 H), 7.913 (d, 1 H), 7.391 (d, 1 H), 7.326-7.292 (m, 3H), 4.901 (s, 2H), 4.872 (s, 2H), 1.398 (s, 9H).

### (2-Amino-6-iod-chinazolin-4-yl)-(1,3-dihydro-isoindol-2-yl)-methanon ("4")

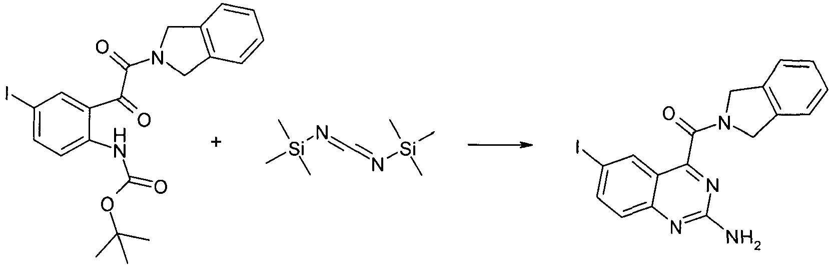

24.50 g {2-[2-(1,3-Dihydro-isoindol-2-yl)-2-oxo-acetyl]-4-iod-phenyl}-carbamoylsäure-tert.-butylester werden in 500 ml Acetonitril unter Argon gelöst. Man gibt 0,756 g Cäsiumfluorid dazu und tropft über 5 min 16,887 ml Bis(trimethylsilyl)carbodiimid zu der Lösung. Es wird 15 min bei Raumtemperatur gerührt und mit 400 ml Dichlormethan versetzt. Nach Zugabe von 400 ml Salzsäure (1 N) fällt das Produkt als weißer Feststoff aus; Ausbeute: 14 g (2-Amino-6-iod-chinazolin-4-yl)-(1,3-dihydro-isoindol-2-yl)-methanon; Retentionszeit LC-MS: 1,66 min;
¹H NMR (500 MHz, DMSO) 8.143 (d, 1 H), 7.957 (dd, 1 H), 7.451 (d, 1 H), 7.361-7.256 (m, 4H), 7.213 (s, 2H), 4.993 (s, 2H), 4.745 (s, 2H).

### [2-Amino-6-(4,4,5,5-tetramethyl-[1,3,2]dioxaborolan-2-yl)-chinazolin-4-yl]-(1,3-dihydro-isoindol-2-yl)-methanon ("5")

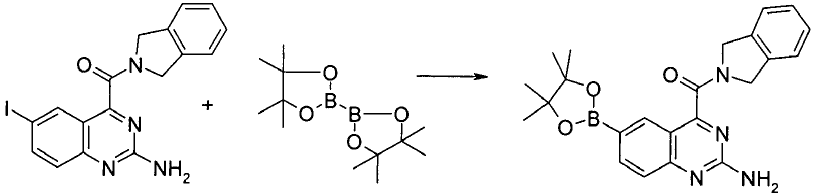

10 g (2-Amino-6-iod-chinazolin-4-yl)-(1,3-dihydro-isoindol-2-yl)-methanon ("4") werden unter Argonatmosphäre in 500 ml Dimethylsulfoxid gelöst. Man gibt 6.1 g Bis(pinacolato)diboron, 8.017 g Kaliumacetat und 981 mg [1,1'-Bis(diphenylphosphino)ferrocen]dichlorpalladium(II) zu dieser Lösung und erhitzt für 60 min auf 80°C. Nach Abkühlen wird das Gemisch mit 250 ml Diethylether versetzt und viermal gegen je 100 ml Wasser extrahiert. Die vereinigten organischen Phasen werden über Natriumsulfat getrocknet, filtriert und das Filtrat eingeengt, bis ein rotes Öl vorliegt. Dieses Öl wird mit Acetonitril verrieben, wobei hell-beige Kristalle entstehen. Der Niederschlag wird filtriert und im Trockenschrank bei 50°C für 12 h getrocknet. Das entstandene Produkt wird ohne weitere Reinigung weiter umgesetzt. Ausbeute: 6.45 g (65%) [2-Amino-6-(4,4,5,5-tetramethyl-[1,3,2]dioxaborolan-2-yl)-chinazolin-4-yl]-(1,3-dihydro-isoindol-2-yl)-methanon;
Retentionszeit LC-MS: 2,08 min;
¹H NMR (400 MHz, DMSO-d₆/TFA-d₁) δ [ppm] 8.38 (d, J = 0.6, 1H), 8.29 (dd, J = 8.4, 1.2, 1 H), 7.78 (d, J = 8.5, 1 H), 7.45 (d, J = 7.3, 1 H), 7.33 (dt, J = 15.1, 6.5, 2H), 7.24 (d, J = 7.2, 1 H), 5.11 (s, 2H), 4.92 (s, 2H), 1.33 (s, 12H).

### Schema 2, Methode A

### 2-[2-Amino-4-(1,3-dihydro-isoindol-2-carbonyl)-chinazolin-6-yl]-benzaldehyd

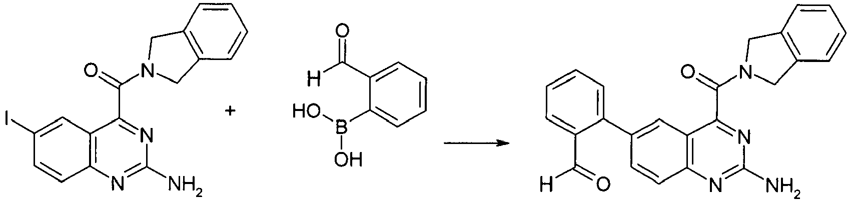

1 g (2-Amino-6-iod-chinazolin-4-yl)-(1,3-dihydro-isoindol-2-yl)-methanon werden in 10 ml Ethanol gelöst. Man gibt 468.3 mg 2-Formylphenylboronsäure, 664 mg Kaliumcarbonat, 58.9 mg [1,1'-Bis(diphenylphosphino)ferrocen]dichlorpalladium(II) und 3 ml Wasser zu dieser Lösung und erhitzt unter Argon für 30 min auf 130°C. Hierbei bildet sich ein Niederschlag, der filtriert und im Trockenschrank bei 50°C für 12 h getrocknet wird. Das entstandene Produkt wird ohne weitere Reinigung weiter umgesetzt. Ausbeute 910 mg 2-[2-Amino-4-(1,3-dihydro-isoindol-2-carbonyl)-chinazolin-6-yl]-benzaldehyd; Retentionszeit LC-MS: 1.86 min.

### [2-Amino-6-[2-(hydroxymethyl)phenyl]chinazolin-4-yl]-isoindolin-2-yl-methanon ("A1") (Vergleichsbeispiel)

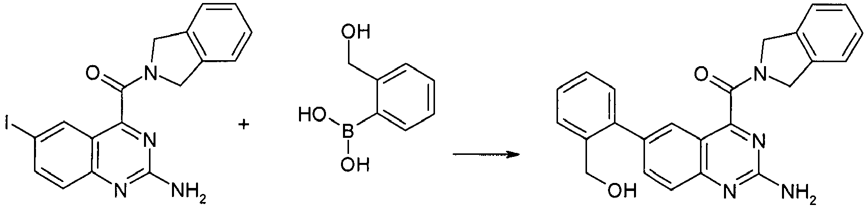

100 mg (2-Amino-6-iod-chinazolin-4-yl)-(1,3-dihydro-isoindol-2-yl)-methanon werden in 2 ml Ethylenglycoldimethylether gelöst. Man gibt 48 mg 2-Hydroxymethylphenylboronsäure, 66 mg Kaliumcarbonat, 14 mg [1,1'-Bis(diphenylphosphino)ferrocene]dichlorpalladium(II) und 50 µl Wasser zu dieser Lösung und erhitzt unter Argon für 30 min auf 130°C. Es wird heiß über Celite abfiltriert und die Lösung unter vermindertem Druck bis zur Trockene eingedampft. Der erhaltene rötliche Rückstand wird mit Acetonitril verrieben, wobei ein beiger Feststoff ausfällt. Dieser wird filtriert, mit Acetonitril gewaschen und über Nacht im Trockenschrank bei 40 °C getrocknet.
Ausbeute: 16 mg (17%) [2-Amino-6-[2-(hydroxymethyl)phenyl]chinazolin-4-yl]-isoindolin-2-yl-methanon; Retentionszeit HPLC: 1.83 min;
¹H NMR (400 MHz, DMSO-d₆/TFA-d₁) δ [ppm] 8.18 - 8.11 (m, 2H), 7.87 (d, J = 9.2, 1 H), 7.61 (d, J = 7.5, 1 H), 7.48 - 7.28 (m, 6H), 7.26 (d, J = 7.0, 1 H), 5.04 (s, 2H), 4.87 (s, 2H), 4.40 (s, 2H).

### [2-Amino-6-[5-fluor-2-(hydroxymethyl)phenyl]chinazolin-4-yl]-isoindolin-2-yl-methanon ("A2") (Vergleichsbeispiel)

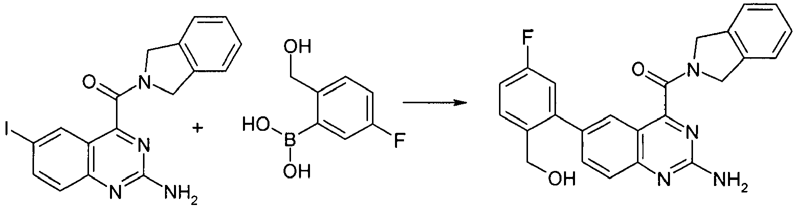

10.405 g (2-Amino-6-iod-chinazolin-4-yl)-(1,3-dihydro-isoindol-2-yl)-methanon werden in 450 ml Ethanol gelöst. Man gibt 5 g 5-Fluor-2-hydroxymethylphenylboronsäure, 10.4 g Kaliumcarbonat, 1 g [1,1'-Bis(diphenylphosphino)-ferrocen]dichlorpalladium(II) und 450 µl Wasser zu dieser Lösung und erhitzt unter Argon für 1 h auf 100°C. Es wird heiß über Celite abfiltriert und mit 500 ml heißem Ethanol nachgewaschen. Das Filtrat wird im Vakuum zur Trockne eingeengt und der erhaltene rötliche Rückstand mit 50 ml Acetonitril verrieben, wobei ein beiger Feststoff ausfällt. Dieser wird filtriert, mit 20 ml Acetonitril gewaschen und 16 h im Trockenschrank bei 40 °C getrocknet Ausbeute: 9.7 g (94%) [2-Amino-6-[5-fluoro-2-(hydroxymethyl)phenyl]-chinazolin-4-yl]-isoindolin-2-yl-methanon; Retentionszeit HPLC: 1.92 min; ¹H NMR (500 MHz, DMSO-d₆/TFA-d₁) δ [ppm] 8.14 - 8.08 (m, 2H), 7.82 (dd, J = 7.8, 1.6, 1 H), 7.58 (dd, J = 8.6, 6.1, 1 H), 7.41 (d, J = 7.2, 1 H), 7.33 - 7.25 (m, 2H), 7.24 - 7.18 (m, 2H), 7.13 (dd, J = 9.6, 2.7, 1 H), 4.99 (s, 2H), 4.82 (s, 2H), 4.31 (s, 2H).

### [2-Amino-6-[2-(methylaminomethyl)phenyl]chinazolin-4-yl]-isoindolin-2-yl-methanon ("A3") (Vergleichsbeispiel)

### a)

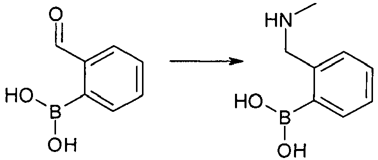

500 mg 2-Formylphenylboronsäure werden in 5 ml Methanol gelöst. Zu dieser Lösung werden 628 µl Methylamin (33%ig in Ethanol) gegeben, auf 0°C abgekühlt und 63 Natriumborhydrid dazugegeben. Man rührt 1 h bei 22°C, gibt 10 ml Wasser dazu und wäscht 3mal mit je 10 ml Ethylacetat. Die vereinigten organischen Phasen werden über Natriumsulfat getrocknet, filtriert und engt das Filtrat zur Trockne ein. Der erhaltene Feststoff wird ohne weitere Aufreinigung in der folgenden Reaktion eingesetzt. Ausbeute: 400 mg (73%) 2-Methylaminomethylphenylboronsäure; Retentionszeit LC-MS: 0.34 min (Gradient "polar").

### b)

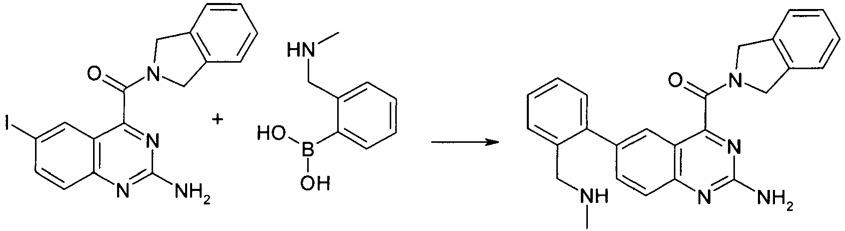

100 mg (2-Amino-6-iod-chinazolin-4-yl)-(1,3-dihydro-isoindol-2-yl)-methanon werden in 2 ml Ethanol gelöst. Man gibt 79 mg 2-Methylaminomethylphenylboronsäure, 66 mg Kaliumcarbonat, 6 mg [1,1'-Bis(diphenylphosphino)-ferrocen]dichlorpalladium(II) und 450 µl Wasser zu dieser Lösung und erhitzt unter Argon für 1 h auf 100°C. Es wird heiß über Celite abfiltriert und mit 500 ml heißem Ethanol nachgewaschen. Das Reaktiongemisch wird unter vermindertem Druck bis zur Trockene eingedampft, in 2 ml DMSO gelöst, filtriert und mittels präparativer HPLC (Fa. Agilent) chromatographiert. Die erhaltenen Produkt- Fraktionen werden anschließend eingeengt und gefriergetrocknet. Ausbeute: 20 mg (21%) [2-Amino-6-[2-(methylaminomethyl)phenyl]chinazolin-4-yl]-isoindolin-2-yl-methanon; Retentionszeit HPLC: 1.42 min;
¹H NMR (500 MHz, DMSO-d₆/TFA-d₁) δ [ppm] 8.08 - 8.05 (m, 1 H), 8.03 (dd, J = 8.6, 2.0, 1H), 7.85 (d, J = 8.6, 1 H), 7.69 - 7.65 (m, 1 H), 7.54 - 7.46 (m, 2H), 7.41 - 7.36 (m, 2H), 7.28 (dt, J = 19.8, 7.1, 2H), 7.21 (d, J = 7.3, 1 H), 5.00 (s, 2H), 4.82 (s, 2H), 4.02 (s, 2H), 2.46 (s, 3H).

### Schema 2, Methode B

### 2-[2-Amino-4-(isoindolin-2-carbonyl)chinazolin-6-yl]-4-fluor-benzaldehyd

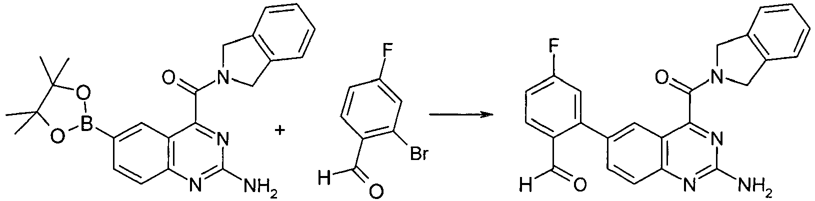

Zu einer Lösung von 500 mg [2-Amino-6-(4,4,5,5-tetramethyl-[1,3,2]dioxa-borolan-2-yl)-chinazolin-4-yl]-(1,3-dihydro-isoindol-2-yl)-methanon in 12 ml Ethanol unter Argon werden 244 mg 2-Brom-4-fluor-benzaldehyd, 0.35 g Kaliumcarbonat, 4 µl Wasser und 98 mg [1,1'-bis(diphenylphosphino)-ferrocen]dichlorpalladium(II) gegeben. Es wird für 4 h auf 120°C erhitzt; dann lässt man abkühlen, wobei sich ein Niederschlag abscheidet. Der Niederschlag wird abfiltriert, in 10 ml Ethylacetat aufgenommen und 3mal mit je 10 ml Wasser gewaschen. Nach Trocknen über Natriumsulfat wird abfiltriert und im Vakuum zur Trockne eingeengt; Ausbeute: 495 mg (91%) 2-[2-Amino-4-(isoindolin-2-carbonyl)chinazolin-6-yl]-4-fluor-benzaldehyd; Retentionszeit HPLC: 1.73 min;
¹H NMR (500 MHz, DMSO-d₆/TFA-d₁) δ [ppm] 9.78 (s, 1H), 8.13 (d, J = 1.7, 1 H), 8.08 (dd, J = 8.7, 2.0, 1 H), 8.02 (dd, J = 8.6, 6.0, 1 H), 7.82 (d, J = 8.7, 1 H), 7.42 - 7.31 (m, 3H), 7.26 (dt, J = 12.8, 6.8, 2H), 7.19 (d, J = 6.9, 1 H), 4.97 (s, 2H), 4.82 (s, 2H).

### 2-[2-Amino-4-(isoindolin-2-carbonyl)chinazolin-6-yl]-5-fluor-benzaldehyd

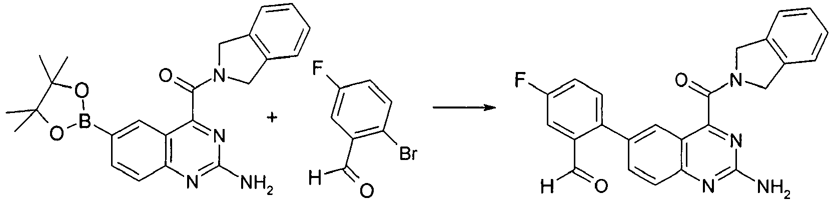

Zu einer Lösung von 2 g [2-Amino-6-(4,4,5,5-tetramethyl-[1,3,2]dioxaborolan-2-yl)-chinazolin-4-yl]-(1,3-dihydro-isoindol-2-yl)-methanon in 200 ml Ethanol unter Argon werden 244 mg 2-Bromo-5-fluoro-benzaldehyd, 1.7 g Kaliumcarbonat, 87 µl Wasser und 196 mg [1,1'-bis(diphenylphosphino)ferrocen]-dichlorpalladium(II) gegeben. Es wird für 30 min auf 120°C erhitzt; heiß über Kieselgur filtriert und das Filtrat eingeengt. Der erhaltene Rückstand wird mit 2 ml Acetonitril verrieben und der entstandene Niederschlag abgesaugt. Ausbeute: 1.9 mg (95%) 2-[2-Amino-4-(isoindolin-2-carbonyl)chinazolin-6-yl]-5-fluor-benzaldehyd; Retentionszeit HPLC: 2.74 min (Gradient "polar").

### 2-[2-Amino-4-(isoindolin-2-carbonyl)chinazolin-6-yl]-5-hydroxy-benzaldehyd

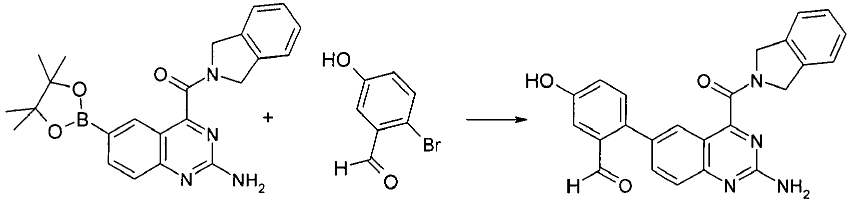

Zu einer Lösung von 150 mg [2-Amino-6-(4,4,5,5-tetramethyl-[1,3,2]dioxa-borolan-2-yl)-chinazolin-4-yl]-(1,3-dihydro-isoindol-2-yl)-methanon in 3 ml Ethanol unter Argon werden 72 mg 2-Bromo-5-hydroxy-benzaldehyd, 100 mg Kaliumcarbonat, 1 ml Wasser und 15 mg [1,1'-bis(diphenylphosphino)-ferrocen]dichlorpalladium(II) gegeben. Es wird für 30 min auf 120°C erhitzt; heiß über Kieselgur filtriert und das Filtrat eingeengt. Der erhaltene Rückstand wurde mit 2 ml Acetonitril verrieben und der entstandene Niederschlag abgesaugt. Ausbeute: 56 mg (38%) 2-[2-Amino-4-(isoindolin-2-carbonyl)-chinazolin-6-yl]-5-hydroxy-benzaldehyd; Retentionszeit HPLC: 1.67 min.

### 2-[2-Amino-4-(isoindolin-2-carbonyl)chinazolin-6-yl]-5-(2-dimethylaminoethoxy)benzaldehyd

### a)

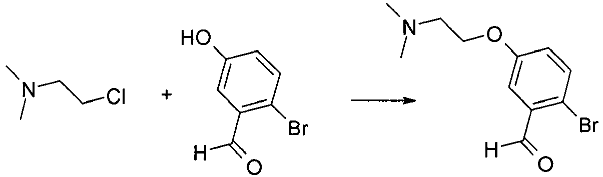

Zu einer Lösung von 200 mg 2-Brom-5-hydroxy-benzaldehyd und 648 mg Cäsiumcarbonat in 5 ml Toluol werden 143 mg (2-Chlor-ethyl)-dimethyl-amin Hydrochlorid zusammen mit 5 mg Tetrabutylammoniumiodid gegeben und 1 h unter Rückfluß erhitzt. Nach Abkühlen auf 22°C wird vom Rückstand abfiltriert, mit 5 ml Ethylacetat vesetzt und 3mal mit jeweils 5 ml 2N-Natronlauge gewaschen. Nach Trocknen über Natriumsulfat wird abfiltriert und im Vakuum zur Trockne eingeengt. Der Rückstand wird an der Umkehrphase säulenchromatographisch gereinigt.
Ausbeute: 116 mg (43%) 2-Bromo-5-(2-dimethylaminoethoxy)benzaldehyd; Retentionszeit HPLC: 1.18 min.

### b)

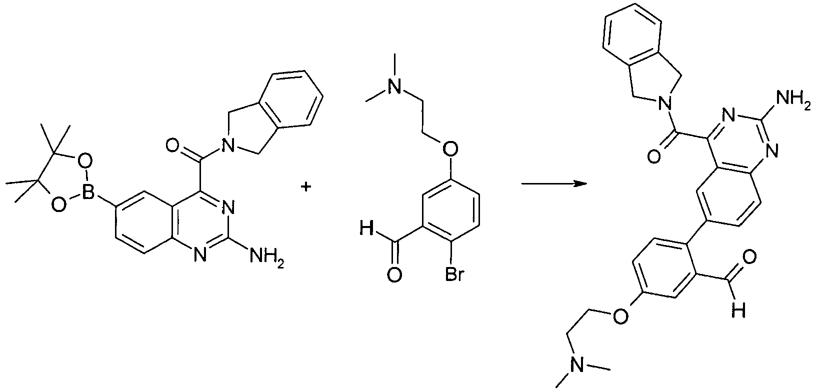

Zu einer Lösung von 150 mg [2-Amino-6-(4,4,5,5-tetramethyl-[1,3,2]dioxaborolan-2-yl)-chinazolin-4-yl]-(1,3-dihydro-isoindol-2-yl)-methanon in 3 ml Ethanol unter Argon werden 98 mg 2-Bromo-5-(2-dimethylaminoethoxy)-benzaldehyd, 100 mg Kaliumcarbonat, 1 ml Wasser und 15 mg [1,1'-bis(diphenylphosphino)ferrocen]dichlorpalladium(II) gegeben. Es wird für 30 min auf 120°C erhitzt; heiß über Kieselgur filtriert und das Filtrat eingeengt. Der erhaltene Rückstand wird mit 2 ml Acetonitril verrieben und der entstandene Niederschlag abgesaugt.
Ausbeute: 150 mg (86%) 2-[2-Amino-4-(isoindolin-2-carbonyl)chinazolin-6-yl]-5-(2-dimethylaminoethoxy)benzaldehyd; Retentionszeit HPLC: 1.37 min.

### 6-[2-Amino-4-(isoindolin-2-carbonyl)chinazolin-6-yl]-1,3-benzodioxol-5-carbaldehyd

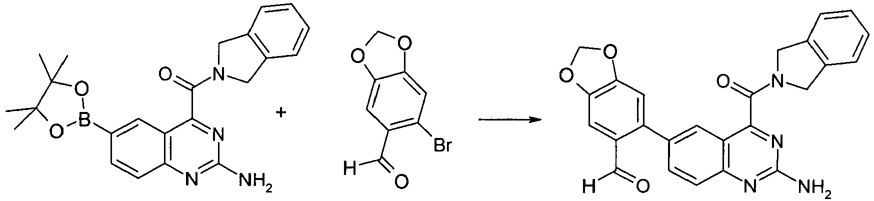

Zu einer Lösung von 500 mg [2-Amino-6-(4,4,5,5-tetramethyl-[1,3,2]dioxaborolan-2-yl)-chinazolin-4-yl]-(1,3-dihydro-isoindol-2-yl)-methanon in 50 ml Ethanol unter Argon werden 280 mg 6-Brom-1,3-benzodioxol-5-carbaldehyd, 331 mg Kaliumcarbonat, 22 µl Wasser und 49 mg [1,1'-bis(diphenylphosphino)ferrocen]dichlorpalladium(II) gegeben. Es wird für 4 h auf 120°C erhitzt; heiß über Kieselgur filtriert und das Filtrat eingeengt. Der erhaltene Rückstand wurde mit 2 ml Acetonitril verrieben und der entstandene Niederschlag abgesaugt.
Ausbeute: 370 mg (70%) 6-[2-Amino-4-(isoindolin-2-carbonyl)chinazolin-6-yl]-1,3-benzodioxol-5-carbaldehyd; Retentionszeit HPLC: 2.57 min (Gradient "polar");
¹H NMR (500 MHz, DMSO-d₆/TFA-d₁) δ [ppm] 9.64 (s, 1H), 8.11 - 8.06 (m, 2H), 7.82 (d, J = 8.4, 1 H), 7.44 (d, J = 7.3, 1 H), 7.40 (s, 1 H), 7.36 - 7.28 (m, 2H), 7.26 (d, J = 7.3, 1H), 7.11 (s, 1H), 6.21 (s, 2H), 5.00 (s, 2H), 4.85 (s, 2H).

### [2-Amino-6-[4-fluor-2-(hydroxymethyl)phenyl]chinazolin-4-yl]-isoindolin-2-yl-methanon ("A4") (Vergleichsbeispiel)

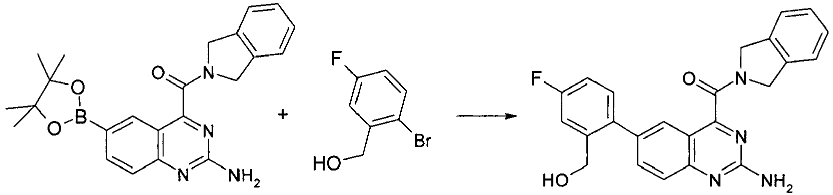

Zu einer Lösung von 150 mg [2-Amino-6-(4,4,5,5-tetramethyl-[1,3,2]dioxaborolan-2-yl)-chinazolin-4-yl]-(1,3-dihydro-isoindol-2-yl)-methanon in 3 ml Ethanol unter Argon werden 74 mg (2-Brom-5-fluor-phenyl)-methanol, 100 mg Kaliumcarbonat, 1 ml Wasser und 15 mg [1,1'-bis(diphenylphosphino)-ferrocen]dichlorpalladium(II) gegeben. Es wird für 30 min auf 120°C erhitzt; heiß über Kieselgur filtriert und das Filtrat im Vakuum eingeengt. Der Rückstand wird an der Umkehrphase säulenchromatographisch gereinigt. Ausbeute: 8 mg (5%) [2-Amino-6-[4-fluoro-2-(hydroxymethyl)phenyl]-chinazolin-4-yl]-isoindolin-2-yl-methanon; Retentionszeit HPLC: 1.51 min;
¹H NMR (400 MHz, DMSO-d₆/TFA-d₁) δ [ppm] 8.08 - 8.02 (m, 2H), 7.81 (d, J = 8.7, 1 H), 7.42 (d, J = 7.2, 1 H), 7.39 - 7.25 (m, 4H), 7.24 (d, J = 6.9, 1 H), 7.16 (td, J = 8.5, 2.8, 1H), 4.99 (s, 2H), 4.82 (s, 2H), 4.36 (s, 2H).

### [2-Amino-6-[5-fluor-2-(hydroxymethyl)phenyl]chinazolin-4-yl]-isoindolin-2-yl-methanon ("A5") (Vergleichsbeispiel)

### a)

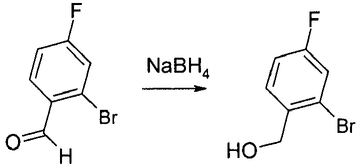

304 mg 2-Brom-4-fluorbenzaldehyd werden bei 22°C portionsweise unter Rühren zu einer Lösung von 28 mg Natriumborhydrid und 5 ml Methanol gegeben und 1 h bei 22°C gerührt. Man gießt auf Wasser, extrahiert dreimal mit Dichlormethan und trocknet die vereinigten organischen Phasen über Natriumsulfat. Nach Filtration wird das Filtrat zur Trockne eingeengt und chromatographisch (reversed phase HPLC) gereinigt.
Ausbeute 250 mg (81%) (2-Brom-4-fluor-phenyl)-methanol;
Retentionszeit LC-MS: 1,39 min.

### b)

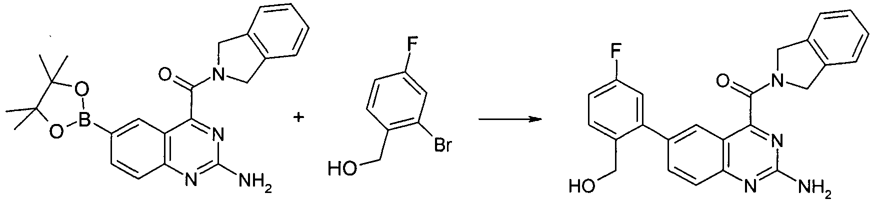

150 mg [2-Amino-6-(4,4,5,5-tetramethyl-[1,3,2]dioxaborolan-2-yl)-chinazolin-4-yl]-(1,3-dihydro-isoindol-2-yl)-methanon, 80 mg (2-Bromo-4-fluoro-phenyl)-methanol und 15 mg [1,1'-bis(diphenylphosphino)ferrocen]dichlorpalladium(II) werden in ein Mikrowellengläschen eingewogen und mit Argon inertisiert. Mit einer Spritze werden unter Argonatmosphäre durch das Septum 1 ml DMF und 500 µl 2N-Sodalösung gegeben. Anschließend wird über 30 min in der Mikrowelle bei 50 W und 120°C behandelt. Die entstehende olivgrüne Suspension wird in Ethylacetat aufgenommen und mit Wasser gewaschen, über Natriumsulfat getrocknet, filtriert und im Vakuum eingeengt. Der erhaltene Rückstand wird mit 2 ml Acetonitril verrieben und der entstandene Niederschlag abgesaugt. Ausbeute: 56 mg (38%) [2-Amino-6-[5-fluor-2-(hydroxymethyl)phenyl]chinazolin-4-yl]-isoindolin-2-yl-methanon; Retentionszeit HPLC: 1.51 min;
¹H NMR (500 MHz, DMSO-d₆/TFA-d₁) δ [ppm] 8.14 - 8.08 (m, 2H), 7.82 (dd, J = 7.8, 1.6, 1 H), 7.58 (dd, J = 8.6, 6.1, 1H), 7.41 (d, J = 7.2, 1 H), 7.33 - 7.25 (m, 2H), 7.24 - 7.18 (m, 2H), 7.13 (dd, J = 9.6, 2.7, 1 H), 4.99 (s, 2H), 4.82 (s, 2H), 4.31 (s, 2H).

### [2-Amino-6-[4,5-difluor-2-(hydroxymethyl)phenyl]chinazolin-4-yl]-isoindolin-2-yl-methanon ("A6")

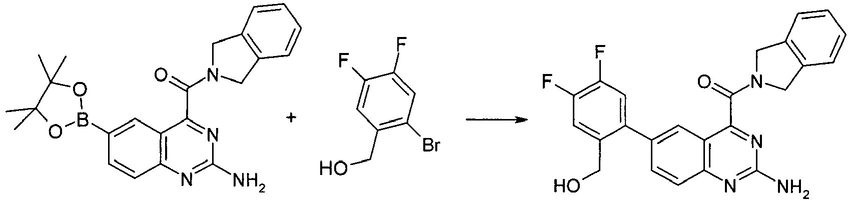

Zu einer Lösung von 100 mg [2-Amino-6-(4,4,5,5-tetramethyl-[1,3,2]dioxa-borolan-2-yl)-chinazolin-4-yl]-(1,3-dihydro-isoindol-2-yl)-methanon in 10 ml Ethanol unter Argon werden 43 mg (2-Brom-4,5-difluor-phenyl)-methanol, 80 mg Kaliumcarbonat, 4 µl Wasser und 16 mg [1,1'-bis(diphenylphosphino)-ferrocenldichlorpalladium(II) gegeben. Es wird für 30 min auf 120°C erhitzt; wobei sich ein Niederschlag bildet. Nach Abkühlen auf 22°C werden 10 ml Wasser dazugegeben und der Niederschlag abfiltriert. Der Rückstand wird 3mal mit je 25 ml Ethanol gewaschen und bei 40°C im Vakuum getrocknet. Ausbeute: 52 mg (63%) [2-Amino-6-[4,5-difluor-2-(hydroxymethyl)phenyl]-chinazolin-4-yl]-isoindolin-2-yl-methanon; Retentionszeit HPLC: 1.98 min;
¹H NMR (500 MHz, DMSO-d₆/TFA-d₁) δ [ppm] 8.09 - 8.04 (m, 2H), 7.83 - 7.79 (m, 1 H), 7.52 (dd, J = 11.8, 8.3, 1 H), 7.41 (d, J = 7.3, 1 H), 7.37 (dd, J = 11.1, 8.0, 1 H), 7.30 (dt, J = 14.9, 7.0, 2H), 7.23 (d, J = 7.3, 1 H), 4.99 (s, 2H), 4.82 (s, 2H), 4.31 (s, 2H).

### [2-Amino-6-[2-(2-dimethylaminoethoxymethyl)-4,5-difluor-phenyl]chinazolin-4-yl]-isoindolin-2-yl-methanon ("A8")

### a)

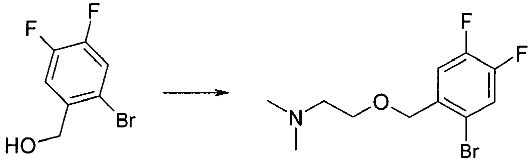

Zu einer Lösung von 100 mg (2-Brom-4,5-difluor-phenyl)-methanol und 78 mg (2-Chloro-ethyl)-dimethylamin Hydrochlorid in 3 ml Tetrahydrofuran werden 2 ml Natronlauge (32%ig) und 5 mg Tetrabutylammoniumiodid gegeben. Es wird 14 h bei 80°C gerührt und auf 22°C abgekühlt. Man wäscht 3mal mit 5 ml Ethylacetat, trocknet über Natriumsulfat, filtriert ab und engt das Filtrat im Vakuum zur Trockne ein. Das erhaltene Öl wird ohne weitere Reinigung in die nächste Stufe eingesetzt.
Ausbeute: 102 mg (77%) [2-(2-Brom-4,5-difluor-benzyloxy)-ethyl]-dimethylamin; Retentionszeit HPLC: 1.29 min

### b)

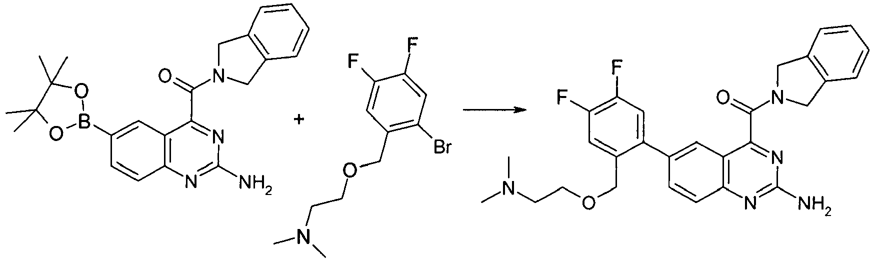

Zu einer Lösung von 150 mg [2-Amino-6-(4,4,5,5-tetramethyl-[1,3,2]dioxaborolan-2-yl)-chinazolin-4-yl]-(1,3-dihydro-isoindol-2-yl)-methanon in 2 ml Ethanol unter Argon werden 85 mg [2-(2-Brom-4,5-difluor-benzyloxy)-ethyl]-dimethyl-amin, 80 mg Kaliumcarbonat, 5 µl Wasser und 12 mg [1,1'-bis(diphenylphosphino)ferrocen]dichlorpalladium(II) gegeben. Es wird für 60 min auf 120°C erhitzt. Anschließend wird über Kieselgur abgesaugt und das Filtrat im Vakuum zur Trockne eingeengt. Der Rückstand wird in 1 ml DMSO gelöst und mittels präparativer HPLC (Fa. Agilent) chromatographiert. Die erhaltenen Produkt-Fraktionen wurden anschließend eingeengt und gefriergetrocknet.
Ausbeute: 54 mg (62%) [2-Amino-6-[2-(2-dimethylaminoethoxymethyl)-4,5-difluoro-phenyl]chinazolin-4-yl]-isoindolin-2-yl-methanon;
Retentionszeit HPLC: 1.67 min;
¹H NMR (500 MHz, DMSO-d₆/TFA-d₁) δ [ppm] 8.08 (dd, J = 6.4, 2.0, 2H), 7.88 - 7.83 (m, 1 H), 7.70 (dd, J = 11.4, 8.6, 1 H), 7.46 (dd, J = 11.1, 8.3, 2H), 7.34 (dt, J = 19.3, 6.9, 2H), 7.26 (d, J = 7.3, 1H), 5.04 (s, 2H), 4.85 (s, 2H), 4.38 (s, 2H), 3.67 - 3.60 (m, 2H), 3.33 - 3.25 (m, 2H), 2.80 (s, 6H).

### [2-Amino-6-[2-(3-dimethylaminopropoxymethyl)-4,5-difluor-phenyl]chinazolin-4-yl]-isoindolin-2-yl-methanon ("A9")

### a)

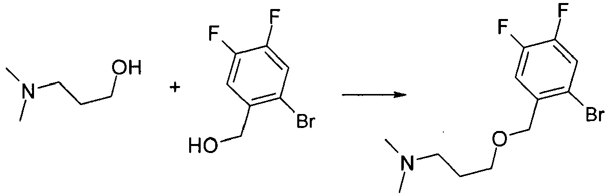

Zu einer Lösung von 200 mg (2-Brom-4,5-difluor-phenyl)-methanol und 170 mg (3-Chlor-propyl)-dimethylamin Hydrochlorid in 4 ml Tetrahydrofuran werden 4 ml Natronlauge (32%ig) und 5 mg Tetrabutylammoniumiodid gegeben. Es wird 14 h bei 80°C gerührt und auf 22°C abgekühlt. Man wäscht 3mal mit 5 ml Ethylacetat, trocknet über Natriumsulfat, filtriert ab und engt das Filtrat im Vakuum zur Trockne ein. Das erhaltene Öl wird ohne weitere Reinigung in die nächste Stufe eingesetzt.
Ausbeute: 230 mg (83%) [2-(2-Brom-4,5-difluor-benzyloxy)-ethyl]-dimethylamin; Retentionszeit HPLC: 1.32 min

### b)

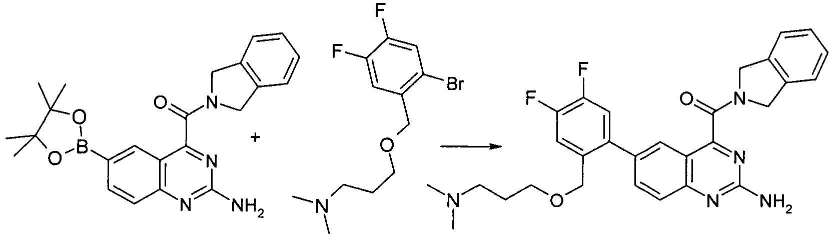

Zu einer Lösung von 150 mg [2-Amino-6-(4,4,5,5-tetramethyl-[1,3,2]dioxa-borolan-2-yl)-chinazolin-4-yl]-(1,3-dihydro-isoindol-2-yl)-methanon in 2 ml Ethanol unter Argon werden 88 mg [3-(2-Brom-4,5-difluor-benzyloxy)-propyl]-dimethyl-amin, 80 mg Kaliumcarbonat, 5 µl Wasser und 12 mg [1,1'-bis(diphenylphosphino)ferrocen]dichlorpalladium(II) gegeben. Es wird für 60 min auf 120°C erhitzt. Anschließend wird über Kieselgur abgesaugt und das Filtrat im Vakuum zur Trockne eingeengt. Der Rückstand wird in 1 ml DMSO gelöst und mittels präparativer HPLC (Fa. Agilent) chromatographiert. Die erhaltenen Produkt-Fraktionen werden anschließend eingeengt und gefriergetrocknet.
Ausbeute: 54 mg (62%) [2-Amino-6-[2-(2-dimethylaminoethoxymethyl)-4,5-difluor-phenyl]chinazolin-4-yl]-isoindolin-2-yl-methanon;
Retentionszeit HPLC: 1.67 min;
¹H NMR (500 MHz, DMSO-d₆/TFA-d₁) δ [ppm] 8.07 (s, 2H), 7.86 (d, J = 7.7, 1 H), 7.58 (s, 1 H), 7.45 (s, 2H), 7.40 - 7.29 (m, 2H), 7.27 (s, 1 H), 5.04 (s, 2H), 4.87 (s, 2H), 4.32 (s, 2H), 3.37 (t, 2H), 3.07 (t, 2H), 2.80 (s, 6H), 1.86 (m, 2H).

### Synthese von Benzylaminen via reduktiver Aminierung

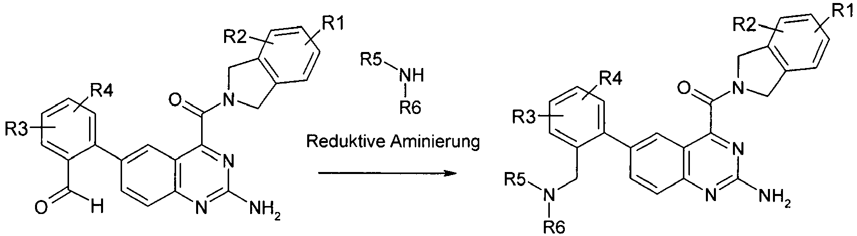

### (2-Amino-6-{2-[(ethyl-methyl-amino)-methyl]-phenyl}-chinazolin-4-yl)-(1,3-dihydro-isoindol-2-yl)-methanon ("A10") (Vergleichsbeispiel)

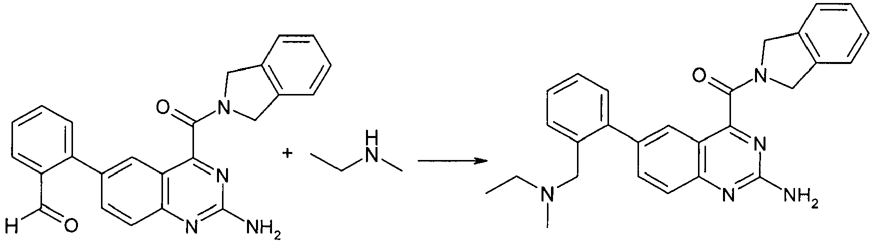

100 mg 2-[2-Amino-4-(1,3-dihydro-isoindol-2-carbonyl)-chinazolin-6-yl]-benz-aldehyd werden in 2 ml 1,2-Dichlorethan und 2 ml Tetrahydrofuran gelöst. Man gibt 44 µl Methylethylamin und 15 µl Eisessig dazu und rührt 6 h bei 60°C. Nach Abkühlen auf 25°C werden 170 mg Natriumtriacetoxyborhydrid zugegeben und weitere 12 h bei 25°C gerührt. Man gießt auf Wasser, extrahiert dreimal mit Dichlormethan und trocknet die vereinigten organischen Phasen über Natriumsulfat. Nach Filtration wird das Filtrat zur Trockne eingeengt und chromatographisch (reversed phase HPLC) gereinigt. Ausbeute: 36 mg (33%) (2-Amino-6-{2-[(ethyl-methyl-amino)-methyl]-phenyl}-chinazolin-4-yl)-(1,3-dihydro-isoindol-2-yl)-methanon;
Retentionszeit LC-MS: 1.41 min;
¹H NMR (500 MHz, DMSO-d₆/TFA-d₁) δ [ppm] 8.101 (s, 1 H), 8.050 (d, 1 H), 7.888 (d, 1 H), 7.708-7.554 (m, 1 H), 7.574-7.507 (m, 2H), 7.442-7.424 (m, 2H), 7.347 (t, 1 H), 7.285 (t, 1 H), 7.296 (d, 1 H), 5.028 (s, 2H), 4.356-4.163 (m, 2H), 3.056-2.794 (m, 2H), 2.508 (s, 3H), 0.992 (t, 3H).

### Synthese von Benzyl-Aminen via Alkylierung

### 1-Brom-2-chlormethyl-4,5-difluor-benzol

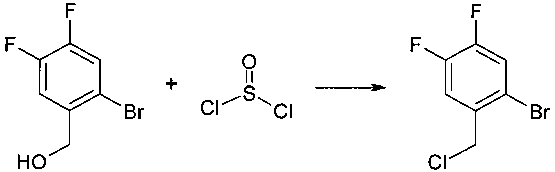

Zu 300 mg (2-Brom-4,5-difluor-phenyl)-methanol in 2.5 ml Dichlormethan werden 146 µl Thionylchlorid in 2.5 ml Dichlormethan getropft und 2 h bei 25°C gerührt. Man engt im Vakuum zur Trockne ein, nimmt in 10 ml Toluol auf und wiederholt den Vorgang. Der Rückstand wird ohne weitere Behandlung in die Folgereaktionen eingesetzt.
Ausbeute: 324 mg (99%) 1-Brom-2-chlormethyl-4,5-difluor-benzol; Retentionszeit HPLC: 3.37 min.

### (2-Brom-4,5-difluor-benzyl)-methylethylamin

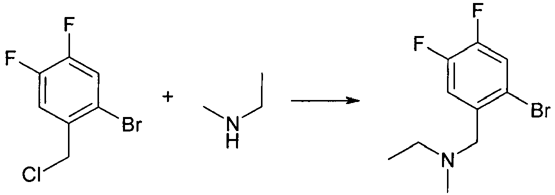

200 mg 1-Brom-2-chlormethyl-4,5-difluor-benzol werden in 5 ml Acetonitril gelöst. Man gibt 540 mg Cäsiumcarbonat und 146 µl Methylethylamin dazu und rührt 16 h bei 80°C. Nach Abkühlen auf 25°C wird filtriert und das Filtrat zur Trockne eingeengt. Man nimmt in 5 ml Ethylacetat auf, wäscht mit 5 ml 2N Natronlauge und trocknet die vereinigten organischen Phasen über Natriumsulfat. Nach Filtration wird das Filtrat zur Trockne eingeengt, worauf man das Produkt als Öl isoliert.
Ausbeute: 170 mg (78%) (2-Brom-4,5-difluor-benzyl)-methylethylamin; Retentionszeit LC-MS: 1.00 min.

### {2-Amino-6-[2-(methylethylamino-methyl)-4,5-difluor-phenyl]-chinazolin-4-yl}-(1,3-dihydro-isoindol-2-yl)-methanon ("A44")

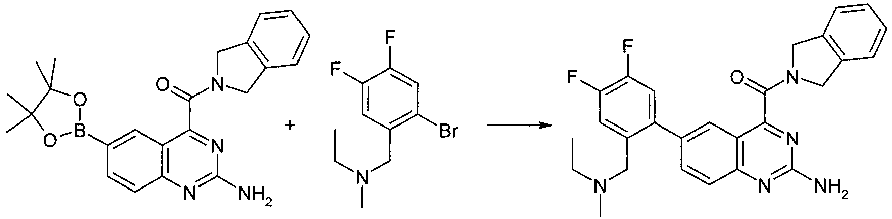

Zu einer Lösung von 200 mg [2-Amino-6-(4,4,5,5-tetramethyl-[1,3,2]dioxa-borolan-2-yl)-chinazolin-4-yl]-(1,3-dihydro-isoindol-2-yl)-methanon in 4 ml Ethanol unter Argon werden 152 mg (2-Brom-4,5-difluor-benzyl)-methyl-ethylamin, 133 mg Kaliumcarbonat, 9 µl Wasser und 20 mg [1,1'-bis(diphenylphosphino)ferrocen]dichlorpalladium(II) gegeben. Es wird für 30 min auf 120°C erhitzt; heiß über Kieselgur filtriert und das Filtrat eingeengt. und chromatographisch (reversed phase HPLC) gereinigt. Ausbeute: 83 mg (37%) {2-Amino-6-[2-(methylethylamino-methyl)-4,5-difluor-phenyl]-chinazolin-4-yl}-(1,3-dihydro-isoindol-2-yl)-methanon; Retentionszeit LC-MS: 1.59 min;
¹H NMR (500 MHz, DMSO-d₆/TFA-d₁) δ [ppm] 8.12 (d, J = 1.9, 1H), 8.04 (dd, J = 1.9, 8.7, 1 H), 7.93 - 7.84 (m, 2H), 7.55 (dd, J = 8.1, 10.9, 1H), 7.45 (d, J = 7.4, 1 H), 7.35 (t, J = 7.2, 1 H), 7.31 (t, J = 7.2, 1 H), 7.25 (d, J = 7.4, 1 H), 5.04 (s, 2H), 4.85 (s, 2H), 4.22 (s, 2H), 3.07 - 2.92 (m, 2H), 2.91 - 2.79 (m, 2H), 1.01 (t, J = 7.2, 6H).

### (2-Brom-4,5-difluor-benzyl)-diethylamin

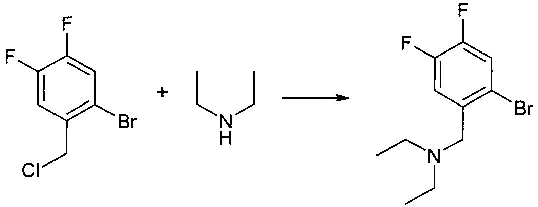

200 mg 1-Brom-2-chlormethyl-4,5-difluor-benzol werden in 5 ml Acetonitril gelöst. Man gibt 540 mg Cäsiumcarbonat und 132 µl Diethylamin dazu und rührt 16 h bei 80°C. Nach Abkühlen auf 25°C wird filtriert und das Filtrat zur Trockne eingeengt. Man nimmt in 5 ml Ethylacetat auf, wäscht mit 5 ml 2N Natronlauge und trocknet die vereinigten organischen Phasen über Natriumsulfat. Nach Filtration wird das Filtrat zur Trockne eingeengt, worauf man das Produkt als Öl isoliert. Ausbeute: 190 mg (83%) (2-Brom-4,5-difluor-benzyl)-diethylamin; Retentionszeit LC-MS: 1.59 min.

### {2-Amino-6-[2-(diethylamino-methyl)-4,5-difluor-phenyl]-chinazolin-4-yl}-(1,3-dihydro-isoindol-2-yl)-methanon ("A45")

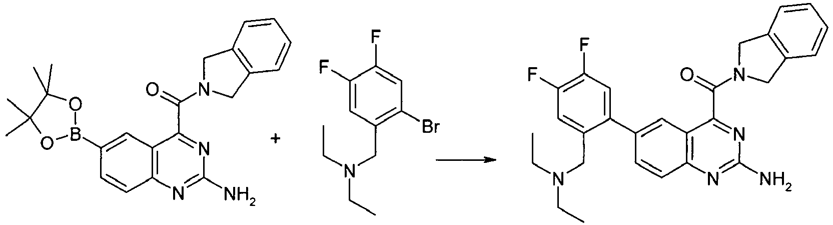

Zu einer Lösung von 170 mg [2-Amino-6-(4,4,5,5-tetramethyl-[1,3,2]dioxaborolan-2-yl)-chinazolin-4-yl]-(1,3-dihydro-isoindol-2-yl)-methanon in 4 ml Ethanol unter Argon werden 148 mg (2-Brom-4,5-difluor-benzyl)-diethylamin, 170 mg Kaliumcarbonat, 7 µl Wasser und 17 mg [1,1'-bis(diphenylphosphino)ferrocen]dichlorpalladium(II) gegeben. Es wird für 30 min auf 120°C erhitzt; heiß über Kieselgur filtriert und das Filtrat eingeengt und chromatographisch (reversed phase HPLC) gereinigt.
Ausbeute: 40 mg (20%) {2-Amino-6-[2-(diethylamino-methyl)-4,5-difluorphenyl]-chinazolin-4-yl}-(1,3-dihydro-isoindol-2-yl)-methanon;
Retentionszeit LC-MS: 1.56 min;
¹H NMR (500 MHz, DMSO-d₆/TFA-d₁) δ [ppm] 8.12 (d, J = 1.9, 1H), 8.04 (dd, J = 1.9, 8.7, 1 H), 7.93 - 7.84 (m, 2H), 7.55 (dd, J = 8.1, 10.9, 1H), 7.45 (d, J = 7.4, 1 H), 7.35 (t, J = 7.2, 1 H), 7.31 (t, J = 7.2, 1 H), 7.25 (d, J = 7.4, 1 H), 5.04 (s, 2H), 4.85 (s, 2H), 4.22 (s, 2H), 3.07 - 2.92 (m, 2H), 2.91 - 2.79 (m, 2H), 1.01 (t, J = 7.2, 6H).

### (2-Brom-4,5-difluor-benzyl)-tert.-butylamin

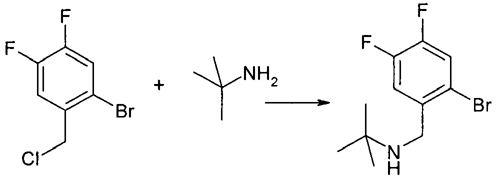

200 mg 1-Brom-2-chlormethyl-4,5-difluor-benzol werden in 5 ml Acetonitril gelöst. Man gibt 540 mg Cäsiumcarbonat und 123 µl tert.-Butylamin dazu und rührt 16 h bei 80°C. Nach Abkühlen auf 25°C wird filtriert und das Filtrat zur Trockne eingeengt. Man nimmt in 5 ml Ethylacetat auf, wäscht mit 5 ml 2N Natronlauge und trocknet die vereinigten organischen Phasen über Natriumsulfat. Nach Filtration wird das Filtrat zur Trockne eingeengt, worauf man das Produkt als Öl isoliert.
Ausbeute: 210 mg (91%) (2-Brom-4,5-difluor-benzyl)-tert.-butylamin; Retentionszeit LC-MS: 1.17 min.

### {2-Amino-6-[2-(tert.-butylamino-methyl)-4,5-difluor-phenyl]-chinazolin-4-yl}-(1,3-dihydro-isoindol-2-yl)-methanon ("A46")

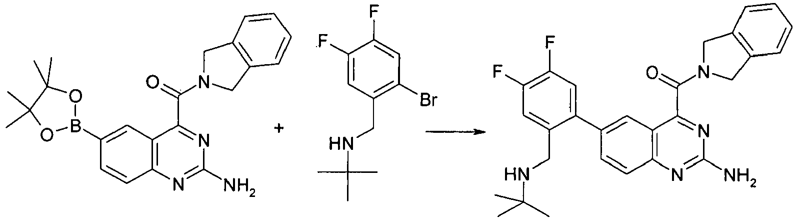

Zu einer Lösung von 250 mg [2-Amino-6-(4,4,5,5-tetramethyl-[1,3,2]dioxa-borolan-2-yl)-chinazolin-4-yl]-(1,3-dihydro-isoindol-2-yl)-methanon in 10 ml Ethanol unter Argon werden 184 mg (2-Brom-4,5-difluor-benzyl)-tert.-butylamin, 166 mg Kaliumcarbonat, 11 µl Wasser und 25 mg [1,1'-bis(diphenylphosphino)ferrocen]dichlorpalladium(II) gegeben. Es wird für 30 min auf 120°C erhitzt; heiß über Kieselgur filtriert und das Filtrat eingeengt. und chromatographisch (reversed phase HPLC) gereinigt.
Ausbeute: 90 mg (31%) {2-Amino-6-[2-(tert.-butylamino-methyl)-4,5-difluorphenyl]-chinazolin-4-yl}-(1,3-dihydro-isoindol-2-yl)-methanon;
Retentionszeit LC-MS: 1.55 min;
¹H NMR (500 MHz, DMSO-d₆/TFA-d₁) δ [ppm] 8.16 (d, J = 1.8, 1H), 8.07 (dd, J = 1.7, 8.7, 1 H), 7.92 - 7.81 (m, 2H), 7.51 (dd, J = 8.1, 10.9, 1H), 7.45 (d, J = 7.4, 1 H), 7.36 (dd, J = 5.1, 9.2, 1 H), 7.31 (t, J = 7.4, 1H), 7.24 (d, J = 7.4, 1 H), 5.06 (s, 2H), 4.84 (s, 2H), 3.99 (s, 2H), 1.09 (s, 9H).

### (2-Brom-4,5-difluor-benzyl)-tert.-butyl-methyl-amin

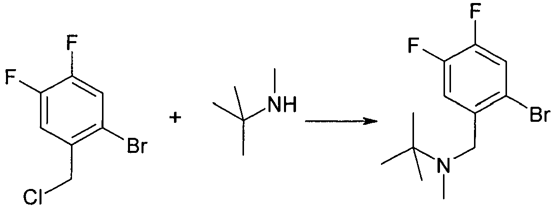

200 mg 1-Brom-2-chlormethyl-4,5-difluor-benzol werden in 5 ml Acetonitril gelöst. Man gibt 540 mg Cäsiumcarbonat und 206 µl tert.-Butylmethylamin dazu und rührt 16 h bei 80°C. Nach Abkühlen auf 25°C wird filtriert und das Filtrat zur Trockne eingeengt. Man nimmt in 5 ml Ethylacetat auf, wäscht mit 5 ml 2N Natronlauge und trocknet die vereinigten organischen Phasen über Natriumsulfat. Nach Filtration wird das Filtrat zur Trockne eingeengt, worauf man das Produkt als Öl isoliert.
Ausbeute: 210 mg (87%) (2-Brom-4,5-difluor-benzyl)-tert.-butyl-methyl-amin; Retentionszeit LC-MS: 1.16 min.

### (2-Amino-6-{2-[(tert.-butyl-methyl-amino)-methyl]-4,5-difluor-phenyl}-chinazolin-4-yl)-(1,3-dihydro-isoindol-2-yl)-methanon ("A47")

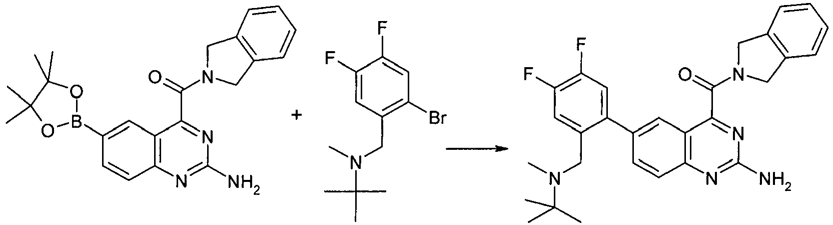

Zu einer Lösung von 150 mg [2-Amino-6-(4,4,5,5-tetramethyl-[1,3,2]dioxaborolan-2-yl)-chinazolin-4-yl]-(1,3-dihydro-isoindol-2-yl)-methanon in 4 ml Ethanol unter Argon werden 171 mg (2-Bromo-4,5-difluor-benzyl)-tert.-butylmethyl-amin, 124 mg Kaliumcarbonat, 9 µl Wasser und 22 mg [1,1'-bis(diphenylphosphino)ferrocen]dichlorpalladium(II) gegeben. Es wird für 30 min auf 120°C erhitzt; heiß über Kieselgur filtriert und das Filtrat eingeengt. und chromatographisch (reversed phase HPLC) gereinigt.
Ausbeute: 20 mg (9%) (2-Amino-6-{2-[(tert.-butyl-methyl-amino)-methyl]-4,5-difluor-phenyl}-chinazolin-4-yl)-(1,3-dihydro-isoindol-2-yl)-methanon; Retentionszeit LC-MS: 2.22 min.

### 1-(2-Brom-4,5-difluor-benzyl)-pyrrolidin

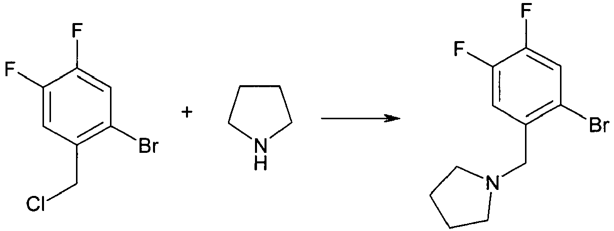

200 mg 1-Brom-2-chlormethyl-4,5-difluor-benzol werden in 5 ml Acetonitril gelöst. Man gibt 540 mg Cäsiumcarbonat und 102 µl Pyrrolidin dazu und rührt 16 h bei 80°C. Nach Abkühlen auf 25°C wird filtriert und das Filtrat zur Trockne eingeengt. Man nimmt in 5 ml Ethylacetat auf, wäscht mit 5 ml 2N Natronlauge und trocknet die vereinigten organischen Phasen über Natriumsulfat. Nach Filtration wird das Filtrat zur Trockne eingeengt, worauf man das Produkt als Öl isoliert. Ausbeute: 190 mg (83%) 1-(2-Brom-4,5-difluor-benzyl)-pyrrolidin; Retentionszeit LC-MS: 1.09 min.

### [2-Amino-6-(4,5-difluor-2-pyrrolidin-1-ylmethyl-phenyl)-chinazolin-4-yl]-(1,3-dihydro-isoindol-2-yl)-methanon ("A48")

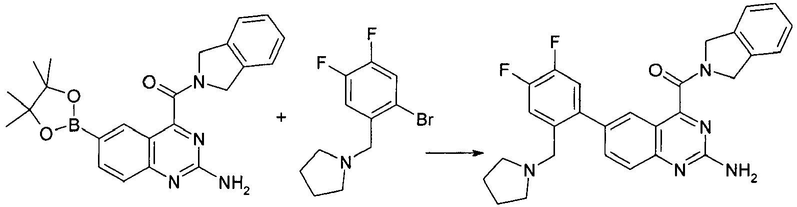

Zu einer Lösung von 150 mg [2-Amino-6-(4,4,5,5-tetramethyl-[1,3,2]dioxaborolan-2-yl)-chinazolin-4-yl]-(1,3-dihydro-isoindol-2-yl)-methanon in 4 ml Ethanol unter Argon werden 129 mg 1-(2-Brom-4,5-difluor-benzyl)-pyrrolidin, 100 mg Kaliumcarbonat, 7 µl Wasser und 17 mg [1,1'-bis(diphenylphosphino)-ferrocen]dichlorpalladium(II) gegeben. Es wird für 30 min auf 120°C erhitzt; heiß über Kieselgur filtriert und das Filtrat eingeengt und chromatographisch (reversed phase HPLC) gereinigt.
Ausbeute: 90 mg (31%) [2-Amino-6-(4,5-difluor-2-pyrrolidin-1-ylmethylphenyl)-chinazolin-4-yl]-(1,3-dihydro-isoindol-2-yl)-methanon;
Retentionszeit LC-MS: 1.60 min;
¹H NMR (500 MHz, DMSO-d₆/TFA-d₁) δ [ppm] 8.11 (d, J = 1.7, 1H), 8.03 (d, J = 8.7, 1 H), 7.87 (dd, J = 8.4, 22.5, 2H), 7.54 (s, 1 H), 7.45 (d, J = 7.5, 1 H), 7.33 (dt, J = 7.2, 14.8, 2H), 7.25 (d, J = 7.4, 1 H), 5.03 (s, 2H), 4.84 (s, 2H), 4.28 (s, 2H), 3.41 (s, 2H), 2.77 (s, 2H), 1.79 (s, 4H).

### 1-(2-Brom-4,5-difluor-benzyl)-2-methyl-pyrrolidin

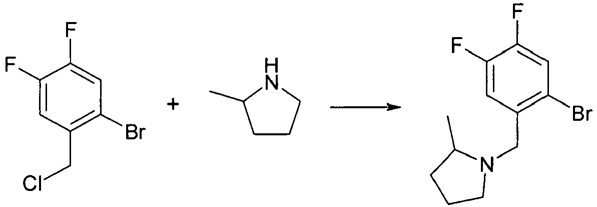

200 mg 1-Brom-2-chlormethyl-4,5-difluor-benzol werden in 5 ml Acetonitril gelöst. Man gibt 540 mg Cäsiumcarbonat und 136 µl 2-Methyl-pyrrolidin dazu und rührt 16 h bei 80°C. Nach Abkühlen auf 25°C wird filtriert und das Filtrat zur Trockne eingeengt. Man nimmt in 5 ml Ethylacetat auf, wäscht mit 5 ml 2N Natronlauge und trocknet die vereinigten organischen Phasen über Natriumsulfat. Nach Filtration wird das Filtrat zur Trockne eingeengt, worauf man das Produkt als Öl isoliert. Ausbeute: 190 mg (80%) 1-(2-Brom-4,5-difluor-benzyl)-2-methyl-pyrrolidin; Retentionszeit LC-MS: 1.09 min.

### {2-Amino-6-[4,5-difluor-2-(2-methyl-pyrrolidin-1-ylmethyl)-phenyl]-chinazolin-4-yl}-(1,3-dihydro-isoindol-2-yl)-methanon ("A49")

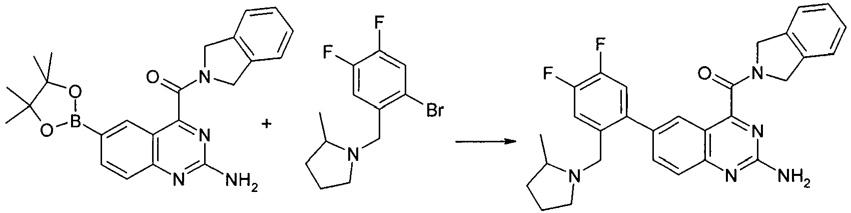

Zu einer Lösung von 150 mg [2-Amino-6-(4,4,5,5-tetramethyl-[1,3,2]dioxaborolan-2-yl)-chinazolin-4-yl]-(1,3-dihydro-isoindol-2-yl)-methanon in 4 ml Ethanol unter Argon werden 136 mg 1-(2-Brom-4,5-difluor-benzyl)-2-methylpyrrolidin, 100 mg Kaliumcarbonat, 7 µl Wasser und 17 mg [1,1'-bis(diphenylphosphino)ferrocen]dichlorpalladium(II) gegeben. Es wird für 30 min auf 120°C erhitzt; heiß über Kieselgur filtriert und das Filtrat eingeengt und chromatographisch (reversed phase HPLC) gereinigt.
Ausbeute: 25 mg (14%) {2-Amino-6-[4,5-difluor-2-(2-methyl-pyrrolidin-1-ylmethyl)-phenyl]-chinazolin-4-yl}-(1,3-dihydro-isoindol-2-yl)-methanon; Retentionszeit LC-MS: 1.65 min;
¹H NMR (500 MHz, DMSO-d₆/TFA-d₁) δ [ppm] 8.10 (d, J = 1.7, 1H), 8.05 (dd, J = 1.9, 8.6, 1H), 7.92 - 7.83 (m, 2H), 7.55 (dd, J = 8.1, 10.9, 1H), 7.47 (d, J = 7.5, 1H), 7.38 - 7.28 (m, 2H), 7.27 (d, J = 7.4, 1 H), 5.10 - 4.96 (m, 2H), 4.90 (d, J = 12.2, 2H), 4.58 (d, J = 13.9, 1 H), 4.12 (d, J = 13.9, 1 H), 3.41 - 3.20 (m, 2H), 2.85 (dd, J = 8.8, 20.0, 1 H), 2.12 - 1.97 (m, 1H), 1.80 - 1.68 (m, 2H), 1.62 - 1.40 (m, 1 H), 1.17 (d, J = 6.4, 3H).

### 1-(2-Brom-4,5-difluor-benzyl)-2,5-dimethyl-pyrrolidin

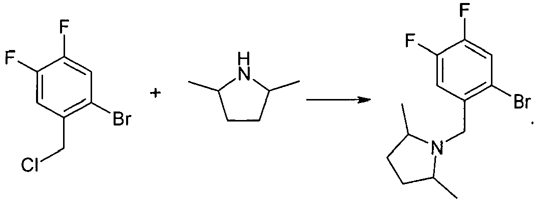

200 mg 1-Brom-2-chlormethyl-4,5-difluor-benzol werden in 5 ml Acetonitril gelöst. Man gibt 540 mg Cäsiumcarbonat und 152 µl 2,5-Dimethyl-pyrrolidine dazu und rührt 16 h bei 80°C. Nach Abkühlen auf 25°C wird filtriert und das Filtrat zur Trockne eingeengt. Man nimmt in 5 ml Ethylacetat auf, wäscht mit 5 ml 2N Natronlauge und trocknet die vereinigten organischen Phasen über Natriumsulfat. Nach Filtration wird das Filtrat zur Trockne eingeengt, worauf man das Produkt als Öl isoliert.
Ausbeute: 210 mg (83%) 1-(2-Brom-4,5-difluor-benzyl)-2,5-dimethyl-pyrrolidin; Retentionszeit LC-MS: 1.19 min.

### {2-Amino-6-[2-(2,5-dimethyl-pyrrolidin-1-ylmethyl)-4,5-difluor-phenyl]-chinazolin-4-yl}-(1,3-dihydro-isoindol-2-yl)-methanon ("A50")

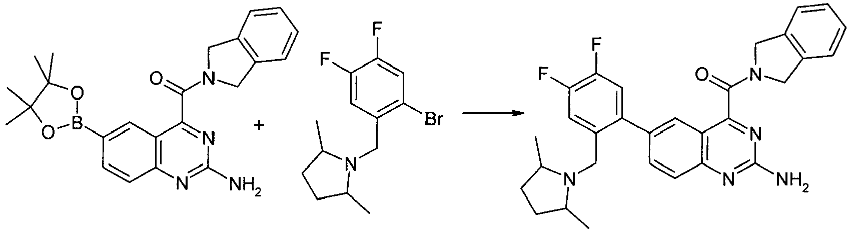

Zu einer Lösung von 150 mg [2-Amino-6-(4,4,5,5-tetramethyl-[1,3,2]dioxaborolan-2-yl)-chinazolin-4-yl]-(1,3-dihydro-isoindol-2-yl)-methanon in 4 ml Ethanol unter Argon werden 143 mg 1-(2-Brom-4,5-difluor-benzyl)-2,5-dimethyl-pyrrolidin, 100 mg Kaliumcarbonat, 7 µl Wasser und 17 mg [1,1'-bis(diphenylphosphino)ferrocen]dichlorpalladium(II) gegeben. Es wird für 30 min auf 120°C erhitzt; heiß über Kieselgur filtriert und das Filtrat eingeengt. und chromatographisch (reversed phase HPLC) gereinigt.
Ausbeute: 40 mg (22%) {2-Amino-6-[2-(2,5-dimethyl-pyrrolidin-1-ylmethyl)-4,5-difluor-phenyl]-chinazolin-4-yl}-(1,3-dihydro-isoindol-2-yl)-methanon; Retentionszeit LC-MS: 1.65 min;
¹H NMR (500 MHz, DMSO-d₆/TFA-d₁) δ [ppm] 8.07 (d, J = 1.6, 1H), 8.05 (d, J = 8.8, 1 H), 7.92 (d, J = 8.6, 1 H), 7.89 (dd, J = 8.2, 11.1, 1 H), 7.59 - 7.51 (m, 1 H), 7.45 (d, J = 7.3, 1 H), 7.38 (dt, J = 7.2, 18.5, 2H), 7.27 (d, J = 7.0, 1 H), 5.03 (s, 2H), 4.89 (s, 2H), 4.38 (s, 2H), 3.32 (q, J = 6.1, 12.4, 2H), 2.02 - 1.89 (m, 2H), 1.57 - 1.45 (m, 2H), 1.05 (d, J = 6.5, 6H).

### [2-Amino-6-(2-chlormethyl-4,5-difluor-phenyl)-chinazolin-4-yl]-(1,3-dihydroisoindol-2-yl)-methanon

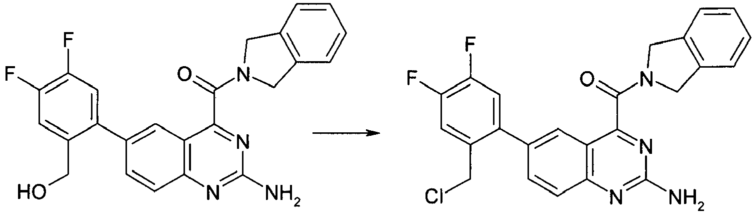

Zu 200 mg [2-Amino-6-(2-hydroxymethyl-4,5-difluor-phenyl)-chinazolin-4-yl]-(1,3-dihydro-isoindol-2-yl)-methanon in 2 ml Dichlormethan werden 67 µl Thionylchlorid in 2 ml Dichlormethan getropft und 2 h bei 25°C gerührt. Man engt im Vakuum zur Trockne ein, nimmt in 10 ml Toluol auf und wiederholt den Vorgang. Der Rückstand wird ohne weitere Behandlung in die Folgereaktionen eingesetzt.
Ausbeute: 198 mg (95%) [2-Amino-6-(2-chlormethyl-4,5-difluor-phenyl)-chinazolin-4-yl]-(1,3-dihydro-isoindol-2-yl)-methanon;
Retentionszeit LC-MS: 2.31 min.

### {2-Amino-6-[4,5-difluor-2-(4-methyl-piperazin-1-ylmethyl)-phenyl]-chinazolin-4-yl}-(1,3-dihydro-isoindol-2-yl)-methanon ("A52")

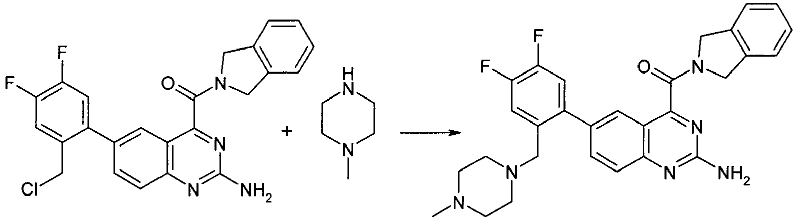

200 mg [2-Amino-6-(2-chlormethyl-4,5-difluor-phenyl)-chinazolin-4-yl]-(1,3-dihydro-isoindol-2-yl)-methanon werden in 5 ml Acetonitril gelöst. Man gibt 289 mg Cäsiumcarbonat und 84 µl Methylpiperazin dazu und rührt 16 h bei 80°C. Nach Abkühlen auf 25°C wird filtriert und das Filtrat zur Trockne eingeengt. Man nimmt in 5 ml Ethylacetat auf, wäscht mit 5 ml 2N Natronlauge und trocknet die vereinigten organischen Phasen über Natriumsulfat. Nach Filtration wird das Filtrat zur Trockne eingeengt und chromatographisch (reversed phase HPLC) gereinigt.
Ausbeute: 14 mg (6%) {2-Amino-6-[4,5-difluor-2-(4-methyl-piperazin-1-ylmethyl)-phenyl]-chinazolin-4-yl}-(1,3-dihydro-isoindol-2-yl)-methanon; Retentionszeit LC-MS: 1.69 min;
¹H NMR (400 MHz, DMSO-d₆/TFA-d₁) δ [ppm] 8.10 (d, J = 1.6, 1H), 8.06 (dd, J = 1.8, 8.6, 1 H), 7.85 (d, J = 8.7, 1 H), 7.76 (dd, J = 8.3, 11.4, 1 H), 7.52 (dd, J = 8.1, 11.1, 1H), 7.45 (d, J = 7.1, 1H), 7.34 (td, J = 6.9, 13.2, 2H), 7.26 (d, J = 6.9, 1 H), 5.03 (s, 2H), 4.82 (s, 2H), 3.96 (s, 2H), 3.53 - 3.00 (m, 9H), 2.86 (s, 3H).

### {2-[2-Amino-4-(1,3-dihydro-isoindol-2-carbonyl)-chinazolin-6-yl]-4,5-difluorbenzyl}-bis-carbaminsäure-tert.-butylester ("A53")

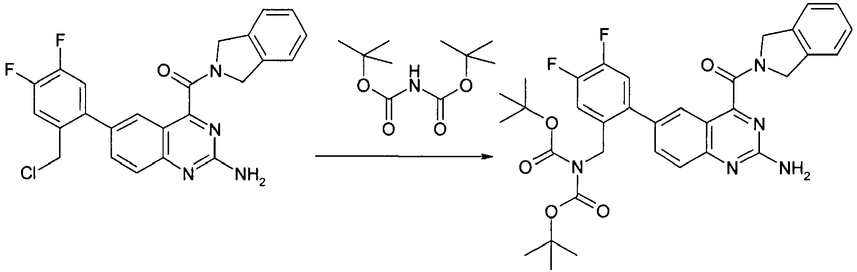

190 mg [2-Amino-6-(2-chlormethyl-4,5-difluor-phenyl)-chinazolin-4-yl]-(1,3-dihydro-isoindol-2-yl)-methanon werden in 2 ml Ethylmethylketon gelöst. Man gibt 381 mg Cäsiumcarbonat, 4 mg Lithiumiodid und 85 mgl Di-tert.-butylimino-dicarboxylat dazu und rührt 3 h bei 70°C. Der. Niederschlag wird filriert, mit Ethylacetat gewaschen und die Mutterlauge eingeengt. Das entstehende Öl wird in Ethylacetat aufgenommen und mit gesättigter Natriumhydrogencarbonat-Lösung gewaschen, bevor man die vereinigten organischen Phasen über Natriumsulfat trocknet. Nach Filtration wird das Filtrat zur Trockne eingeengt.
Ausbeute: 250 mg (84%) {2-[2-Amino-4-(1,3-dihydro-isoindol-2-carbonyl)-chinazolin-6-yl]-4,5-difluor-benzyl}-bis-carbaminsäure-tert.-butylester;
Retentionszeit LC-MS: 2.49 min (Methode unpolar).

### [2-Amino-6-(2-aminomethyl-4,5-difluor-phenyl)-chinazolin-4-yl]-(1,3-dihydroisoindol-2-yl)-methanon ("A54")

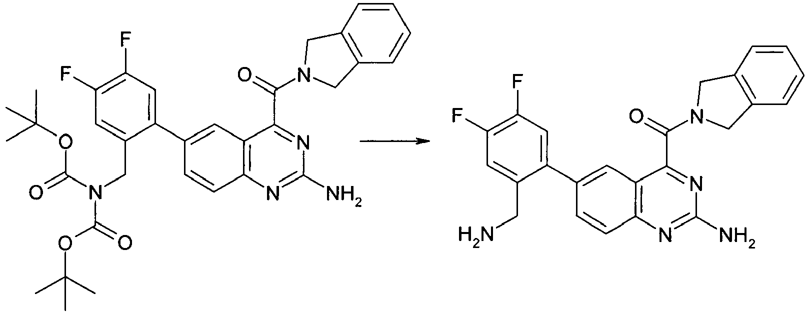

250 mg {2-[2-Amino-4-(1,3-dihydro-isoindol-2-carbonyl)-chinazolin-6-yl]-4,5-difluor-benzyl}-bis-carbaminsäure-tert.-butylester werden in 4 ml Dioxan/HCl (1 M) gelöst. Man rührt 1 h bei 25°C und engt im Vakuum zur Trockne ein. Ausbeute: 190 mg (100%) [2-Amino-6-(2-aminomethyl-4,5-difluor-phenyl)-chinazolin-4-yl]-(1,3-dihydro-isoindol-2-yl)-methanon;
Retentionszeit LC-MS: 1.55 min;
¹H NMR (400 MHz, DMSO-d₆/TFA-d₁) δ [ppm] 8.08 (dd, J = 1.6, 10.1, 2H), 7.85 (d, J = 8.5, 1 H), 7.50 - 7.40 (m, 3H), 7.33 (s, 2H), 7.26 (d, J = 7.2, 1 H), 5.02 (s, 2H), 4.83 (s, 2H), 4.14 (s, 2H).

### 3-Amino-N-{2-[2-amino-4-(1,3-dihydro-isoindol-2-carbonyl)-chinazolin-6-yl]-4,5-difluor-benzyl}-propionamid Hydrochlorid ("A55")

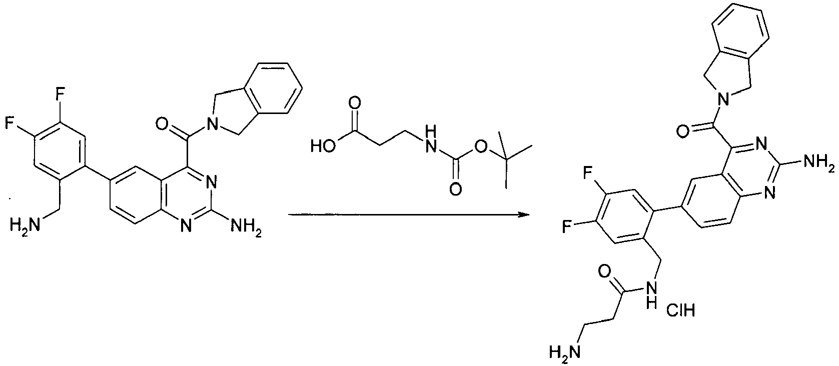

Zu einer Lösung von 46 mg 3-tert.-Butoxycarbonylamino-propioncarbonsäure in 1 ml DMF werden 85 mg TBTU (O-(Benzotriazol-1-yl)-N,N,N',N'-tetra-methyluronium Tetrafluoroborat), 112 µl 4-Methylmorpholin und 88 mg [2-Amino-6-(2-aminomethyl-4,5-difluor-phenyl)-chinazolin-4-yl]-(1,3-dihydro-isoindol-2-yl)-methanon gegeben. Es wird 16 h bei 22°C gerührt und das Produkt direkt säulenchromatographisch isoliert.
Ausbeute: 25 mg (23%) 3-Amino-N-{2-[2-amino-4-(1,3-dihydro-isoindol-2-carbonyl)-chinazolin-6-yl]-4,5-difluor-benzyl}-propionamid Hydrochlorid; Retentionszeit LC-MS: 1.59 min;
¹H NMR (400 MHz, DMSO-d₆/TFA-d₁) δ [ppm] 8.12 - 8.04 (m, 2H), 7.85 (d, J = 8.5, 1 H), 7.50 - 7.39 (m, 3H), 7.39 - 7.29 (m, 2H), 7.26 (d, J = 7.2, 1H), 5.02 (s, 2H), 4.83 (s, 2H), 4.14 (s, 2H), 2.94 (t, J = 6.8, 2H), 2.48 (d, J = 6.7, 2H).

### N-{2-[2-Amino-4-(1,3-dihydro-isoindol-2-carbonyl)-chinazolin-6-yl]-4,5-difluorbenzyl}-3-dimethylamino-propionamid ("A56")

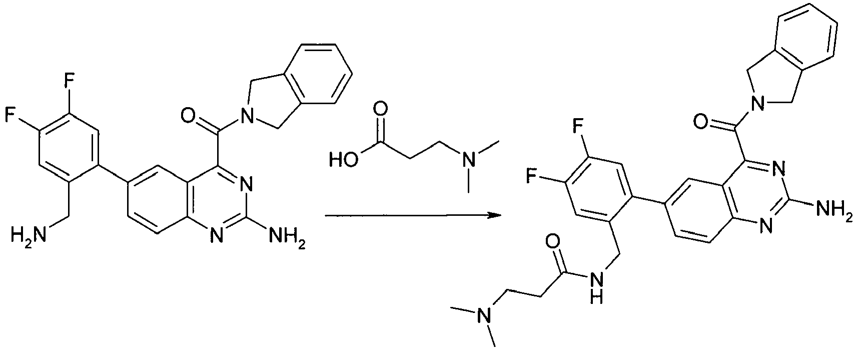

Zu einer Lösung von 43 mg 3-Dimethylamino-propioncarbonsäure Hydrochlorid in 1 ml DMF werden 85 mg TBTU (O-(Benzotriazol-1-yl)-N,N,N',N'-tetramethyluronium Tetrafluoroborat), 112 µl 4-Methylmorpholin und 100 mg [2-Amino-6-(2-aminomethyl-4,5-difluor-phenyl)-chinazolin-4-yl]-(1,3-dihydroisoindol-2-yl)-methanon gegeben. Es wird 16 h bei 22°C gerührt und das Produkt direkt säulenchromatographisch isoliert.
Ausbeute: 25 mg (23%) N-{2-[2-Amino-4-(1,3-dihydro-isoindol-2-carbonyl)-chinazolin-6-yl]-4,5-difluor-benzyl}-3-dimethylamino-propionamid; Retentionszeit LC-MS: 1.60 min;
¹H NMR (500 MHz, DMSO-d₆/TFA-d₁) δ [ppm] 8.08 (dd, J = 1.7, 7.6, 2H), 7.86 (d, J = 9.4, 1H), 7.44 (ddd, J = 8.1, 11.5, 19.1, 3H), 7.34 (dt, J = 7.2, 18.5, 2H), 7.26 (d, J = 7.5, 1 H), 5.04 (s, 2H), 4.85 (s, 2H), 4.15 (s, 2H), 3.24 (t, J = 7.1, 2H), 2.72 (s, 3H), 2.60 (t, J = 4.7, 2H), 2.11 (s, 3H).

### {2-Amino-6-[4,5-difluor-2-(2-hydroxy-ethoxymethyl)-phenyl]-chinazolin-4-yl}-(1,3-dihydro-isoindol-2-yl)-methanon ("A59")

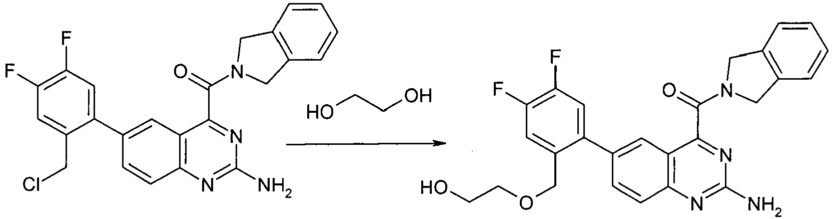

Man gibt 10 mg Natriumhydrid (60%ige Natriumhydrid Suspension in Paraffinöl) portionsweise zu 14 µl Ethylenglycol in 2 ml THF und rührt 1 h bei 25°C. Zu dieser Lösung tropft man eine Lösung aus 100 mg [2-Amino-6-(2-chlormethyl-5-fluor-phenyl)-chinazolin-4-yl]-(1,3-dihydro-isoindol-2-yl)-methanon zusammen mit 101 µl Triethylamin in 2 ml Tetrahydrofuran (getrocknet). Anschließend wird über Nacht bei 70°C gerührt und zur Trockne eingeengt. Man nimmt in 1 ml Dimethylsulfoxid auf und reinigt chromatographisch (reversed phase HPLC).
Ausbeute: 8 mg (8%) {2-Amino-6-[4,5-difluor-2-(2-hydroxy-ethoxymethyl)phenyl]-chinazolin-4-yl}-(1,3-dihydro-isoindol-2-yl)-methanon;
Retentionszeit LC-MS: 2.50 min;
¹H NMR (500 MHz, DMSO-d₆/TFA-d₁) δ [ppm] 8.13 (dd, J = 8.7, 1.9, 1H), 8.09 (d, J = 1.7, 1 H), 7.85 (d, J = 8.6, 1 H), 7.62 (dd, J = 11.6, 8.4, 1 H), 7.44 (t, J = 9.4, 2H), 7.33 (dt, J = 18.2, 7.1, 2H), 7.26 (d, J = 7.3, 1H), 5.04 (s, 2H), 4.86 (s, 2H), 4.32 (s, 2H), 3.47 (t, J = 5.0, 2H), 3.36 (t, J = 5.0, 2H).

### [2-Amino-6-(4,5-difluor-2-{2-[2-(2-hydroxy-ethoxy)-ethoxy]-ethoxymethyl}-phenyl)-chinazolin-4-yl]-(1,3-dihydro-isoindol-2-yl)-methanon ("A60")

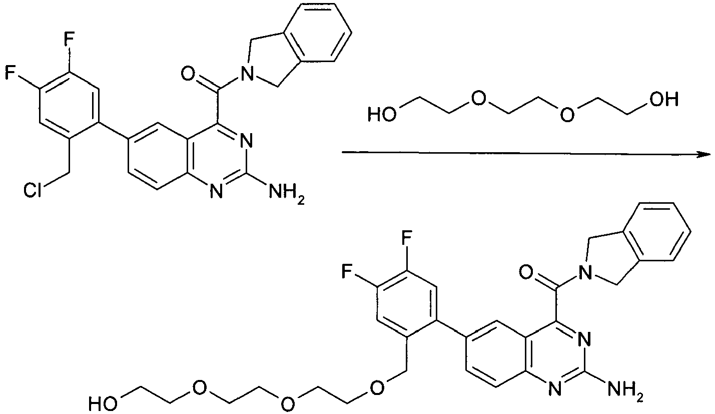

Man gibt 10 mg Natriumhydrid (60%ige Natriumhydrid Suspension in Paraffinöl) portionsweise zu 1 ml Triethylenglycol in 2 ml THF und rührt 1 h bei 25°C. Zu dieser Lösung tropft man eine Lösung aus 100 mg [2-Amino-6-(2-chlormethyl-5-fluor-phenyl)-chinazolin-4-yl]-(1,3-dihydro-isoindol-2-yl)-methanon zusammen mit 29 µl Triethylamin in 2 ml Tetrahydrofuran (getrocknet). Anschließend wird über Nacht bei 70°C gerührt und zur Trockne eingeengt. Man nimmt in 1 ml Dimethylsulfoxid auf und reinigt chromatographisch (reversed phase HPLC).
Ausbeute: 38 mg (33%) [2-Amino-6-(4,5-difluor-2-{2-[2-(2-hydroxy-ethoxy)-ethoxy]-ethoxymethyl}-phenyl)-chinazolin-4-yl]-(1,3-dihydro-isoindol-2-yl)-methanon; Retentionszeit LC-MS: 2.00 min;
¹H NMR (500 MHz, DMSO-d₆/TFA-d₁) δ [ppm] 8.13 (dd, J = 8.7, 1.9, 1H), 8.08 (d, J = 1.8, 1 H), 7.85 (d, J = 8.6, 1 H), 7.57 (dd, J = 11.6, 8.4, 1 H), 7.45 (dd, J = 13.2, 5.9, 2H), 7.33 (dt, J = 18.0, 7.2, 2H), 7.26 (d, J = 7.3, 1 H), 5.04 (s, 2H), 4.86 (s, 2H), 4.32 (s, 2H), 3.52 - 3.40 (m, 12H).

### (2-Amino-6-{4,5-difluor-2-[2-(2-hydroxy-ethoxy)-ethoxymethyl]-phenyl}-chinazolin-4-yl)-(1,3-dihydro-isoindol-2-yl)-methanon ("A61")

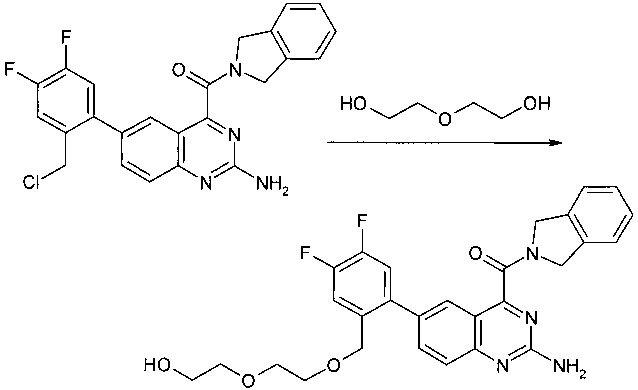

Man gibt 10 mg Natriumhydrid (60%ige Natriumhydrid Suspension in Paraffinöl) portionsweise zu 1 g Diethylenglycol in 2 ml THF und rührt 1 h bei 25°C. Zu dieser Lösung tropft man eine Lösung aus 100 mg [2-Amino-6-(2-chlormethyl-5-fluor-phenyl)-chinazolin-4-yl]-(1,3-dihydro-isoindol-2-yl)-methanon zusammen mit 29 µl Triethylamin in 2 ml Tetrahydrofuran (getrocknet). Anschließend wird über Nacht bei 70°C gerührt und zur Trockne eingeengt. Man nimmt in 1 ml Dimethylsulfoxid auf und reinigt chromatographisch (reversed phase HPLC).
Ausbeute: 30 mg (28%) (2-Amino-6-{4,5-difluor-2-[2-(2-hydroxy-ethoxy)-ethoxymethyl]-phenyl}-chinazolin-4-yl)-(1,3-dihydro-isoindol-2-yl)-methanon Retentionszeit LC-MS: 1.99 min;
¹H NMR (500 MHz, DMSO-d₆/TFA-d₁) δ [ppm] 8.12 (dd, J = 8.6, 1.8, 1H), 8.07 (d, J = 1.8, 1 H), 7.85 (d, J = 8.8, 1 H), 7.57 (dd, J = 11.5, 8.3, 1 H), 7.45 (dd, J = 13.1, 6.0, 2H), 7.33 (dt, J = 18.2, 7.2, 2H), 7.26 (d, J = 7.2, 1H), 5.04 (s, 2H), 4.86 (s, 2H), 4.32 (s, 2H), 3.49 - 3.43 (m, 6H), 3.38 (t, J = 5.1, 2H).

### {2-Amino-6-[4,5-difluor-2-(2-{2-[2-(2-hydroxy-ethoxy)-ethoxy]-ethoxy}-ethoxy-methyl)-phenyl]-chinazolin-4-yl}-(1,3-dihydro-isoindol-2-yl)-methanon ("A62")

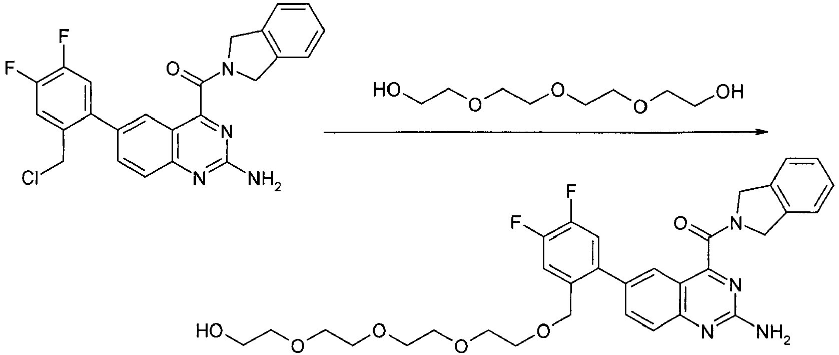

Man gibt 20 mg Natriumhydrid (60%ige Natriumhydrid Suspension in Paraffinöl) portionsweise zu 1 g Tetraethylenglycol in 2 ml THF und rührt 1 h bei 25°C. Zu dieser Lösung tropft man eine Lösung aus 200 mg [2-Amino-6-(2-chlormethyl-5-fluor-phenyl)-chinazolin-4-yl]-(1,3-dihydro-isoindol-2-yl)-methanon zusammen mit 57 µl Triethylamin in 2 ml Tetrahydrofuran (getrocknet). Anschließend wird über Nacht bei 70°C gerührt und zur Trockne eingeengt. Man nimmt in 2 ml Dimethylsulfoxid auf und reinigt chromatographisch (reversed phase HPLC).
Ausbeute: 80 mg (32%) {2-Amino-6-[4,5-difluor-2-(2-{2-[2-(2-hydroxy-ethoxy)-ethoxy]-ethoxy}-ethoxymethyl)-phenyl]-chinazolin-4-yl}-(1,3-dihydro-isoindol-2-yl)-methanon; Retentionszeit LC-MS: 2.05 min;
¹H NMR (500 MHz, DMSO-d₆/TFA-d₁) δ [ppm] 8.12 (dd, J = 8.7, 1.6, 1H), 8.08 (d, J = 1.7, 1 H), 7.86 (d, J = 8.6, 1 H), 7.57 (dd, J = 11.5, 8.3, 1 H), 7.44 (t, J = 9.4, 2H), 7.33 (dt, J = 18.5, 7.4, 2H), 7.26 (d, J = 7.5, 1H), 5.04 (s, 2H), 4.86 (s, 2H), 4.32 (s, 2H), 3.54 - 3.39 (m, 16H).

### [2-Amino-6-(2-aminoxymethyl-4,5-difluor-phenyl)-chinazolin-4-yl]-(1,3-dihydroisoindol-2-yl)-methanon ("A63")

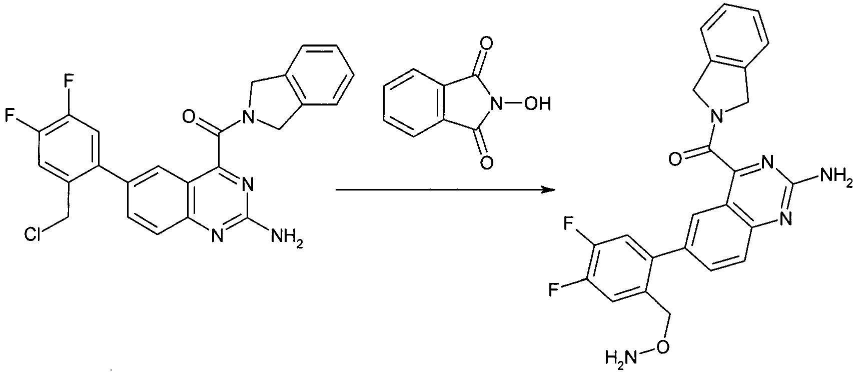

Man gibt 57 mg Kaliumcarbonat zu 34 mg N-Hydroxyphthalimid in 1 ml 1-Methyl-2-pyrrolidon und rührt 1 h bei 25°C. Zu dieser Lösung gibt man 100 mg [2-Amino-6-(2-chlormethyl-5-fluor-phenyl)-chinazolin-4-yl]-(1,3-dihydroisoindol-2-yl)-methanon. Anschließend wird über Nacht bei 25°C gerrührt. Anschließend wird mit 10 ml Eiswasser versetzt, der entstehende Niederschlag abfiltriert und in 20 ml Dichlormethan suspendiert. Man gibt 20 µl Hydraziniumhydroxid dazu und rührt 4 h bei 25°C. Man filtriert vom Niederschlag ab, engt das Filtrat zur Trockne ein, nimmt in 1 ml Dimethylsulfoxid auf und reinigt chromatographisch (reversed phase HPLC).
Ausbeute: 29 mg (29%) [2-Amino-6-(2-aminoxymethyl-4,5-difluor-phenyl)-chinazolin-4-yl]-(1,3-dihydro-isoindol-2-yl)-methanon;
Retentionszeit LC-MS: 1.82 min.

### [2-Amino-6-(4,5-difluor-2-hydroxymethyl-phenyl)-chinazolin-4-yl]-(1,3-dihydroisoindol-2-yl)-methanon ("A64")

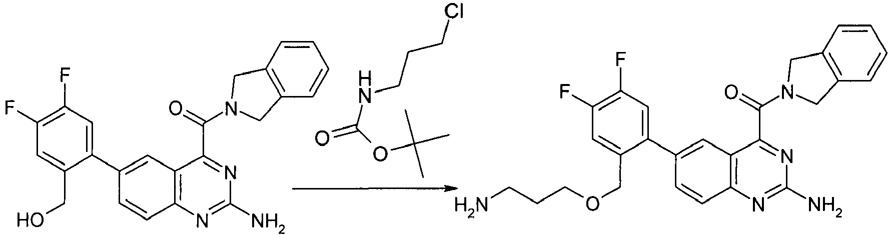

100 mg [2-Amino-6-(4,5-difluor-2-hydroxymethyl-phenyl)-chinazolin-4-yl]-(1,3-dihydro-isoindol-2-yl)-methanon werden zusammen mit 90 mg (3-Chlor-propyl)-carbaminsäure-*tert*.-butylester in 3 ml Tetrahydrofuran gelöst. Man gibt 2 ml Natronlauge (32%ig) und 2 mg Tetrabutylammoniumiodid dazu und rührt 16 h bei 80°C. Es wird 3mal mit 5 ml Ethylacetat extrahiert. Man trocknet die vereinigten organischen Phasen über Natriumsulfat. Nach Filtration wird das Filtrat zur Trockne eingeengt, in 3 ml Dichlormethan aufgenommen und mit 1 ml 4N HCl in Dioxan versetzt. Nach 1 h bei 25°C wird im Vakuum zur Trockne eingeengt und chromatographisch (reversed phase HPLC) gereinigt.
Ausbeute: 18 mg (16%) {2-Amino-6-[2-(3-amino-propoxymethyl)-4,5-difluor-phenyl]-chinazolin-4-yl}-(1,3-dihydro-isoindol-2-yl)-methanon;
Retentionszeit LC-MS: 1.66 min.

### 3-Amino-propionsäure-2-[2-amino-4-(1,3-dihydro-isoindol-2-carbonyl)-chinazolin-6-yl]-4,5-difluor-benzylester Dihydrochlorid ("A65")

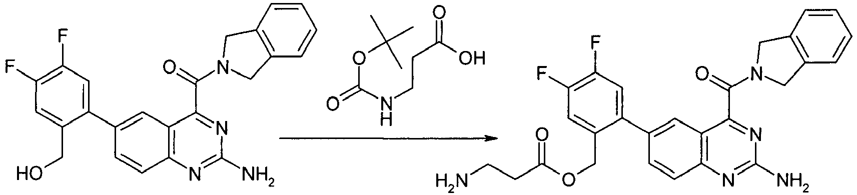

Zu einer Lösung von 875 mg 3-tert.-Butoxycarbonylamino-propionsäure in 5 ml Dichlormethan gibt man 477 mg Dicyclohexylcarbodiimid und rührt 1 h bei 25°C. Es wird vom Niederschlag abfiltriert und zu einer Lösung von 500 mg [2-Amino-6-(4,5-difluor-2-hydroxymethyl-phenyl)-chinazolin-4-yl]-(1,3-dihydro-isoindol-2-yl)-methanon und 70 mg 4-(Dimethylamino)-pyridin (DMAP) in 10 ml Tetrahydrofuran gegeben. Es wird für 16 h bei 25°C gerührt und zur Trockne eingeengt. Flashchromatographie liefert 480 mg 3-tert.-Butoxycarbonylamino-propionsäure-2-[2-amino-4-(1,3-dihydro-isoindol-2-carbonyl)-chinazolin-6-yl]-4,5-difluor-benzylester. Dieses Material wird in 10 ml Dichlormethan gelöst und unter Eiskühlung mit 5 ml 4N HCl in Dioxan versetzt. Anschließend wird 1 h bei 25°C weitergerührt, wobei sich ein Niederschlag abscheidet. Dieser wird abgesaugt und im Vakuum bei 40°C getrocknet.
Ausbeute: 330 mg (50%) 3-Amino-propionsäure 2-[2-amino-4-(1,3-dihydro-isoindol-2-carbonyl)-chinazolin-6-yl]-4,5-difluor-benzylester Dihydrochlorid; Retentionszeit LC-MS: 1.65 min;
¹H NMR (500 MHz, DMSO-d₆/TFA-d₁) δ [ppm] 8.11 (d, J = 1.7, 1 H), 8.07 (dd, J = 1.9, 8.7, 1 H), 7.89 (d, J = 8.6, 1 H), 7.63 (dd, J = 8.2, 11.3, 1 H), 7.49 - 7.43 (m, 2H), 7.34 (dt, J = 7.2, 17.8, 2H), 7.26 (d, J = 7.3, 1 H), 5.05 (s, 2H), 4.99 (s, 2H), 4.88 (s, 2H), 3.06 (t, J = 6.9, 2H), 2.73 (t, J = 6.9, 2H).

### (S)-2,5-Diamino-pentansäure-2-[2-amino-4-(1,3-dihydro-isoindol-2-carbonyl)-chinazolin-6-yl]-4,5-difluor-benzylester Diformiat ("A66")

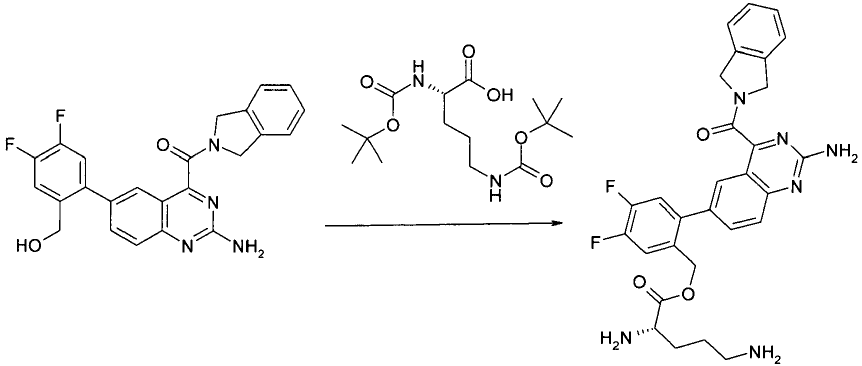

Zu einer Lösung von 308 mg (S)-2,5-Bis-tert.-butoxycarbonylamino-pentansäure in 2 ml Dichlormethan gibt man 95 mg Dicyclohexylcarbodiimid und rührt 1 h bei 25°C. Es wird vom Niederschlag abfiltriert und zu einer Lösung von 100 mg [2-Amino-6-(4,5-difluor-2-hydroxymethyl-phenyl)-chinazolin-4-yl]-(1,3-dihydro-isoindol-2-yl)-methanon und 3 mg 4-(Dimethylamino)-pyridin (DMAP) in 1 ml Tetrahydrofuran gegeben. Es wird für 16 h bei 25°C gerührt und zur Trockne eingeengt. Beim Verreiben mit Petrolether wird (S)-2,5-Bis-tert.-butoxycarbonylamino-pentansäure-2-[2-amino-4-(1,3-dihydro-isoindol-2-carbonyl)-chinazolin-6-yl]-4,5-difluor-benzylester als gelber Feststoff erhalten. Dieses Material wird in 2 ml Dichlormethan gelöst und unter Eiskühlung mit 4 ml 4N HCl in Dioxan versetzt. Anschließend wird 3 h bei 25°C weitergerührt und anschließend im Vakuum zur Trockne eingeengt. Der Rückstand wird in 2 ml Dimethylsulfoxid gelöst und chromatographisch (reversed phase HPLC) gereinigt.
Ausbeute: 28 mg (22%) (S)-2,5-Diamino-pentansäure 2-[2-amino-4-(1,3-dihydro-isoindol-2-carbonyl)-chinazolin-6-yl]-4,5-difluor-benzylester Diformiat; Retentionszeit LC-MS: 1.45 min;
¹H NMR (500 MHz, DMSO-d₆/TFA-d₁) δ [ppm] 8.15 - 8.07 (m, 2H), 7.86 (d, J = 8.8, 1 H), 7.56 (dd, J = 11.7, 8.4, 1 H), 7.44 (d, J = 7.3, 1 H), 7.34 (dt, J = 18.0, 9.4, 3H), 7.26 (d, J = 7.5, 1 H), 5.04 (s, 2H), 4.87 (s, 2H), 4.36 (s, 2H), 3.79 (dd, J = 11.4, 6.2, 1 H), 3.21 (dd, J = 9.8, 4.5, 2H), 2.18 (s, 1 H), 1.95 - 1.71 (m, 3H).

### 3-Dimethylamino-propionsäure-2-[2-amino-4-(1,3-dihydro-isoindol-2-carbonyl)-chinazolin-6-yl]-4,5-difluor-benzylester ("A67")

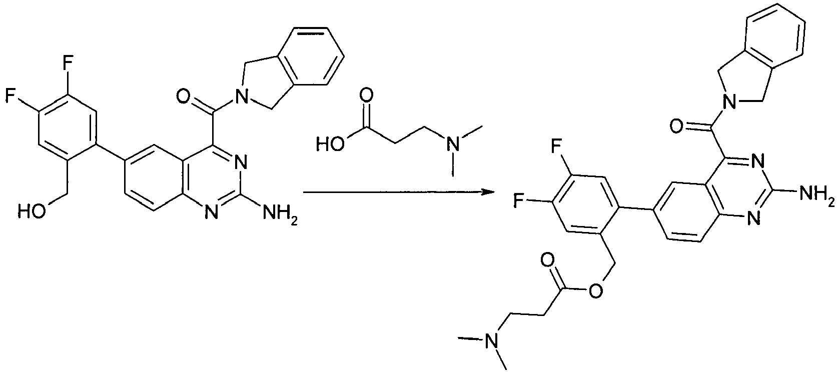

Zu einer Lösung von 2.131 g 3-Dimethylamino-propionsäure Hydrochlorid in 50 ml Dichlormethan gibt man 1.4 g Triethylamin und 1.431 g Dicyclohexylcarbodiimid und rührt 1 h bei 25°C. Es wird vom Niederschlag abfiltriert und zu einer Lösung von 1.5 g [2-Amino-6-(4,5-difluor-2-hydroxymethyl-phenyl)-chinazolin-4-yl]-(1,3-dihydro-isoindol-2-yl)-methanon und 21 mg 4-(Dimethylamino)-pyridin (DMAP) in 50 ml Tetrahydrofuran gegeben. Es wird für 16 h bei 25°C gerührt und zur Trockne eingeengt. Man nimmt in 50 ml Ethylacetat auf, wäscht 3mal mit 20 ml Wasser und trocknet die vereinigten organischen Phasen über Natriumsulfat. Nach Filtration wird das Filtrat zur Trockne eingeengt und chromatographisch (reversed phase HPLC) gereinigt. Die vereinigten Fraktionen werden zur Trockne eingeengt und aus 20 ml Acetonitril umkristallisiert.
Ausbeute: 560 mg (30%) 3-Dimethylamino-propionsäure-2-[2-amino-4-(1,3-dihydro-isoindol-2-carbonyl)-chinazolin-6-yl]-4,5-difluor-benzylester; Retentionszeit LC-MS: 1.67 min;
¹H NMR (500 MHz, DMSO-d₆/TFA-d₁) δ [ppm] 8.09 (d, J = 1.7, 1H), 8.06 (dd, J = 8.6, 2.0, 1 H), 7.88 (d, J = 8.6, 1 H), 7.64 (dd, J = 11.4, 8.3, 1 H), 7.48 (dd, J = 10.9, 8.0, 1 H), 7.44 (d, J = 7.5, 1 H), 7.33 (dt, J = 17.6, 7.3, 2H), 7.26 (d, J = 7.3, 1 H), 5.04 (s, 2H), 4.97 (s, 2H), 4.87 (s, 2H), 3.32 (t, J = 7.4, 2H), 2.89 (t, J = 7.3, 2H), 2.83 (s, 6H).

### (S)-2,6-Bis-tert.-butoxycarbonylamino-hexansäure-2-[2-amino-4-(1,3-dihydro-isoindol-2-carbonyl)-chinazolin-6-yl]-4,5-difluor-benzylester ("A68")

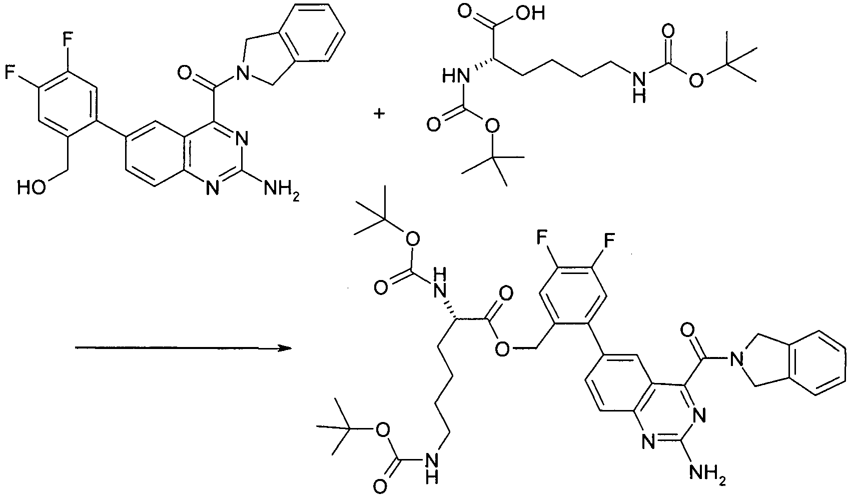

Zu einer Lösung von 4.807 g (S)-2,6-Bis-tert.-butoxycarbonylamino-hexansäure in 50 ml Dichlormethan gibt man 1.431 g Dicyclohexylcarbodiimid und rührt 1 h bei 25°C. Es wird vom Niederschlag abfiltriert und zu einer Lösung von 1.5 g [2-Amino-6-(4,5-difluor-2-hydroxymethyl-phenyl)-chinazolin-4-yl]-(1,3-dihydro-isoindol-2-yl)-methanon und 21 mg 4-(Dimethylamino)-pyridin (DMAP) in 100 ml Tetrahydrofuran gegeben. Es wird für 16 h bei 25°C gerührt, vom Ungelösten filtriert und das Filtrat zur Trockne eingeengt. Der Rückstand wird chromatographisch an der Normalphase gereinigt.
Ausbeute: 1.85 g (70%) (S)-2,6-Bis-tert.-butoxycarbonylamino-hexansäure-2-[2-amino-4-(1,3-dihydro-isoindol-2-carbonyl)-chinazolin-6-yl]-4,5-difluor-benzylester; Retentionszeit LC-MS: 2.67 min;
¹H NMR (500 MHz, DMSO-d₆) δ [ppm] 8.14 (d, J = 1.7, 1H), 8.08 (dd, J = 8.6, 1.9, 1 H), 7.89 (d, J = 8.6, 1 H), 7.72 (dd, J = 11.3, 8.2, 1H), 7.51 (dd, J = 10.9, 8.0, 1 H), 7.44 (d, J = 7.4, 1 H), 7.34 (dt, J = 18.5, 7.2, 2H), 7.27 (d, J = 7.3, 1 H), 5.14 - 5.03 (m, 4H), 4.93 - 4.84 (m, 2H), 4.17 (t, J = 6.5, 1 H), 2.82 - 2.75 (m, 2H), 1.93 - 1.76 (m, 2H), 1.59 (dt, J = 15.3, 7.5, 2H), 1.53 - 1.32 (m, 20H).

Analog erhält man:

### (S)-2-tert-Butoxycarbonylamino-6-dimethylamino- hexansäure-2-[2-amino-4-(1,3-dihydro-isoindol-2-carbonyl)-chinazolin-6-yl]-4,5-difluor-benzylester ("A70a")

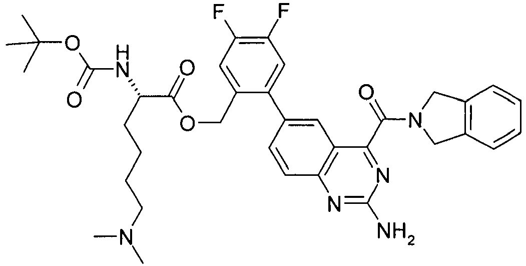

Ausbeute: 1 g (63%) (S)-2-tert.-Butoxycarbonylamino-6-dimethylamino-hexansäure-2-[2-amino-4-(1,3-dihydro-isoindol-2-carbonyl)-chinazolin-6-yl]-4,5-difluor-benzylester;
Retentionszeit LC-MS: 1.87 min;
¹H NMR (500 MHz, DMSO-de/TFA-d₁) δ [ppm] 8.14 (1 H, d, J 1.6), 8.06 (1 H, dd, J 8.6, 1.7), 7.89 (1 H, d, J 8.7), 7.56 (1 H, dd, J 11.3, 8.1), 7.45 - 7.28 (4 H, m), 7.24 (1 H, d, J 7.4), 5.07 (2 H, s), 5.00 (2 H, dd, J 25.8, 12.8), 4.89 (2 H, s), 3.93 (1 H, dd, J 9.6, 4.8), 3.21 (2 H, s), 3.03 (2 H, t, J 7.7), 2.61 (3 H, t, J 3.5, 1.8), 1.71 - 1.56 (5 H, m), 1.46 - 1.20 (11 H, m).

### (S)-2,6-Diamino-hexansäure-2-[2-amino-4-(1,3-dihydro-isoindol-2-carbonyl)-chinazolin-6-yl]-4,5-difluor-benzylester Formiat ("A71")

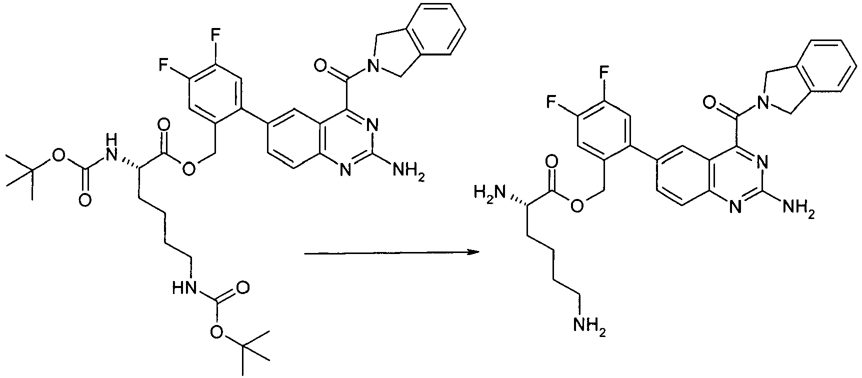

Zu 1.8 g (S)-2,6-Bis-tert.-butoxycarbonylamino-hexansäure-2-[2-amino-4-(1,3-dihydro-isoindol-2-carbonyl)-chinazolin-6-yl]-4,5-difluor-benzylester in 50 ml Dichlormethan gibt man unter Eiskühlung 515 µl Trifluoressigsäure. Anschließend wird 16 h bei 25°C weitergerührt, mit 20 ml n-Heptan versetzt und anschließend im Vakuum zur Trockne eingeengt. Der Rückstand wird in 4 ml Acetonitril gelöst und chromatographisch (reversed phase HPLC) gereinigt. Ausbeute: 1.39 g (97%) (S)-2,6-Diamino-hexansäure-2-[2-amino-4-(1,3-dihydro-isoindol-2-carbonyl)-chinazolin-6-yl]-4,5-difluorbenzylester Formiat;
Retentionszeit LC-MS: 1.45 min;
¹H NMR (500 MHz, DMSO-d₆) δ [ppm] 8.14 (d, J = 1.7, 1H), 8.08 (dd, J = 8.6, 1.9, 1 H), 7.89 (d, J = 8.6, 1 H), 7.72 (dd, J = 11.3, 8.2, 1 H), 7.51 (dd, J = 10.9, 8.0, 1 H), 7.44 (d, J = 7.4, 1 H), 7.34 (dt, J = 18.5, 7.2, 2H), 7.27 (d, J = 7.3, 1 H), 5.14 - 5.03 (m, 4H), 4.93 - 4.84 (m, 2H), 4.17 (t, J = 6.5, 1 H), 2.82 - 2.75 (m, 2H), 1.93 - 1.76 (m, 2H), 1.59 (dt, J = 15.3, 7.5, 2H), 1.53 - 1.32 (m, 2H).

Analog erhält man:

### (S)-2-Amino-6-dimethylamino-hexansäure-2-[2-amino-4-(1,3-dihydro-isoindol-2-carbonyl)-chinazolin-6-yl]-4,5-difluor-benzylester ("A73a")

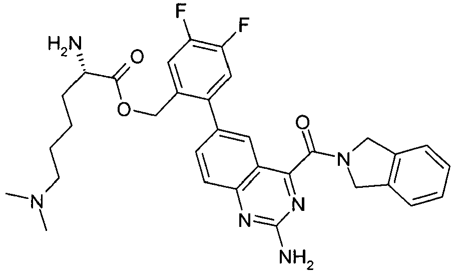

Ausbeute: 1.99 g (98%) (S)-2-Amino-6-dimethylamino-hexansäure-2-[2-amino-4-(1,3-dihydro-isoindol-2-carbonyl)-chinazolin-6-yl]-4,5-difluor-benzylester trihydrochlorid;
Retentionszeit LC-MS: 1.43 min;
¹H NMR (500 MHz, DMSO-d₆/TFA-d₁) δ [ppm] 8.17 (1 H, d, J 1.8), 8.06 (1 H, dd, J 8.7, 1.9), 7.93 (1 H, d, J 8.6), 7.68 (1 H, dd, J 11.2, 8.1), 7.42 (2 H, t, J 9.3), 7.34 (2 H, dt, J 18.2, 7.2), 7.26 (1 H, d, J 7.3), 5.17 - 5.02 (4 H, m), 4.90 (2 H, d, J 14.1), 4.18 (1 H, t, J 6.5), 3.07 (2 H, t, J 8.1), 2.62 (3 H, dt, J 3.6, 1.7), 1.96 - 1.83 (2 H, m), 1.77 - 1.66 (2 H, m), 1.56 - 1.37 (2 H, m).

### 2-Amino-4-methyl-pentansäure-2-[2-amino-4-(1,3-dihydro-isoindol-2-carbonyl)-chinazolin-6-yl]-4,5-difluorbenzylester ("A74")

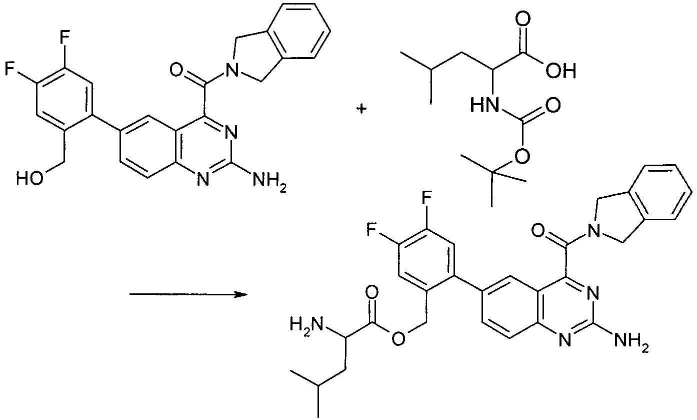

Zu einer Lösung von 321 mg 2-tert.-Butoxycarbonylamino-4-methyl-pentansäure in 2 ml Dichlormethan gibt man 143 mg Dicyclohexylcarbodiimid und rührt 1 h bei 25°C. Es wird vom Niederschlag abfiltriert und zu einer Lösung von 150 mg [2-Amino-6-(4,5-difluor-2-hydroxymethyl-phenyl)-chinazolin-4-yl]-(1,3-dihydro-isoindol-2-yl)-methanon und 2 mg 4-(Dimethylamino)-pyridin (DMAP) in 2 ml Tetrahydrofuran gegeben. Es wird für 16 h bei 25°C gerührt, vom Ungelösten filtriert und das Filtrat zur Trockne eingeengt. Der Rückstand wird in 2 ml Dichlormethan aufgenommen, mit 100 µl Trifluoressigsäure versetzt und 16 h bei 25°C gerührt. Anschließend gibt man 3 ml n-Heptan dazu und engt im Vakuum zur Trockne ein. Der Rückstand wird in 4 ml Acetonitril/Wasser (1:1) gelöst und chromatographisch (reversed phase HPLC) gereinigt.
Ausbeute: 94 mg (50%) 2-Amino-4-methyl-pentansäure 2-[2-amino-4-(1,3-dihydro-isoindol-2-carbonyl)-chinazolin-6-yl]-4,5-difluorbenzylester; Retentionszeit LC-MS: 1.77 min;
¹H NMR (500 MHz, DMSO-d₆[TFA-d₁) δ [ppm] 8.10 (d, J = 1.5, 1H), 8.08 (dd, J = 8.6, 2.0, 1 H), 7.85 (d, J = 8.6, 1 H), 7.71 (dd, J = 11.4, 8.1, 1 H), 7.53 (dd, J = 10.8, 8.1, 1 H), 7.44 (d, J = 7.3, 1 H), 7.34 (dt, J = 18.3, 7.2, 2H), 7.27 (d, J = 7.3, 1 H), 5.09 (s, 2H), 5.02 (s, 2H), 4.86 (s, 2H), 4.08 (t, J = 7.1, 1 H), 1.67 (td, J = 13.4, 6.7, 1 H), 1.56 (t, J = 7.2, 2H), 0.86 (t, J = 6.7, 6H).

### (S)-2-tert.-Butoxycarbonylamino-propionsäure-2-[2-amino-4-(1,3-dihydro-isoindol-2-carbonyl)-chinazolin-6-yl]-4,5-difluor-benzylester ("A75")

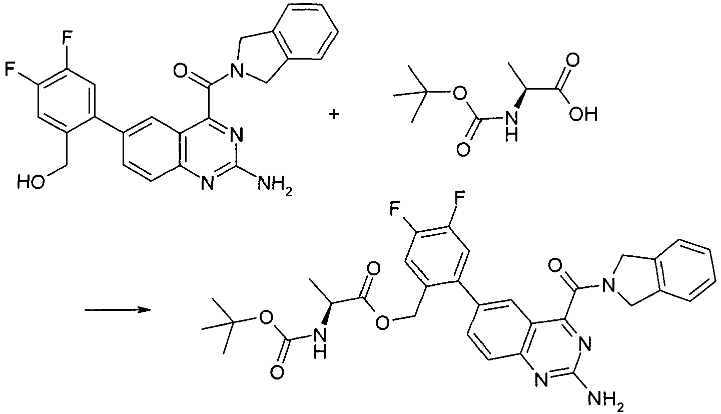

Zu einer Lösung von 263 mg (S)-2-tert.-Butoxycarbonylamino-propansäure in 2 ml Dichlormethan gibt man 143 mg Dicyclohexylcarbodiimid und rührt 1 h bei 25°C. Es wird vom Niederschlag abfiltriert und zu einer Lösung von 200 mg [2-Amino-6-(4,5-difluor-2-hydroxymethyl-phenyl)-chinazolin-4-yl]-(1,3-dihydro-isoindol-2-yl)-methanon und 3 mg 4-(Dimethylamino)-pyridin (DMAP) in 2 ml Tetrahydrofuran gegeben. Es wird für 16 h bei 25°C gerührt, vom Ungelösten filtriert und das Filtrat zur Trockne eingeengt. Der Rückstand wird chromatographisch an der Normalphase gereinigt.
Ausbeute: 270 mg (97%) (S)-2-tert.-Butoxycarbonylamino-propionsäure-2-[2-amino-4-(1,3-dihydro-isoindol-2-carbonyl)-chinazolin-6-yl]-4,5-difluor-benzylester;
Retentionszeit LC-MS: 2.46 min;
¹H NMR (500 MHz, DMSO-d₆/TFA-d₁) δ [ppm] 8.14 (d, J = 1.7, 1H), 8.07 (dd, J = 8.6, 1.8, 1H), 7.89 (d, J = 8.6, 1 H), 7.69 (dd, J = 11.4, 8.3, 1 H), 7.47 (dt, J = 13.9, 7.0, 1 H), 7.44 (d, J = 7.3, 1 H), 7.34 (dt, J = 18.0, 7.2, 2H), 7.26 (d, J = 7.3, 1 H), 5.07 (s, 2H), 4.98 (d, J = 5.9, 2H), 4.89 (s, 2H), 3.54 - 3.43 (m, 1 H), 1.42 (s, 9H), 1.40 (d, J = 7.2, 3H).

### (S)-2-Amino-propionsäure-2-[2-amino-4-(1,3-dihydro-isoindol-2-carbonyl)-chinazolin-6-yl]-4,5-difluor-benzylester Dihydrochlorid ("A76")

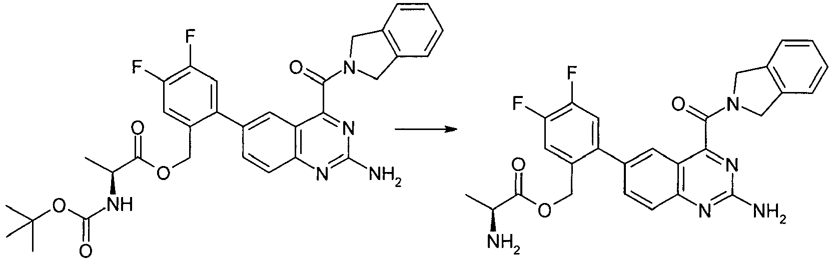

Zu 220 mg (S)-2-tert.-Butoxycarbonylamino-propionsäure-2-[2-amino-4-(1,3-dihydro-isoindol-2-carbonyl)-chinazolin-6-yl]-4,5-difluor-benzylester in 4 ml Dichlormethan gibt man unter Eiskühlung 911 µl 4N HCl in Dioxan. Anschließend wird 1 h bei 25°C weitergerührt, der Niederschlag abgesaugt und mit 4 ml Dichlormethan gewaschen. Der Rückstand wird aus Ethanol umkristallisiert.
Ausbeute: 60 mg (29%) (S)-2-Amino-propionsäure-2-[2-amino-4-(1,3-dihydro-isoindol-2-carbonyl)-chinazolin-6-yl]-4,5-difluor-benzylester Dihydrochlorid; Retentionszeit LC-MS: 1.63 min;
¹H NMR (500 MHz, DMSO-d₆/TFA-d₁) δ [ppm] 8.14 (d, J = 1.7, 1H), 8.07 (dd, J = 8.6, 1.8, 1 H), 7.89 (d, J = 8.6, 1 H), 7.69 (dd, J = 11.4, 8.3, 1 H), 7.47 (dt, J = 13.9, 7.0, 1 H), 7.44 (d, J = 7.3, 1 H), 7.34 (dt, J = 18.0, 7.2, 2H), 7.26 (d, J = 7.3, 1 H), 5.08 (d, J = 1.3, 2H), 5.04 (s, 2H), 4.89 (s, 2H), 4.20 (q, J = 7.2, 1 H), 1.40 (d, J = 7.2, 3H).

### 2,2-Dimethyl-propionsäure-2-[2-amino-4-(1,3-dihydro-isoindol-2-carbonyl)-chinazolin-6-yl]-4,5-difluor-benzylester ("A77")

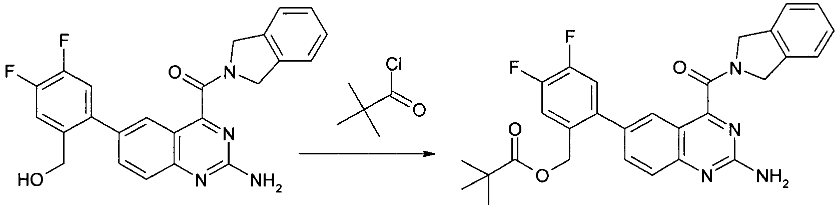

Zu einer Lösung von 200 mg [2-Amino-6-(4,5-difluor-2-hydroxymethyl-phenyl)-chinazolin-4-yl]-(1,3-dihydro-isoindol-2-yl)-methanon und 2 mg 4-(Dimethyl-amino)-pyridin (DMAP) in 4 ml Dimethylformamid werden 47 µl Pivalinsäurechlorid gegeben und 16 h bei 80°C gerührt. Man gibt 8 ml Ethylacetat und 8 ml Wasser dazu, trennt die organische Phase ab und engt sie im Vakuum zur Trockne ein. Der Rückstand wird in 1 ml Dimethylsulfoxid gelöst und chromatographisch (reversed phase HPLC) gereinigt.
Ausbeute: 26 mg (15%) 2,2-Dimethyl-propionsäure-2-[2-amino-4-(1,3-dihydro-isoindol-2-carbonyl)-chinazolin-6-yl]-4,5-difluor-benzylester;
Retentionszeit LC-MS: 2.59 min;
¹H NMR (500 MHz, DMSO-d₆/TFA-d₁) δ [ppm] 8.11 (d, J = 1.8, 1H), 8.06 (dd, J = 8.6, 1.8, 1 H), 7.88 (d, J = 8.6, 1 H), 7.54 - 7.47 (m, 1 H), 7.43 (t, J = 8.7, 2H), 7.33 (dt, J = 19.1, 7.2, 2H), 7.25 (d, J = 7.5, 1 H), 5.04 (s, 2H), 4.95 (s, 2H), 4.87 (s, 2H), 1.06 (s, 9H).

### Isobuttersäure-2-[2-amino-4-(1,3-dihydro-isoindol-2-carbonyl)-chinazolin-6-yl]-4,5-difluor-benzylester ("A78")

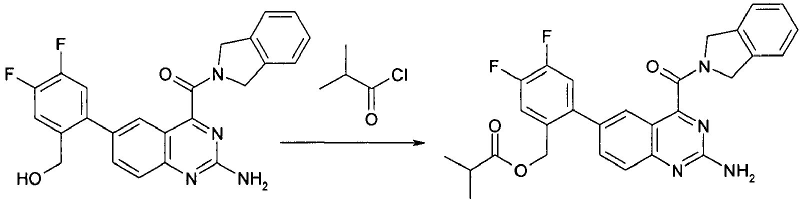

Zu einer Lösung von 150 mg [2-Amino-6-(4,5-difluor-2-hydroxymethyl-phenyl)-chinazolin-4-yl]-(1,3-dihydro-isoindol-2-yl)-methanon und 2 mg 4-(Dimethyl-amino)-pyridin (DMAP) in 5 ml Dimethylformamid werden 40 µl Isobuttersäurechlorid gegeben und 16 h bei 80°C gerührt. Man gibt 8 ml Ethylacetat und 8 ml Wasser dazu, trennt die organische Phase ab und engt sie im Vakuum zur Trockne ein. Der Rückstand wird in 1 ml Dimethylsulfoxid gelöst und chromatographisch (reversed phase HPLC) gereinigt.
Ausbeute: 48 mg (28%) Isobuttersäure-2-[2-amino-4-(1,3-dihydro-isoindol-2-carbonyl)-chinazolin-6-yl]-4,5-difluor-benzylester;
Retentionszeit LC-MS: 2.49 min;
¹H NMR (500 MHz, DMSO-d₆/TFA-d₁) δ [ppm] 8.09 - 8.04 (m, 2H), 7.85 (d, J = 8.6, 1 H), 7.57 (dd, J = 11.4, 8.3, 1 H), 7.46 (dd, J = 13.2, 7.7, 2H), 7.33 (dt, J = 18.9, 7.1, 2H), 7.25 (d, J = 7.2, 1 H), 5.02 (s, 2H), 4.92 (s, 2H), 4.85 (s, 2H), 2.51 - 2.44 (m, 1 H), 0.99 (d, J = 7.0, 6H).

### Propionsäure-2-[2-amino-4-(1,3-dihydro-isoindol-2-carbonyl)-chinazolin-6-yl]-4,5-difluor-benzylester ("A79")

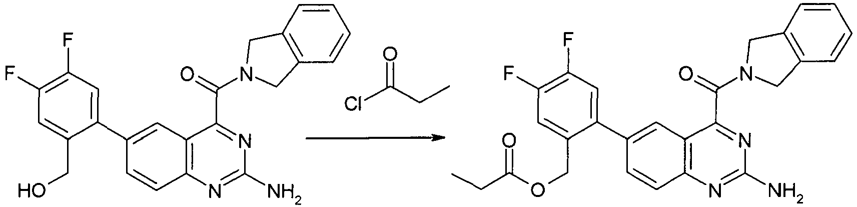

Zu einer Lösung von 150 mg [2-Amino-6-(4,5-difluor-2-hydroxymethyl-phenyl)-chinazolin-4-yl]-(1,3-dihydro-isoindol-2-yl)-methanon und 2 mg 4-(Dimethyl-amino)-pyridin (DMAP) in 5 ml Dimethylformamid werden 33 µl Propionsäurechlorid gegeben und 16 h bei 80°C gerührt. Man gibt 8 ml Ethylacetat und 8 ml Wasser dazu, trennt die organische Phase ab und engt sie im Vakuum zur Trockne ein. Der Rückstand wird in 1 ml Dimethylsulfoxid gelöst und chromatographisch (reversed phase HPLC) gereinigt.
Ausbeute: 30 mg (18%) Propionsäure-2-[2-amino-4-(1,3-dihydro-isoindol-2-carbonyl)-chinazolin-6-yl]-4,5-difluor-benzylester;
Retentionszeit LC-MS: 2.37 min;
¹H NMR (500 MHz, DMSO-d₆) δ [ppm] 8.09 - 8.03 (m, 2H), 7.86 (d, J = 8.4, 1 H), 7.58 (dd, J = 11.4, 8.3, 1 H), 7.48 - 7.42 (m, 2H), 7.33 (dt, J = 19.1, 7.2, 2H), 7.25 (d, J = 7.5, 1H), 5.03 (s, 2H), 4.90 (s, 2H), 4.86 (s, 2H), 2.27 (q, J = 7.5, 2H), 0.95 (t, J = 7.5, 3H).

### 2-tert.-Butoxycarbonylamino-2-methyl-propionsäure-2-[2-amino-4-(1,3-dihydro-isoindol-2-carbonyl)-chinazolin-6-yl]-4,5-difluor-benzylester ("A80")

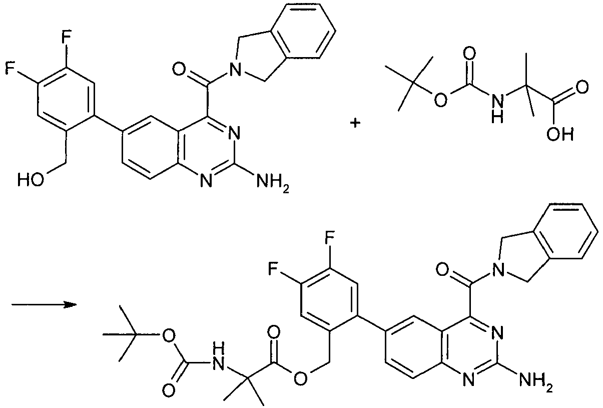

Zu einer Lösung von 4.2 g 2-tert.-Butoxycarbonylamino-2-methyl-propionsäure in 100 ml Dichlormethan gibt man 2.2 g Dicyclohexylcarbodiimid und rührt 1 h bei 25°C. Es wird vom Niederschlag abfiltriert und zu einer Lösung von 1.5 g [2-Amino-6-(4,5-difluor-2-hydroxymethyl-phenyl)-chinazolin-4-yl]-(1,3-dihydro-isoindol-2-yl)-methanon und 20 mg 4-(Dimethylamino)-pyridin (DMAP) in 50 ml Tetrahydrofuran gegeben. Es wird für 48 h bei 25°C gerührt, vom Ungelösten filtriert und das Filtrat zur Trockne eingeengt. Der Rückstand wird chromatographisch an der Normalphase gereinigt.
Ausbeute: 1.3 g (61%) 2-tert.-Butoxycarbonylamino-2-methyl-propionsäure-2-[2-amino-4-(1,3-dihydro-isoindol-2-carbonyl)-chinazolin-6-yl]-4,5-difluor-benzylester; Retentionszeit LC-MS: 2.53 min;
¹H NMR (500 MHz, DMSO-d₆/TFA-d₁) δ [ppm] 8.11 (d, J = 1.7, 1H), 8.08 (dd, J = 8.7, 1.9, 1 H), 7.89 (d, J = 8.6, 1 H), 7.54 (t, J = 9.8, 1 H), 7.43 (dd, J = 10.9, 8.3, 2H), 7.33 (dt, J = 18.3, 7.2, 2H), 7.26 (d, J = 7.3, 1H), 5.06 (s, 2H), 4.95 (s, 2H), 4.89 (s, 2H), 1.31 (s, 6H), 1.29 (s, 9H).

### 2-Amino-2-methyl-propionsäure-2-[2-amino-4-(1,3-dihydro-isoindol-2-carbonyl)-chinazolin-6-yl]-4,5-difluor-benzylester Dihydrochlorid ("A81")

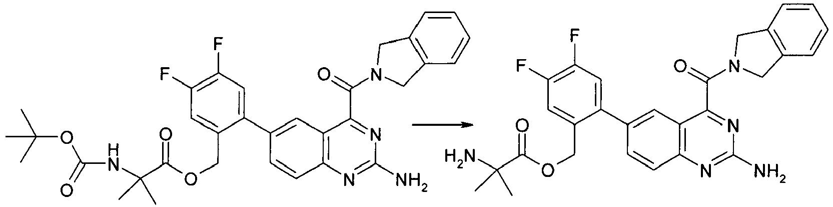

Zu 250 mg (2-tert.-Butoxycarbonylamino-2-methyl-propionsäure-2-[2-amino-4-(1,3-dihydro-isoindol-2-carbonyl)-chinazolin-6-yl]-4,5-difluor-benzylester in 5 ml Dichlormethan gibt man unter Eiskühlung 2.4 ml 4N HCl in Dioxan. Anschließend wird 1 h bei 25°C weitergerührt, der Niederschlag abgesaugt und mit 4 ml Dichlormethan gewaschen. Der Rückstand wird aus Isopropanol umkristallisiert.
Ausbeute: 175 mg (73%) 2-Amino-2-methyl-propionsäure-2-[2-amino-4-(1,3-dihydro-isoindol-2-carbonyl)-chinazolin-6-yl]-4,5-difluor-benzylester Dihydrochlorid; Retentionszeit LC-MS: 1.69 min;
¹H NMR (500 MHz, DMSO-d₆/TFA-d₁) δ [ppm] 8.12 (d, J = 1.7, 1H), 8.07 (dd, J = 8.6, 1.8, 1 H), 7.88 (d, J = 8.6, 1 H), 7.68 (dd, J = 11.5, 8.2, 1 H), 7.49 (dd, J = 10.9, 8.0, 1 H), 7.44 (d, J = 7.5, 1 H), 7.34 (dt, J = 15.2, 7.2, 2H), 7.27 (d, J = 7.3, 1 H), 5.13 (s, 2H), 5.04 (s, 2H), 4.89 (s, 2H), 1.42 (s, 6H).

### Essigsäure-2-[2-amino-4-(1,3-dihydro-isoindol-2-carbonyl)-chinazolin-6-yl]-4,5-difluor-benzylester ("A82")

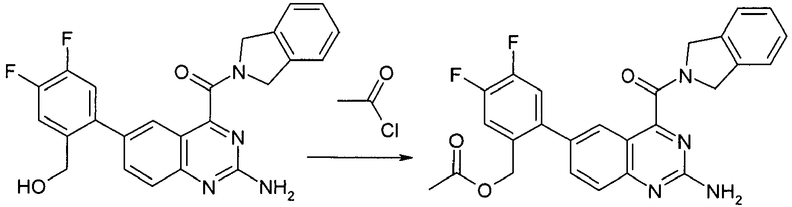

Zu einer Lösung von 150 mg [2-Amino-6-(4,5-difluor-2-hydroxymethyl-phenyl)-chinazolin-4-yl]-(1,3-dihydro-isoindol-2-yl)-methanon und 2 mg 4-(Dimethyl-amino)-pyridin (DMAP) in 5 ml Dimethylformamid werden 27 µl Acetylchlorid gegeben und 16 h bei 80°C gerührt. Man gibt 8 ml Ethylacetat und 8 ml Wasser dazu, trennt die organische Phase ab und engt sie im Vakuum zur Trockne ein. Der Rückstand wird in 1 ml Dimethylsulfoxid gelöst und chromatographisch (reversed phase HPLC) gereinigt.
Ausbeute: 31 mg (19%) Essigsäure-2-[2-amino-4-(1,3-dihydro-isoindol-2-carbonyl)-chinazolin-6-yl]-4,5-difluor-benzylester;
Retentionszeit LC-MS: 2.26 min;
¹H NMR (500 MHz, DMSO-d₆/TFA-d₁) δ [ppm] 8.07 (dd, J = 15.5, 5.2, 2H), 7.87 (d, J = 8.6, 1 H), 7.58 (d, J = 8.3, 1 H), 7.44 (d, J = 6.2, 2H), 7.38 - 7.27 (m, 2H), 7.26 (s, 1 H), 5.03 (s, 2H), 4.89 (s, 2H), 4.86 (s, 2H), 1.97 (s, 3H).

### 6-tert.-Butoxycarbonylamino-hexansäure-2-[2-amino-4-(1,3-dihydro-isoindol-2-carbonyl)-chinazolin-6-yl]-4,5-difluor-benzylester ("A83")

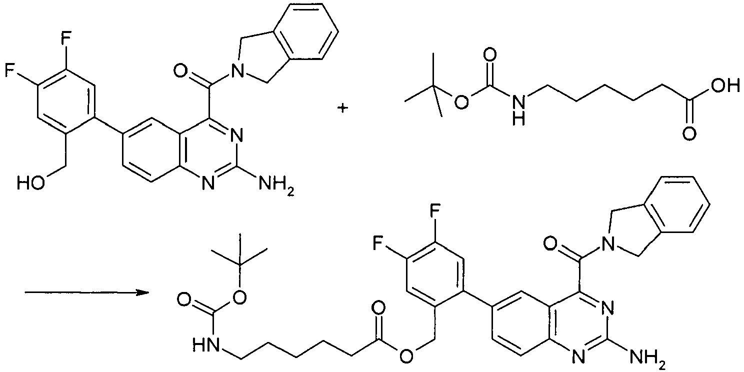

Zu einer Lösung von 214 mg 6-tert.-Butoxycarbonylamino-hexansäure in 5 ml Dichlormethan gibt man 95 mg Dicyclohexylcarbodiimid und rührt 1 h bei 25°C. Es wird vom Niederschlag abfiltriert und zu einer Lösung von 200 mg [2-Amino-6-(4,5-difluor-2-hydroxymethyl-phenyl)-chinazolin-4-yl]-(1,3-dihydro-isoindol-2-yl)-methanon und 3 mg 4-(Dimethylamino)-pyridin (DMAP) in 5 ml Tetrahydrofuran gegeben. Es wird für 48 h bei 25°C gerührt, vom Ungelösten filtriert und das Filtrat zur Trockne eingeengt. Der Rückstand wird chromatographisch an der Normalphase gereinigt.
Ausbeute: 44 mg (15%) 6-tert.-Butoxycarbonylamino-hexansäure-2-[2-amino-4-(1,3-dihydro-isoindol-2-carbonyl)-chinazolin-6-yl]-4,5-difluor-benzylester;
Retentionszeit LC-MS: 2.61 min;
¹H NMR (500 MHz, DMSO-d₆) δ [ppm] 8.03 (d, J = 1.7, 1 H), 7.97 (dd, J = 8.6, 1.8, 1H), 7.81 (d, J = 8.8, 1 H), 7.45 (dd, J = 11.4, 8.3, 1 H), 7.37 - 7.29 (m, 2H), 7.25 (dt, J = 18.9, 7.3, 2H), 7.17 (d, J = 7.3, 1H), 4.97 (s, 2H), 4.84 (s, 2H), 4.80 (s, 2H), 2.85 (t, J = 7.1, 2H), 2.17 (t, J = 7.4, 2H), 1.42 - 1.35 (m, 2H), 1.34 - 1.25 (m, 11 H), 1.17 - 1.09 (m, 2H).

### 6-Amino-hexansäure-2-[2-amino-4-(1,3-dihydro-isoindol-2-carbonyl)-chinazolin-6-yl]-4,5-difluor-benzylester Dihydrochlorid ("A84")

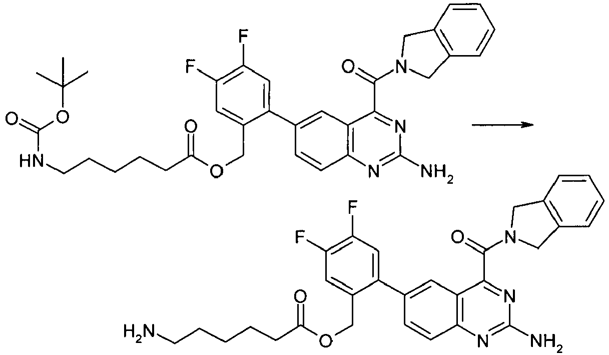

Zu 300 mg (2-tert.-Butoxycarbonylamino-2-methyl-propionsäure-2-[2-amino-4-(1,3-dihydro-isoindol-2-carbonyl)-chinazolin-6-yl]-4,5-difluor-benzylester in 5 ml Dichlormethan gibt man unter Eiskühlung 2.4 ml 4N HCl in Dioxan. Anschließend wird 1 h bei 25°C weitergerührt, der Niederschlag abgesaugt und mit 4 ml Dichlormethan gewaschen.
Ausbeute: 85 mg (32%) 6-Amino-hexansäure-2-[2-amino-4-(1,3-dihydro-isoindol-2-carbonyl)-chinazolin-6-yl]-4,5-difluor-benzyester Dihydrochlorid; Retentionszeit LC-MS: 1.74 min;
¹H NMR (400 MHz, DMSO-d₆/TFA-d₁) δ [ppm] 8.06 (d, J = 9.8, 2H), 7.88 (d, J = 8.2, 1 H), 7.59 (dd, J = 10.9, 8.5, 1 H), 7.52 - 7.42 (m, 2H), 7.34 (dt, J = 15.0, 7.3, 2H), 7.26 (d, J = 7.2, 1H), 5.03 (s, 2H), 4.92 (s, 2H), 4.86 (s, 2H), 2.76 (t, J = 7.5, 2H), 2.29 (t, J = 7.4, 2H), 1.60 - 1.50 (m, 2H), 1.46 (dd, J = 15.5, 7.9, 2H), 1.32 - 1.21 (m, 2H).

### 5-tert.-Butoxycarbonylamino-pentansäure-2-[2-amino-4-(1,3-dihydro-isoindol-2-carbonyl)-chinazolin-6-yl]-4,5-difluor-benzylester ("A85")

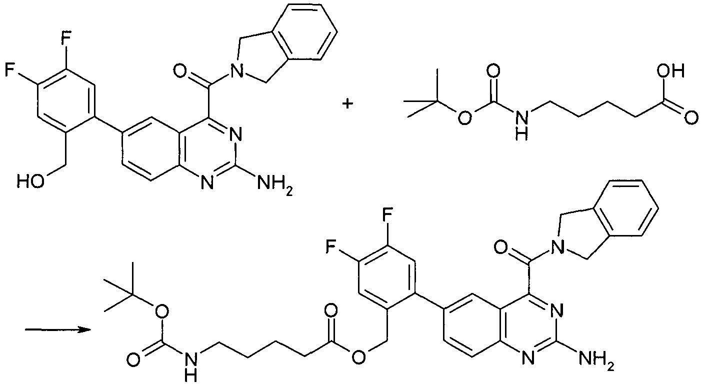

Zu einer Lösung von 402 mg 6-tert.-Butoxycarbonylamino-pentansäure in 10 ml Dichlormethan gibt man 191 mg Dicyclohexylcarbodiimid und rührt 1 h bei 25°C. Es wird vom Niederschlag abfiltriert und zu einer Lösung von 400 mg [2-Amino-6-(4,5-difluoro-2-hydroxymethyl-phenyl)-chinazolin-4-yl]-(1,3-dihydro-isoindol-2-yl)-methanon und 6 mg 4-(Dimethylamino)-pyridin (DMAP) in 10 ml Tetrahydrofuran gegeben. Es wird für 48 h bei 25°C gerührt, vom Ungelösten filtriert und das Filtrat zur Trockne eingeengt. Der Rückstand wird chromatographisch an der Normalphase gereinigt.
Ausbeute: 580 mg (99%) 5-tert.-Butoxycarbonylamino-pentansäure-2-[2-amino-4-(1,3-dihydro-isoindol-2-carbonyl)-chinazolin-6-yl]-4,5-difluor-benzyester; Retentionszeit LC-MS: 2.53 min;
¹H NMR (500 MHz, DMSO-d₆/TFA-d₁) δ [ppm] 8.03 (d, J = 1.7, 1H), 7.97 (dd, J = 8.6, 1.8, 1 H), 7.81 (d, J = 8.8, 1 H), 7.45 (dd, J = 11.4, 8.3, 1 H), 7.35 (d, J = 7.3, 1 H), 7.31 (dd, J = 10.8, 8.1, 1 H), 7.25 (dt, J = 18.9, 7.3, 2H), 7.17 (d, J = 7.3, 1H), 4.97 (s, 2H), 4.84 (s, 2H), 4.80 (s, 2H), 2.85 (t, J = 7.1, 2H), 2.17 (t, J = 7.4, 2H), 1.42 - 1.34 (m, 2H), 1.31 (s, 9H), 1.30 - 1.24 (m, 2H), 1.18 - 1.09 (m, 2H).

### 5-Amino-pentansäure-2-[2-amino-4-(1,3-dihydro-isoindol-2-carbonyl)-chinazolin-6-yl]-4,5-difluor-benzylester Dihydrochlorid ("A86")

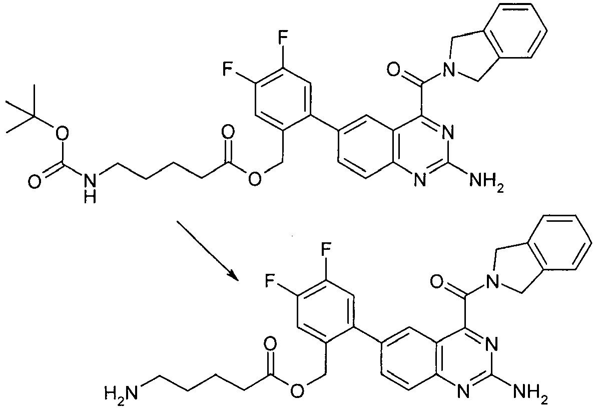

Zu 230 mg (2-tert.-Butoxycarbonylamino-2-methyl-propionsäure-2-[2-amino-4-(1,3-dihydro-isoindol-2-carbonyl)-chinazolin-6-yl]-4,5-difluor-benzylester in 3 ml Dichlormethan gibt man unter Eiskühlung 1.8 ml 4N HCl in Dioxan. Anschließend wird 1 h bei 25°C weitergerührt, der Niederschlag abgesaugt und mit 2 ml Dichlormethan gewaschen.
Ausbeute: 168 mg (76%) 5-Amino-pentansäure-2-[2-amino-4-(1,3-dihydro-isoindol-2-carbonyl)-chinazolin-6-yl]-4,5-difluor-benzyester Dihydrochlorid;
Retentionszeit LC-MS: 1.75 min;
¹H NMR (500 MHz, DMSO-d₆/TFA-d₁) δ [ppm] 8.09 - 8.04 (m, 2H), 7.87 (d, J = 9.0, 1 H), 7.59 (dd, J = 11.4, 8.3, 1 H), 7.50 - 7.43 (m, 2H), 7.33 (dt, J = 15.2, 7.3, 2H), 7.26 (d, J = 7.5, 1H), 5.03 (s, 2H), 4.92 (s, 2H), 4.86 (s, 2H), 2.79 (t, J = 6.7, 2H), 2.35 (t, J = 6.8, 2H), 1.63 - 1.47 (m, 4H).

### (S)-Pyrrolidin-1,2-dicarbonsäure-2-{2-[2-amino-4-(1,3-dihydro-isoindol-2-carbonyl)-chinazolin-6-yl]-4,5-difluor-benzyl}-ester-1-tert.-butylester ("A87")

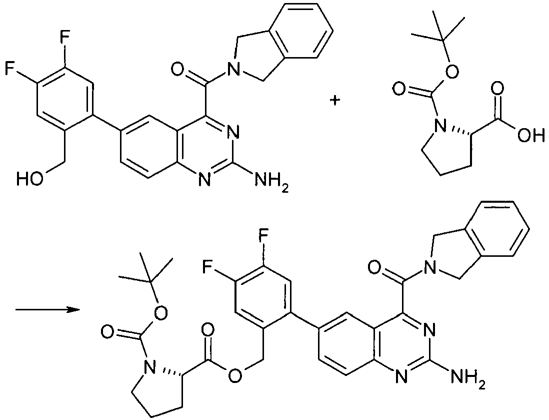

Zu einer Lösung von 2.1 g (S)-Pyrrolidin-1,2-dicarbonsäure-1-tert.-butylester in 25 ml Dichlormethan gibt man 1 g Dicyclohexylcarbodiimid und rührt 1 h bei 25°C. Es wird vom Niederschlag abfiltriert und zu einer Lösung von 1 g [2-Amino-6-(4,5-difluor-2-hydroxymethyl-phenyl)-chinazolin-4-yl]-(1,3-dihydro-isoindol-2-yl)-methanon und 14 mg 4-(Dimethylamino)-pyridin (DMAP) in 50 ml Tetrahydrofuran gegeben. Es wird für 48 h bei 25°C gerührt, vom Ungelösten filtriert und das Filtrat zur Trockne eingeengt. Der Rückstand wird chromatographisch an der Normalphase gereinigt.
Ausbeute: 1.5 g (100%) (S)-Pyrrolidin-1,2-dicarbonsäure-2-{2-[2-amino-4-(1,3-dihydro-isoindol-2-carbonyl)-chinazolin-6-yl]-4,5-difluor-benzyl}-ester-1-tert.-butylester; Retentionszeit LC-MS: 2.53 min;
¹H NMR (500 MHz, DMSO-d₆/TFA-d₁) δ [ppm] 8.03 (d, J = 4.2, 1 H), 7.95 (dd, J = 8.6, 1.8, 1 H), 7.79 (t, J = 7.8, 1 H), 7.43 (t, J = 9.4, 1 H), 7.34 - 7.17 (m, 4H), 7.13 (t, J = 7.2, 1 H), 5.01 - 4.75 (m, 6H), 4.09 - 3.96 (m, 1 H), 3.28 - 3.14 (m, 2H), 2.04 - 1.91 (m, 1 H), 1.72 - 1.56 (m, 3H), 1.31 - 1.04 (m, 9H).

### (S)-Pyrrolidin-2-carbonsäure-2-[2-amino-4-(1,3-dihydro-isoindol-2-carbonyl)-chinazolin-6-yl]-4,5-difluor-benzylester ("A88")

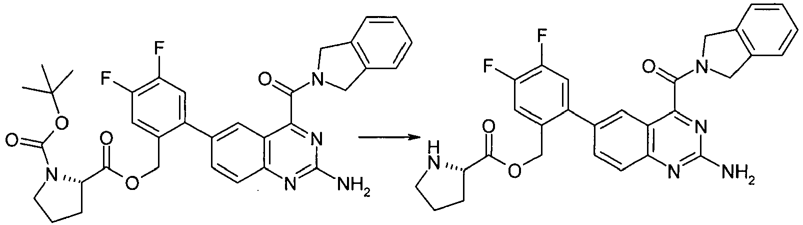

Zu 1.5 g (S)-Pyrrolidin-1,2-dicarbonsäure-2-{2-[2-amino-4-(1,3-dihydro-isoindol-2-carbonyl)-chinazolin-6-yl]-4,5-difluor-benzyl}-ester-1-tert.-butylester in 50 ml Dichlormethan gibt man unter Eiskühlung 10 ml Trifluoressigsäure. Anschließend wird 1 h bei 25°C weitergerührt, mit 10 ml n-Heptan versetzt und im Vakuum zur Trockne eingeengt. Der Rückstand wird in 100 ml Acetonitril/Wasser 3:1 suependiert, mit Bicarbonat neutralisiert (∼ pH 8) und das ausgefallene Material abgesaugt. Man kristallisiert aus 100 ml Acetonitril um.
Ausbeute: 900 mg (71%) (S)-Pyrrolidin-2-carbonsäure-2-[2-amino-4-(1,3-dihydro-isoindol-2-carbonyl)-chinazolin-6-yl]-4,5-difluor-benzylester;
Retentionszeit LC-MS: 1.72 min;
¹H NMR (500 MHz, DMSO-d₆/TFA-d₁) δ [ppm] 8.12 (d, J = 1.8, 1H), 8.08 (dd, J = 8.6, 1.8, 1 H), 7.88 (d, J = 8.6, 1 H), 7.70 (dd, J = 11.4, 8.1, 1 H), 7.50 (dd, J = 10.9, 8.0, 1H), 7.44 (d, J = 7.3, 1H), 7.34 (dt, J = 14.9, 7.2, 2H), 7.27 (d, J = 7.3, 1 H), 5.09 (s, 2H), 5.04 (s, 2H), 4.88 (s, 2H), 4.51 (dd, J = 8.3, 7.1, 1 H), 3.33 - 3.21 (m, 2H), 2.34 - 2.22 (m, 1 H), 2.02 - 1.84 (m, 3H).

### 4-tert.-Butoxycarbonylamino-buttersäure-2-[2-amino-4-(1,3-dihydro-isoindol-2-carbonyl)-chinazolin-6-yl]-4,5-difluor-benzylester ("A89")

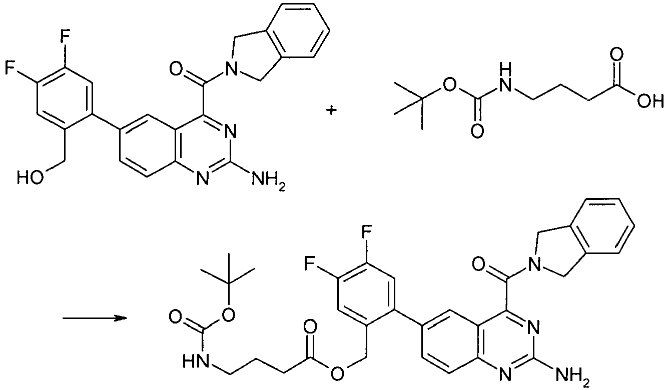

Zu einer Lösung von 1.88 g 4-tert.-Butoxycarbonylamino-buttersäure in 50 ml Dichlormethan gibt man 954 mg Dicyclohexylcarbodiimid und rührt 1 h bei 25°C. Es wird vom Niederschlag abfiltriert und zu einer Lösung von 1 g [2-Amino-6-(4,5-difluor-2-hydroxymethyl-phenyl)-chinazolin-4-yl]-(1,3-dihydro-isoindol-2-yl)-methanon und 14 mg 4-(Dimethylamino)-pyridin (DMAP) in 50 ml Tetrahydrofuran gegeben. Es wird für 48 h bei 25°C gerührt, vom Ungelösten filtriert und das Filtrat zur Trockne eingeengt. Der Rückstand wird chromatographisch an der Normalphase gereinigt.
Ausbeute: 1.2 g (84%) 4-tert.-Butoxycarbonylamino-buttersäure-2-[2-amino-4-(1,3-dihydro-isoindol-2-carbonyl)-chinazolin-6-yl]-4,5-difluor-benzylester; Retentionszeit LC-MS: 2.48 min.

### 4-Amino-buttersäure-2-[2-amino-4-(1,3-dihydro-isoindol-2-carbonyl)-chinazolin-6-yl]-4,5-difluor-benzylester Dihydrochlorid ("A90")

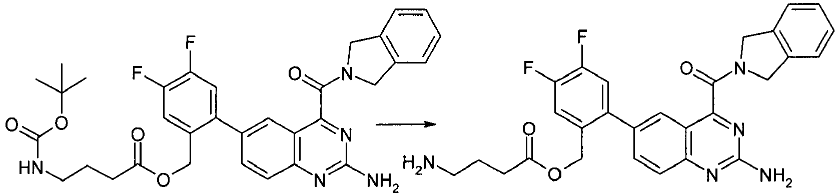

Zu 300 mg 4-tert.-Butoxycarbonylamino-buttersäure-2-[2-amino-4-(1,3-dihydro-isoindol-2-carbonyl)-chinazolin-6-yl]-4,5-difluor-benzylester in 5 ml Dichlormethan gibt man unter Eiskühlung 250 µl 4N HCl in Dioxan. Anschließend wird 1 h bei 25°C weitergerührt, der Niederschlag abgesaugt und mit 2 ml Dichlormethan gewaschen.
Ausbeute: 256 mg (89%) 4-Amino-buttersäure-2-[2-amino-4-(1,3-dihydro-isoindol-2-carbonyl)-chinazolin-6-yl]-4,5-difluor-benzylester Dihydrochlorid Retentionszeit LC-MS: 1.70 min;
¹H NMR (400 MHz, DMSO-d₆/TFA-d₁) δ [ppm] 8.09 - 8.03 (m, 2H), 7.91 - 7.86 (m, 1 H), 7.61 (dd, J = 11.4, 8.2, 1 H), 7.52 - 7.47 (m, 1 H), 7.45 (d, J = 8.8, 1 H), 7.33 (td, J = 13.2, 6.7, 2H), 7.26 (d, J = 6.9, 1H), 5.02 (s, 2H), 4.93 (s, 2H), 4.85 (s, 2H), 2.82 - 2.77 (m, 2H), 2.44 (t, J = 7.3, 2H), 1.82 - 1.73 (m, 2H).

### 3-(tert.-Butoxycarbonyl-methyl-amino)-propionsäure-2-[2-amino-4-(1,3-dihydro-isoindol-2-carbonyl)-chinazolin-6-yl]-4,5-difluor-benzylester ("A91")

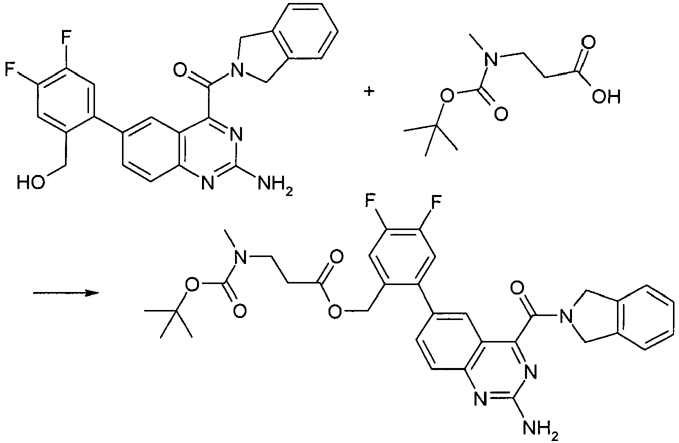

Zu einer Lösung von 1.88 g 4-tert.-Butoxycarbonylamino-propionsäure in 50 ml Dichlormethan gibt man 954 mg Dicyclohexylcarbodiimid und rührt 1 h bei 25°C. Es wird vom Niederschlag abfiltriert und zu einer Lösung von 1 g [2-Amino-6-(4,5-difluor-2-hydroxymethyl-phenyl)-chinazolin-4-yl]-(1,3-dihydro-isoindol-2-yl)-methanon und 14 mg 4-(Dimethylamino)-pyridin (DMAP) in 50 ml Tetrahydrofuran gegeben. Es wird für 48 h bei 25°C gerührt, vom Ungelösten filtriert und das Filtrat zur Trockne eingeengt. Der Rückstand wird chromatographisch an der Normalphase gereinigt.
Ausbeute: 840 mg (59%) 3-(tert.-Butoxycarbonyl-methyl-amino)-propionsäure-2-[2-amino-4-(1,3-dihydro-isoindol-2-carbonyl)-chinazolin-6-yl]-4,5-difluor-benzylester; Retentionszeit LC-MS: 2.54 min;
¹H NMR (400 MHz, DMSO-d₆/TFA-d₁) δ [ppm] 8.11 (d, J = 1.9, 1 H), 8.08 - 8.03 (m, 1 H), 7.88 (d, J = 8.7, 1 H), 7.62 - 7.52 (m, 1 H), 7.43 (dd, J = 12.8, 6.2, 2H), 7.32 (dt, J = 14.5, 7.3, 2H), 7.25 (d, J = 7.3, 1 H), 5.04 (s, 2H), 4.93 (s, 2H), 4.87 (s, 2H), 3.35 (t, J = 7.0, 2H), 2.74 (s, 3H), 2.54 - 2.44 (m, 2H), 1.36 (d, J = 6.7, 9H).

### 3-Methylamino-propionsäure-2-[2-amino-4-(1,3-dihydro-isoindol-2-carbonyl)-chinazolin-6-yl]-4,5-difluor-benzylester Dihydrochlorid ("A92")

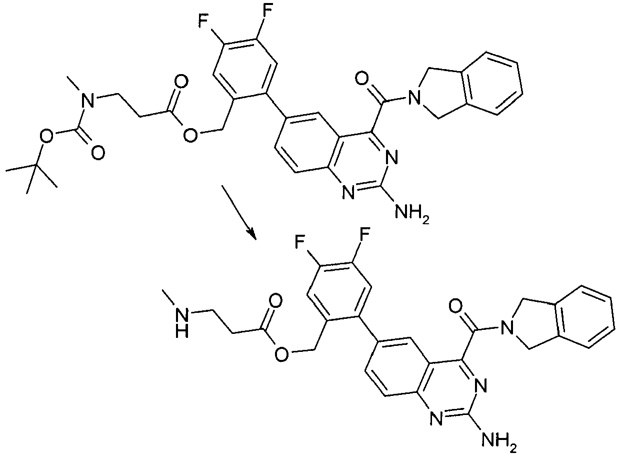

Zu 300 mg 3-(tert.-Butoxycarbonyl-methyl-amino)-propionsäure-2-[2-amino-4-(1,3-dihydro-isoindol-2-carbonyl)-chinazolin-6-yl]-4,5-difluor-benzylester in 5 ml Dichlormethan gibt man unter Eiskühlung 242 µl 4N HCl in Dioxan. Anschließend wird 1 h bei 25°C weitergerührt, der Niederschlag abgesaugt und mit 2 ml Dichlormethan gewaschen.
Ausbeute: 260 mg (97%) 3-Methylamino-propionsäure-2-[2-amino-4-(1,3-dihydro-isoindol-2-carbonyl)-chinazolin-6-yl]-4,5-difluor-benzylester Dihydrochlorid; Retentionszeit LC-MS: 1.64 min;
¹H NMR (400 MHz, DMSO-d₆/TFA-d₁) δ [ppm] 8.10 (d, J = 1.9, 1H), 8.07 (dd, J = 8.7, 1.9, 1 H), 7.90 (d, J = 8.7, 1 H), 7.63 (dd, J = 11.4, 8.3, 1 H), 7.44 (d, J = 7.6, 2H), 7.33 (dt, J = 14.5, 7.3, 2H), 7.26 (d, J = 7.3, 1H), 5.05 (s, 2H), 4.98 (s, 2H), 4.88 (s, 2H), 3.15 (t, J = 7.0, 2H), 2.80 (t, J = 7.0, 2H), 2.61 (s, 3H).

### (R)-3-tert.-Butoxy-2-tert.-butoxycarbonylamino-propionsäure-2-[2-amino-4-(1,3-dihydro-isoindol-2-carbonyl)-chinazolin-6-yl]-4,5-difluor-benzylester ("A93")

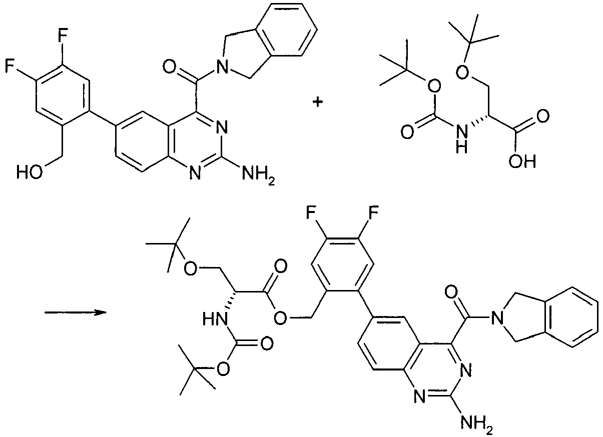

Zu einer Lösung von 2.4 g (R)-3-tert.-Butoxy-2-tert.-butoxycarbonylamino-propionsäure in 50 ml Dichlormethan gibt man 954 mg Dicyclohexylcarbodiimid und rührt 1 h bei 25°C. Es wird vom Niederschlag abfiltriert und zu einer Lösung von 1 g [2-Amino-6-(4,5-difluor-2-hydroxymethyl-phenyl)-chinazolin-4-yl]-(1,3-dihydro-isoindol-2-yl)-methanon und 14 mg 4-(Dimethylamino)-pyridin (DMAP) in 50 ml Tetrahydrofuran gegeben. Es wird für 48 h bei 25°C gerührt, vom Ungelösten filtriert und das Filtrat zur Trockne eingeengt. Der Rückstand wird chromatographisch an der Normalphase gereinigt.
Ausbeute: 1.2 g (79%) (R)-3-tert.-Butoxy-2-tert.-butoxycarbonylamino-propionsäure-2-[2-amino-4-(1,3-dihydro-isoindol-2-carbonyl)-chinazolin-6-yl]-4,5-difluor-benzylester; Retentionszeit LC-MS: 2.79 min.

Analog erhält man:

### (R)-2-Amino-3-hydroxy-propionsäure-2-[2-amino-4-(1,3-dihydro-isoindol-2-carbonyl)-chinazolin-6-yl]-4,5-difluor-benzylester Dihydrochlorid ("A96")

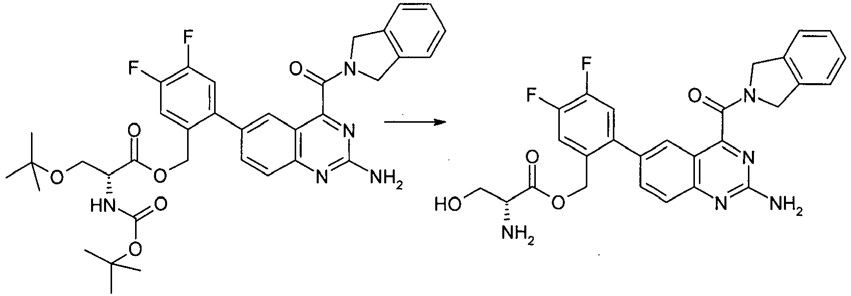

Zu 850 mg 3-(tert.-Butoxycarbonyl-methyl-amino)-propionsäure-2-[2-amino-4-(1,3-dihydro-isoindol-2-carbonyl)-chinazolin-6-yl]-4,5-difluor-benzylester in 20 ml Dichlormethan gibt man unter Eiskühlung 1.26 ml 4N HCl in Dioxan. Anschließend wird 1 h bei 25°C weitergerührt, im Vakuum zur Trockne eingeengt und aus Ethanol umkristallisiert.
Ausbeute: 400 mg (54%) (R)-2-Amino-3-hydroxy-propionsäure-2-[2-amino-4-(1,3-dihydro-isoindol-2-carbonyl)-chinazolin-6-yl]-4,5-difluor-benzylester Dihydrochlorid; Retentionszeit LC-MS: 1.60 min;
¹H NMR (400 MHz, DMSO-d₆/TFA-d₁) δ [ppm] 8.14 (d, J = 1.5, 1 H), 8.09 (dd, J = 8.7, 2.0, 1 H), 7.88 (d, J = 8.7, 1 H), 7.72 (dd, J = 11.4, 8.2, 1 H), 7.50 (dd, J = 11.0, 7.9, 1 H), 7.44 (d, J = 7.0, 1 H), 7.34 (dt, J = 13.4, 6.6, 2H), 7.27 (d, J = 7.0, 1 H), 5.10 (q, J = 12.7, 2H), 5.04 (s, 2H), 4.88 (s, 2H), 4.27 (t, J = 3.5, 1 H), 3.84 (ddd, J = 34.3, 11.7, 3.6, 2H).

Analog erhält man:

### (tert.-Butoxycarbonyl-methyl-amino)-essigsäure-2-[2-amino-4-(1,3-dihydro-isoindol-2-carbonyl)-chinazolin-6-yl]-4,5-difluor-benzylester ("A105")

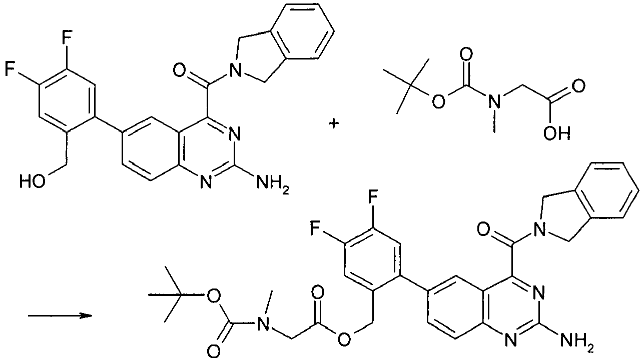

Zu einer Lösung von 2.625 g (tert.-Butoxycarbonyl-methyl-amino)-essigsäure in 100 ml Dichlormethan gibt man 1.43 g Dicyclohexylcarbodiimid und rührt 1 h bei 25°C. Es wird vom Niederschlag abfiltriert und zu einer Lösung von 1.5 g [2-Amino-6-(4,5-difluor-2-hydroxymethyl-phenyl)-chinazolin-4-yl]-(1,3-dihydro-isoindol-2-yl)-methanon und 14 mg 4-(Dimethylamino)-pyridin (DMAP) in 100 ml Tetrahydrofuran gegeben. Es wird für 48 h bei 25°C gerührt, vom Ungelösten filtriert und das Filtrat zur Trockne eingeengt. Der Rückstand wird chromatographisch an der Normalphase gereinigt.
Ausbeute: 1.0 g (48%) (tert.-Butoxycarbonyl-methyl-amino)-essigsäure-2-[2-amino-4-(1,3-dihydro-isoindol-2-carbonyl)-chinazolin-6-yl]-4,5-difluor-benzylester; Retentionszeit LC-MS: 2.49 min;
¹H NMR (500 MHz, DMSO-d₆/TFA-d₁) δ [ppm] 8.11 (d, J = 2.1, 1H), 8.08 - 8.04 (m, 1 H), 7.88 (dd, J = 8.6, 4.2, 1 H), 7.60 (ddd, J = 22.1, 11.2, 8.2, 1 H), 7.46 (dd, J = 12.8, 7.9, 2H), 7.33 (dt, J = 17.5, 7.2, 2H), 7.25 (t, J = 6.1, 1 H), 5.05 (s, 2H), 5.01 (s, 2H), 4.87 (d, J = 8.3, 2H), 3.96 - 3.83 (m, 2H), 2.79 (m, 3H), 1.43 - 1.23 (m, 9H).

### Methyl-aminoessigsäure-2-[2-amino-4-(1,3-dihydro-isoindol-2-carbonyl)-chinazolin-6-yl]-4,5-difluor-benzylester Dihydrochlorid ("A108")

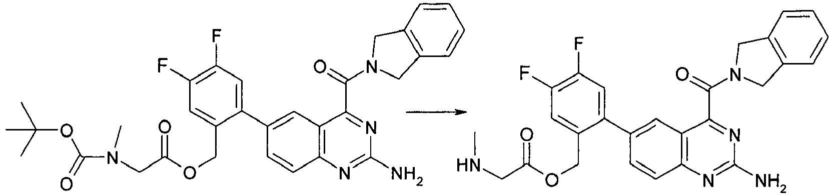

Zu 1 g (tert.-Butoxycarbonyl-methyl-amino)-essigsäure-2-[2-amino-4-(1,3-dihydro-isoindol-2-carbonyl)-chinazolin-6-yl]-4,5-difluor-benzylester in 20 ml Dichlormethan gibt man unter Eiskühlung 3.3 ml 4N HCl in Dioxan. Anschließend wird 1 h bei 25°C weitergerührt, der Niederschlag abfiltriert und mit 3 ml Dichlormethan nachgewaschen.
Ausbeute: 940 mg (98%) Methyl-aminoessigsäure-2-[2-amino-4-(1,3-dihydro-isoindol-2-carbonyl)-chinazolin-6-yl]-4,5-difluor-benzylester Dihydrochlorid; Retentionszeit LC-MS: 1.65 min;
¹H NMR (400 MHz, DMSO-d₆/TFA-d₁) δ [ppm] 8.15 (s, 1H), 8.05 (d, J = 8.7, 1H), 7.91 (d, J = 8.7, 1H), 7.70 - 7.61 (m, 1H), 7.49 - 7.28 (m, 4H), 7.25 (d, J = 7.3, 1 H), 5.07 (s, 2H), 5.06 (s, 2H), 4.90 (s, 2H), 4.08 (s, 2H), 2.68 (s, 3H).

### Dimethyl-aminoessigsäure-2-[2-amino-4-(1,3-dihydro-isoindol-2-carbonyl)-chinazolin-6-yl]-4,5-difluor-benzylester Dihydrochlorid ("A112")

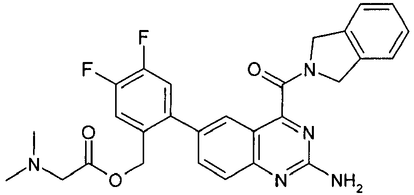

Ausbeute: 390 mg (29%) Dimethyl-aminoessigsäure-2-[2-amino-4-(1,3-dihydro-isoindol-2-carbonyl)-chinazolin-6-yl]-4,5-difluor-benzylester Dihydrochlorid; Retentionszeit LC-MS: 1.71 min;
¹H NMR (400 MHz, DMSO-d₆/TFA-d₁) δ [ppm] 8.07 (d, J = 1.7, 1H), 8.02 (dd, J = 8.6, 1.9, 1 H), 7.85 (d, J = 8.7, 1 H), 7.66 (dd, J = 11.4, 8.2, 1 H), 7.46 (dd, J = 11.0, 8.1, 1 H), 7.40 (d, J = 7.2, 1 H), 7.29 (dt, J = 14.5, 7.2, 2H), 7.21 (d, J = 7.0, 1 H), 5.02 (s, 2H), 5.00 (s, 2H), 4.83 (s, 2H), 4.25 (s, 2H), 2.84 (s, 6H).

### Dimethyl-carbaminsäure-2-[2-amino-4-(1,3-dihydro-isoindol-2-carbonyl)-chinazolin-6-yl]-4,5-difluor-benzylester ("A114")

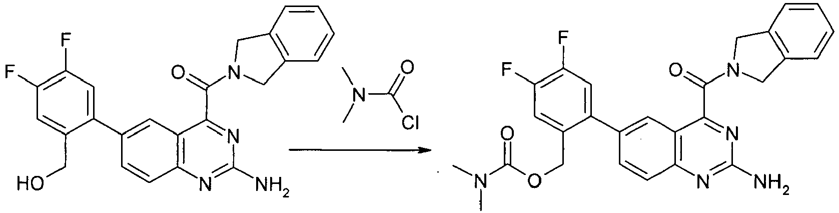

Zu einer Lösung von 100 mg [2-Amino-6-(4,5-difluor-2-hydroxymethyl-phenyl)-chinazolin-4-yl]-(1,3-dihydro-isoindol-2-yl)-methanon und 2 mg 4-(Dimethyl-amino)-pyridin (DMAP) in 5 ml Dimethylformamid (DMF) gibt man 20 µl Dimethyl-carbaminsäurechlorid und erhitzt für 18 h auf 80°C. Nach 18 h werden 10 ml Methanol zugesetzt, im Vakuum zur Trockne eingeengt und chromatographisch an der Normalphase gereinigt.
Ausbeute: 26 mg (22%) Dimethyl-carbaminsäure-2-[2-amino-4-(1,3-dihydro-isoindol-2-carbonyl)-chinazolin-6-yl]-4,5-difluor-benzylester;
Retentionszeit LC-MS: 2.23 min;
¹H NMR (500 MHz, DMSO-d₆) δ [ppm] 8.07 (dd, J = 15.5, 5.2, 2H), 7.87 (d, J = 8.6, 1 H), 7.58 (d, J = 8.3, 1 H), 7.44 (d, J = 6.2, 2H), ), 7.38 - 7.27 (m, 2H), 7.21 (d, J = 7.2, 1 H), 4.98 (s, 2H), 4.86 (s, 2H), 4.82 (s, 2H), 2.73 (s, 3H), 2.66 (s, 3H).

### (2-Dimethylamino-ethyl)-carbaminsäure-2-[2-amino-4-(1,3-dihydro-isoindol-2-carbonyl)-chinazolin-6-yl]-4,5-difluor-benzylester ("A115")

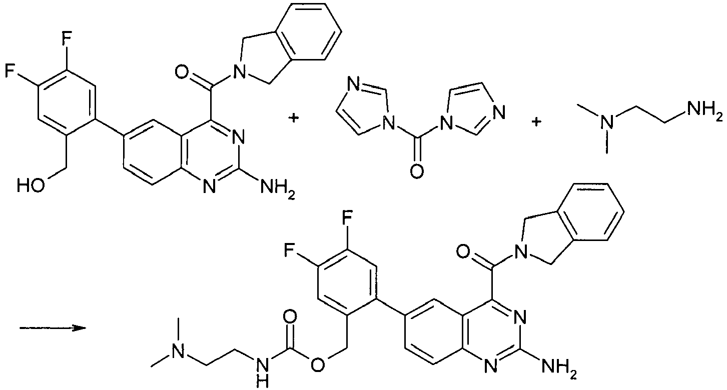

400 mg 1,1'-Carbonyldiimidazol werden in 10 ml Pyridin gelöst und auf 0° C gekühlt. Hierzu wird eine Lösung von 1.0 g [2-Amino-6-(4,5-difluor-2-hydroxymethyl-phenyl)-chinazolin-4-yl]-(1,3-dihydro-isoindol-2-yl)-methanon gelöst in 10 ml Pyridin gegeben. Anschließend wird 2 h bei 0°C und 2 h bei 25°C gerührt. Dann werden 300 µl N,N-Dimethylethylendiamin dazugegeben und 18 h bei 25°C weitergerührt. Man gibt auf 250 ml 1 N HCl, neutralisiert mit Bicarbonatlösung und wäscht 3mal mit je 150 ml Dichlormethan. Die organischen Phasen werden 2mal mit je 50 ml Wasser gewaschen, über Natriumsulfat getrocknet, filtriert und im Vakuum zur Trockne eingeengt. Der Rückstand wird in 1 ml Dimethylsulfoxid gelöst und chromatographisch (reversed phase) gereinigt.
Ausbeute: 366 mg (29%) (2-Dimethylamino-ethyl)-carbaminsäure-2-[2-amino-4-(1,3-dihydro-isoindol-2-carbonyl)-chinazolin-6-yl]-4,5-difluor-benzylester; Retentionszeit LC-MS: 1.72 min;
¹H NMR (500 MHz, DMSO-d₆/TFA-d₁) δ [ppm] 8.00 (s, 1H), 7.95 (dd, J = 8.6, 1.8, 1 H), 7.79 (d, J = 8.6, 1 H), 7.43 (dd, J = 18.2, 8.5, 1 H), 7.32 (d, J = 7.3, 1 H), 7.29 - 7.18 (m, 3H), 7.14 (d, J = 7.3, 1 H), 4.97 (s, 2H), 4.82 (s, 2H), 4.79 (s, 2H), 3.25 (t, J = 5.9, 2H), 3.07 (t, J = 6.0, 2H), 2.73 (s, 6H).

### 4-Methyl-piperazin-1-carbonsäure-2-[2-amino-4-(1,3-dihydro-isoindol-2-carbonyl)-chinazolin-6-yl]-4,5-difluor-benzylester ("A116")

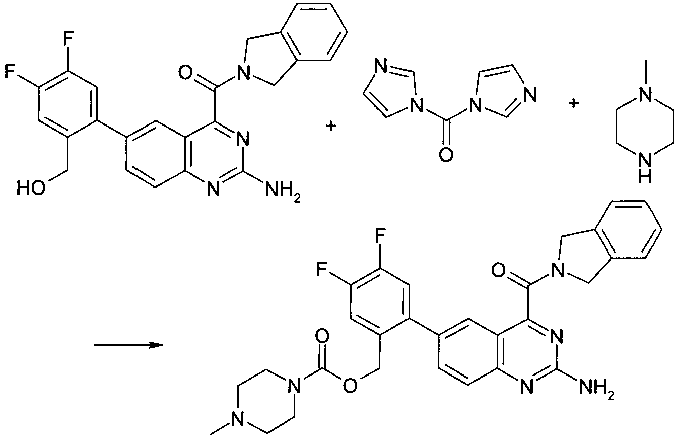

400 mg 1,1'-Carbonyldiimidazol werden in 10 ml Pyridin gelöst und auf 0° C gekühlt. Hierzu wird eine Lösung von 1.0 g [2-Amino-6-(4,5-difluor-2-hydroxymethyl-phenyl)-chinazolin-4-yl]-(1,3-dihydro-isoindol-2-yl)-methanon gelöst in 10 ml Pyridin gegeben. Anschließend wird 2 h bei 0°C und 2 h bei 25°C gerührt. Dann werden 300 µl N-Methylpiperazin dazugegeben und 18 h bei 25°C weitergerührt. Man gibt auf 250 ml 1 N HCl, neutralisiert mit Bicarbonatlösung und wäscht 3mal mit je 150 ml Dichlormethan. Die organischen Phasen werden 2mal mit je 50 ml Wasser gewaschen, über Natriumsulfat getrocknet, filtriert und im Vakuum zur Trockne eingeengt. Der Rückstand wird in 1 ml Dimethylsulfoxid gelöst und chromatographisch (reversed phase) gereinigt.
Ausbeute: 352 mg (27%) 4-Methyl-piperazin-1-carbonsäure-2-[2-amino-4-(1,3-dihydro-isoindol-2-carbonyl)-chinazolin-6-yl]-4,5-difluor-benzylester; Retentionszeit LC-MS: 1.73 min;
¹H NMR (500 MHz, DMSO-d₆) δ [ppm] 7.75 - 7.71 (m, 1 H), 7.58 - 7.51 (m, 2H), 7.48 - 7.40 (m, 2H), 7.32 (t, J = 7.2, 1 H), 7.27 (q, J = 7.3, 2H), 7.17 (s, 1 H), 4.95 (s, 2H), 4.94 (s, 2H), 4.73 (s, 2H), 3.39 - 3.16 (m, 3H), 3.18 (dd, J = 8.2, 4.9, 4H), 2.11 (s, 4H).

### (2-Amino-ethyl)-carbaminsäure-2-[2-amino-4-(1,3-dihydro-isoindol-2-carbonyl)-chinazolin-6-yl]-4,5-difluor-benzylester ("A117")

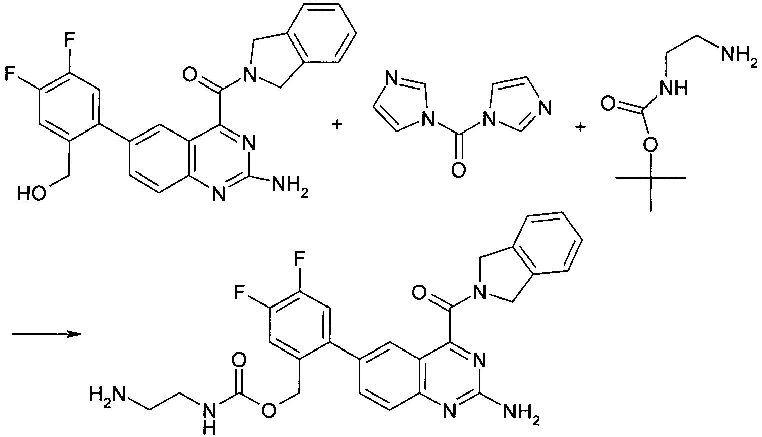

400 mg 1,1'-Carbonyldiimidazol werden in 10 ml Pyridin gelöst und auf 0° C gekühlt. Hierzu wird eine Lösung von 1.0 g [2-Amino-6-(4,5-difluor-2-hydroxymethyl-phenyl)-chinazolin-4-yl]-(1,3-dihydro-isoindol-2-yl)-methanon gelöst in 10 ml Pyridin gegeben. Anschließend wird 2 h bei 0°C und 2 h bei 25°C gerührt. Dann werden 400 mg N-Boc-ethylendiamin dazugegeben und 18 h bei 25°C weitergerührt. Man gibt auf 250 ml 1 N HCl und rührt 3 h bei 25°C, neutralisiert mit Bicarbonatlösung und wäscht 3mal mit je 150 ml Dichlormethan. Die organischen Phasen werden 2mal mit je 50 ml Wasser gewaschen, über Natriumsulfat getrocknet, filtriert und im Vakuum zur Trockne eingeengt. Der Rückstand wird in 1 ml Dimethylsulfoxid gelöst und chromatographisch (reversed phase) gereinigt.
Ausbeute: 286 mg (44%) (2-Amino-ethyl)-carbaminsäure-2-[2-amino-4-(1,3-dihydro-isoindol-2-carbonyl)-chinazolin-6-yl]-4,5-difluor-benzylester; Retentionszeit LC-MS: 1.65 min,
¹H NMR (400 MHz, DMSO-d₆/TFA-d₁) δ [ppm] 8.09 - 8.03 (m, 2H), 7.84 (d, J = 8.7, 1 H), 7.57 (t, J = 9.9, 1 H), 7.49 (d, J = 9.8, 1 H), 7.44 (d, J = 7.8, 1 H), 7.33 (dt, J = 14.7, 7.3, 2H), 7.26 (d, J = 7.3, 1H), 5.02 (s, 2H), 4.88 (s, 2H), 4.85 (s, 2H), 3.20 (t, J = 6.3, 2H), 2.85 (t, J = 6.2, 2H).

### Carbonsäure-2-[2-amino-4-(1,3-dihydro-isoindol-2-carbonyl)-chinazolin-6-yl]-4,5-difluor-benzylester-2-tert.-butoxycarbonylamino-ethylester ("A118")

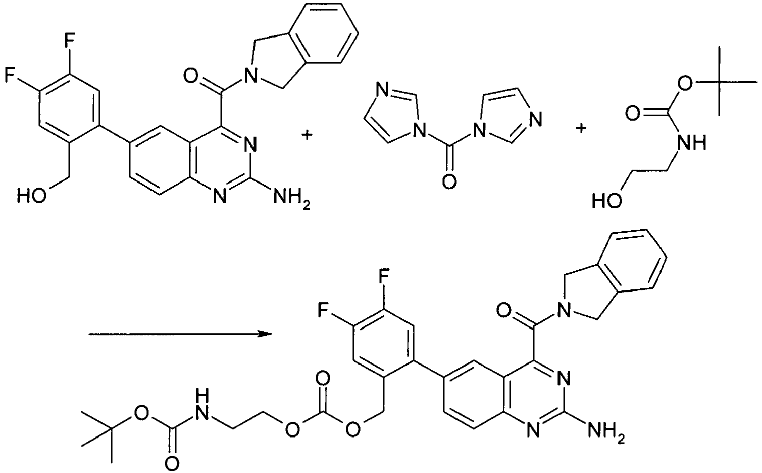

400 mg 1,1'-Carbonyldiimidazol werden in 10 ml Pyridin gelöst und auf 0° C gekühlt. Hierzu wird eine Lösung von 1.0 g [2-Amino-6-(4,5-difluor-2-hydroxymethyl-phenyl)-chinazolin-4-yl]-(1,3-dihydro-isoindol-2-yl)-methanon gelöst in 10 ml Pyridin gegeben. Anschließend wird 2 h bei 0°C und 2 h bei 25°C gerührt. Dann werden 400 mg tert.-Butyl-N-(2-hydroxyethyl)carbamat und 400 µl 1,8-Diazabicyclo[5.4.0]undec-7-en dazugegeben und 18 h bei 25°C weitergerührt. Man gibt auf 250 ml 1 N HCl, neutralisiert mit Bicarbonatlösung und wäscht 3mal mit je 150 ml Dichlormethan. Die organischen Phasen werden 2mal mit je 50 ml Wasser gewaschen, über Natriumsulfat getrocknet, filtriert und im Vakuum zur Trockne eingeengt. Der Rückstand wird in 1 ml Dimethylsulfoxid gelöst und chromatographisch (reversed phase) gereinigt. Ausbeute: 286 mg (44%) Carbonsäure-2-[2-amino-4-(1,3-dihydro-isoindol-2-carbonyl)-chinazolin-6-yl]-4,5-difluor-benzylester-2-tert.-butoxycarbonylamino-ethylester.
Retentionszeit LC-MS: 2.43 min;
¹H NMR (400 MHz, DMSO-d₆/TFA-d₁) δ [ppm] 8.08 - 8.01 (m, 1H), 7.91 (d, J = 8.7, 1 H), 7.55 (dd, J = 18.6, 10.2, 1 H), 7.50 - 7.26 (m, 5H), 7.23 (d, J = 7.5, 1 H), 5.07 (s, 2H), 4.98 (s, 2H), 4.88 (s, 2H), 4.02 (t, J = 5.4, 2H), 3.21 (t, J = 5.5, 2H), 1.40 (s, 9H).

### Carbonsäure-2-[2-amino-4-(1,3-dihydro-isoindol-2-carbonyl)-chinazolin-6-yl]-4,5-difluor-benzylester-2-amino-ethylester ("A119")

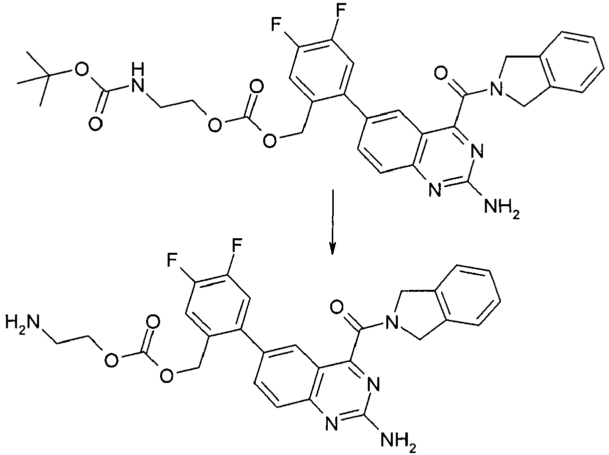

Zu 371 mg Carbonsäure-2-[2-amino-4-(1,3-dihydro-isoindol-2-carbonyl)-chinazolin-6-yl]-4,5-difluor-benzylester-2-tert.-butoxycarbonylamino-ethylester in 4 ml Dichlormethan gibt man unter Eiskühlung 2 ml Trifluoressigsäure. Anschließend wird 2 h bei 25°C weitergerührt, mit 5 ml n-Heptan versetzt und im Vakuum zur Trockne eingeengt. Der Rückstand wird in 500 µl Dimethyl-sulfoxid gelöst und chromatographisch (reversed phase) gereinigt.
Ausbeute: 267 mg (86%) Carbonsäure-2-[2-amino-4-(1,3-dihydro-isoindol-2-carbonyl)-chinazolin-6-yl]-4,5-difluor-benzylester-2-amino-ethylester; Retentionszeit LC-MS: 1.68 min;
¹H NMR (400 MHz, DMSO-d₆/TFA-d₁) δ [ppm] 8.10 (s, 1H), 8.06 (dd, J = 8.7, 1.9, 1 H), 7.90 (d, J = 8.7, 1 H), 7.59 (dd, J = 11.3, 8.2, 1H), 7.48 - 7.39 (m, 2H), 7.38 - 7.28 (m, 2H), 7.25 (d, J = 7.0, 1 H), 5.06 (s, 2H), 5.05 (s, 2H), 4.89 (s, 2H), 4.30 - 4.24 (m, 2H), 3.17 - 3.11 (m, 2H).

### Carbonsäure-2-[2-amino-4-(1,3-dihydro-isoindol-2-carbonyl)-chinazolin-6-yl]-4,5-difluor-benzylester-2-dimethylamino-ethylester ("A120")

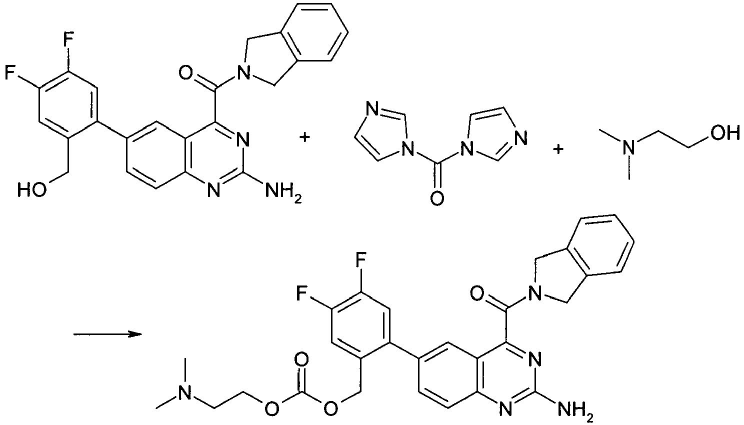

200 mg 1,1'-Carbonyldiimidazol werden in 10 ml Pyridin gelöst und auf 0° C gekühlt. Hierzu wird eine Lösung von 500 mg [2-Amino-6-(4,5-difluor-2-hydroxymethyl-phenyl)-chinazolin-4-yl]-(1,3-dihydro-isoindol-2-yl)-methanon gelöst in 10 ml Pyridin gegeben. Anschließend wird 2 h bei 0°C und 2 h bei 25°C gerührt. Dann werden 200 mg 2-(Dimethylamino)-ethanol und 200 µl 1,8-Diazabicyclo[5.4.0]undec-7-en dazugegeben und 18 h bei 25°C weitergerührt. Man gibt auf 250 ml 1 N HCl, neutralisiert mit Bicarbonatlösung und wäscht 3mal mit je 150 ml Dichlormethan. Die organischen Phasen werden 2mal mit je 50 ml Wasser gewaschen, über Natriumsulfat getrocknet, filtriert und im Vakuum zur Trockne eingeengt. Der Rückstand wird in 1 ml Dimethylsulfoxid gelöst und chromatographisch (reversed phase) gereinigt.
Ausbeute: 90 mg (14%) Carbonsäure-2-[2-amino-4-(1,3-dihydro-isoindol-2-carbonyl)-chinazolin-6-yl]-4,5-difluor-benzylester-2-dimethylamino-ethylester. Retentionszeit LC-MS: 1.71 min;
¹H NMR (400 MHz, DMSO-d₆/TFA-d₁) δ [ppm] 8.05 (m, 2H), 7.85 (d, J = 9.4, 1 H), 7.66 - 7.58 (m, 1 H), 7.56 - 7.48 (m, 1 H), 7.45 (d, J = 7.2, 1 H), 7.39 - 7.28 (m, 2H), 7.26 (d, J = 6.9, 1 H), 5.04 (s, 2H), 5.02 (s, 2H), 4.85 (s, 2H), 4.38 - 4.34 (m, 2H), 3.44 - 3.38 (m, 2H), 2.54 (s, 6H).

### [2-[2-Amino-4-(isoindolin-2-carbonyl)chinazolin-6-yl]-4,5-difluor-phenyl]methyl-di-tert.-butylphosphat ("A121")

### a) Phosphorsäure-di-tert-butylester

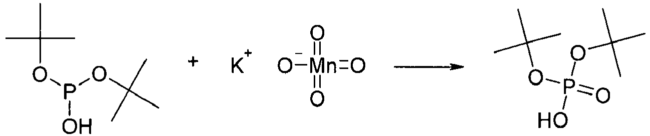

Zu einer Lösung von 10 ml Di-tert.-butyl-phosphit und 3 g Kaliumhydrogencarbonat in 50 ml Wasser gibt man portionsweise bei 0°C 5.6 g Kaliumpermanganat. Danach lässt man auf 25°C erwärmen und rührt 1 h. Anschließend wird für 15 min auf 60°C erhitzt und vom Niederschlag abfiltriert. Durch Ansäuern des Filtrats unter Eiskühlung durch tropfenweise Zugabe von konzentrierter Salzsäure wird das Produkt ausgefällt. Das Produkt wird abfiltriert, mit 5 ml Wasser gewaschen und im Vakuum über Phosphorpentoxid getrocknet; Ausbeute: 6.3 g (63%) Phosphorsäure-di-tert.-butylester.

### b) Tetrabutylammonium-phosphorsäure-di-tert.-butylester

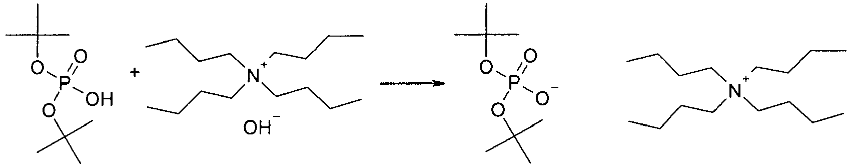

41.9 ml Tetra-n-butylammoniumhydroxid (20%ige Lösung in Wasser) in 50 ml Wasser gelöst und unter Rühren und Eiskühlung 6.8 g Phosphorsaeure-di-tert.-butylester (75%ig), gelöst in 5 ml Aceton, tropfenweise zugegeben. Die erhaltene Lösung wird lyophilisiert; Ausbeute: 14.6 g (99%) Tetrabutylammonium-di-tert.-butylester-phosphat (Gehalt ca. 40%).

### c) [2-Amino-6-(2-chlormethyl-4,5-difluor-phenyl)-chinazolin-4-yl]-(1,3-dihydro-isoindol-2-yl)-methanon

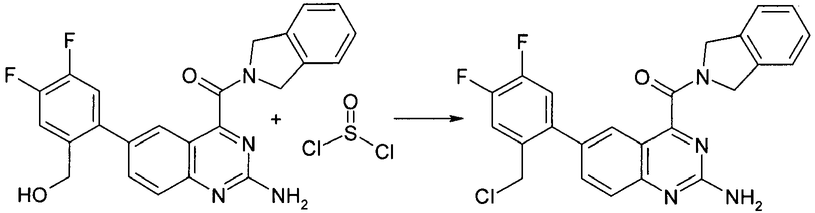

2 g [2-Amino-6-(4,5-difluor-2-hydroxymethyl-phenyl)-chinazolin-4-yl]-(1,3-dihydro-isoindol-2-yl)-methanon werden mit 50 ml Thionylchlorid versetzt und 1 h bei 25°C gerührt. Anschließend wird im Vakuum zur Trockne eingeengt, der Rückstand noch je 2mal mit je 50 ml Dichlormethan aufgenommen und erneut im Vakuum zur Trocken eingeengt.
Ausbeute: 2.2 g (97%) [2-Amino-6-(2-chlormethyl-4,5-difluor-phenyl)-chinazolin-4-yl]-(1,3-dihydro-isoindol-2-yl)-methanon;
Retentionszeit LC-MS: 2.33 min.

### d) [2-[2-Amino-4-(isoindolin-2-carbonyl)chinazolin-6-yl]-4,5-difluorphenyl]methyl-di-tert.-butylphosphat

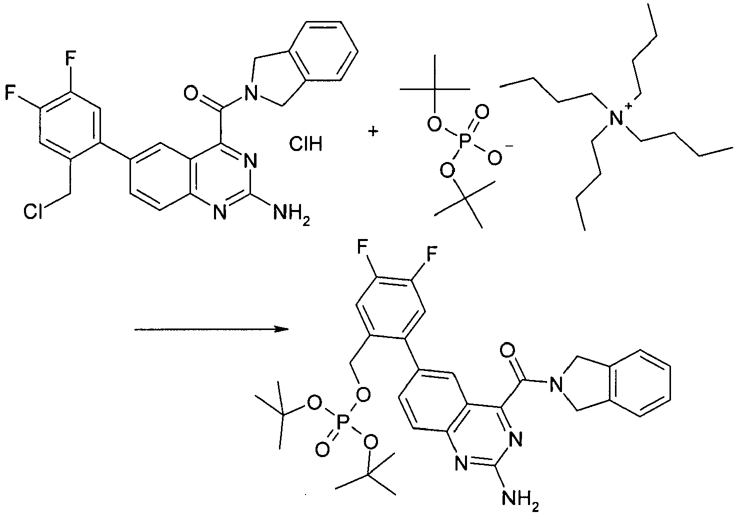

Zu 270 mg [2-Amino-6-(2-chlormethyl-4,5-difluor-phenyl)-chinazolin-4-yl]-(1,3-dihydro-isoindol-2-yl)-methanon gelöst in 20 ml Acetonitril werden 800 mg Tetrabutylammonium-di-tert.butylester-phosphat gegeben und die erhaltene Lösung für 2 h auf 80°C erhitzt. Nach Abkühlen auf 25°C wird vom Ungelösten abfiltriert und das Filtrat im Vakuum zur Trockne eingeengt. Der Rückstand wird in 1 ml Dimethylsulfoxid gelöst und chromatographisch (reversed phase) gereinigt.
Ausbeute: 1.3 g (46%) [2-[2-Amino-4-(isoindolin-2-carbonyl)chinazolin-6-yl]-4,5-difluor-phenyl]methyl-di-tert.-butylphosphat;
Retentionszeit LC-MS: 2.47 min;
¹H NMR (500 MHz, DMSO-d₆/TFA-d₁) δ [ppm] 8.13 (d, J = 1.8, 1 H), 8.07 (dd, J = 8.6, 1.8, 1 H), 7.89 (dd, J = 8.9, 2.8, 1 H), 7.55 (dd, J = 11.4, 8.3, 1 H), 7.46 - 7.39 (m, 2H), 7.32 (dt, J = 14.7, 7.1, 2H), 7.24 (d, J = 7.3, 1H), 5.06 (s, 2H), 4.84 (dd, J = 21.2, 12.6, 4H), 1.31 (s, 18H).

### [2-[2-Amino-4-(isoindolin-2-carbonyl)chinazolin-6-yl]-4,5-difluor-phenyl]methyl dihydrogenphosphat ("A122")

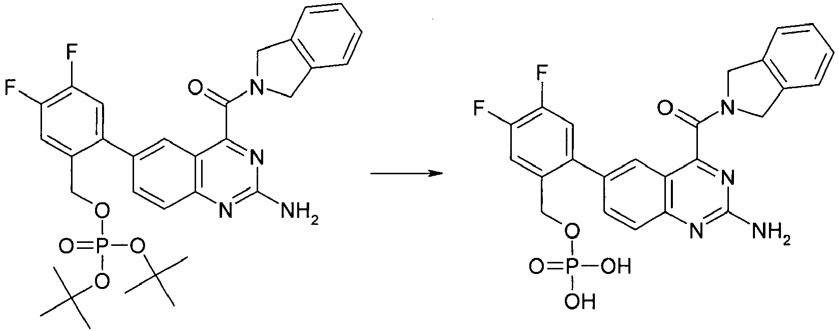

1.3 g [2-[2-Amino-4-(isoindolin-2-carbonyl)chinazolin-6-yl]-4,5-difluorphenyl]methyl-di-tert.-butylphosphat werden in 20 ml Trifluoressigsäure für 1 h bei 25°C gerührt. Man engt im Vakuum bei 25°C ein und nimmt den Rückstand in 100 ml Wasser auf. Mit Bicarbonatlösung wird pH 6 eingestellt und der entstehende Niederschlag abfiltriert. Trocknen im Vakuum bei 40°C liefert Produkt.
Ausbeute: 1.3 g (46%) [2-[2-Amino-4-(isoindolin-2-carbonyl)chinazolin-6-yl]-4,5-difluor-phenyl]methyl-dihydrogenphosphat;
Retentionszeit LC-MS: 2.47 min;
¹H NMR (500 MHz, DMSO-d₆/TFA-d₁) δ [ppm] 8.04 (d, J = 1.8, 1H), 8.01 - 7.97 (m, 1 H), 7.81 (d, J = 8.6, 1 H), 7.51 (dd, J = 11.2, 8.4, 1 H), 7.34 (dd, J = 11.4, 7.9, 2H), 7.29 - 7.20 (m, 2H), 7.18 (d, J = 7.3, 1 H), 4.98 (s, 2H), 4.83 (s, 2H), 4.75 (d, J = 7.3, 2H).

### 2-[2-Amino-4-(isoindolin-2-carbonyl)chinazolin-6-yl]-4,5-difluor-phenyl]methyl diethylphosphat ("A123")

### a) Tetrabutylammonium-phosphorsäure-diethylester

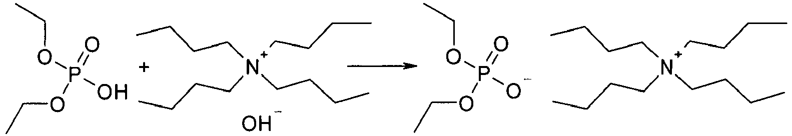

12.8 ml Tetra-n-butylammoniumhydroxid (20%ige Lösung in Wasser) wird in 20 ml Wasser gelöst und unter Rühren und Eiskühlung 1 g Phosphorsäure-diethylester (75%ig), gelöst in 2 ml Aceton, tropfenweise zugegeben. Die erhaltene Lösung wird lyophilisiert.
Ausbeute: 3.3 g Tetrabutylammonium-phosphorsäure-diethylester (Gehalt ca. 40%).

### b) [2-[2-Amino-4-(isoindolin-2-carbonyl)chinazolin-6-yl]-4,5-difluorphenyl]methyl-diethylphosphat

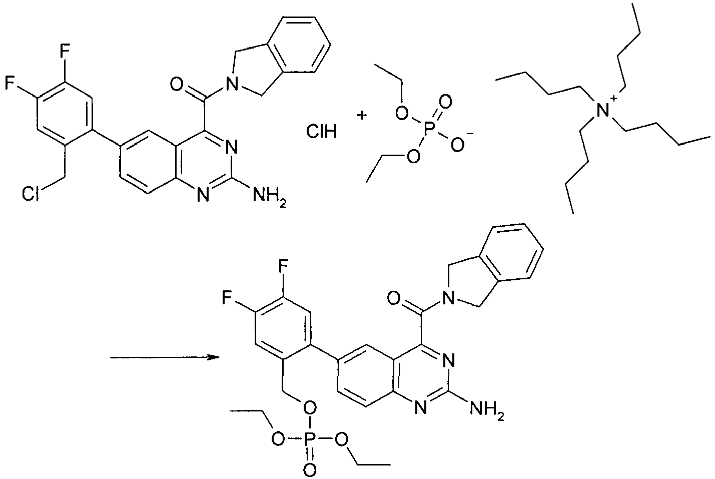

Zu 270 mg [2-Amino-6-(2-chlormethyl-4,5-difluor-phenyl)-chinazolin-4-yl]-(1,3-dihydro-isoindol-2-yl)-methanon gelöst in 20 ml Acetonitril werden 800 mg Tetrabutylammonium-di-ethylester-phosphat gegeben und die erhaltene Lösung für 2 h auf 80°C erhitzt. Nach Abkühlen auf 25°C wird vom Ungelösten abfiltriert und das Filtrat im Vakuum zur Trockne eingeengt. Der Rückstand wird in 1 ml Dimethylsulfoxid gelöst und chromatographisch (reversed phase) gereinigt.
Ausbeute: 109 mg (32%) [2-[2-Amino-4-(isoindolin-2-carbonyl)chinazolin-6-yl]-4,5-difluor-phenyl]methyl-diethylphosphat;
Retentionszeit LC-MS: 2.00 min;
¹H NMR (500 MHz, DMSO-d₆/TFA-d₁) δ [ppm] 8.05 (d, J = 1.5, 1H), 7.99 (d, J = 8.6, 1 H), 7.82 (d, J = 8.6, 1 H), 7.52 (dd, J = 11.2, 8.1, 1 H), 7.40 - 7.32 (m, 2H), 7.25 (dt, J = 18.5, 7.2, 2H), 7.18 (d, J = 7.2, 1 H), 4.98 (s, 2H), 4.82 (s, 4H), 3.86 - 3.75 (m, 4H), 1.06 (t, J = 7.1, 6H).

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| Verbindung Nr. | Name und/oder Struktur |
|---|---|
| "A6" | [2-Amino-6-[4,5-difluor-2-(hydroxymethyl)phenyl]chinazolin-4-yl]-isoindolin-2-yl-methanon |
| "A8" | [2-Amino-6-[2-(2-dimethylaminoethoxymethyl)-4,5-difluor-phenyl]chinazolin-4-yl]-isoindolin-2-yl-methanon |
| "A9" | [2-Amino-6-[2-(3-dimethylaminopropoxymethyl)-4,5-difluor-phenyl]chinazolin-4-yl]-isoindolin-2-yl-methanon |
| "A44" | {2-Amino-6-[2-(methylethylamino-methyl)-4,5-difluor-phenyl]-chinazolin-4-yl}-(1,3-dihydro-isoindol-2-yl)-methanon |
| "A45" | {2-Amino-6-[2-(diethylamino-methyl)-4,5-difluor-phenyl]-chinazolin-4-yl}-(1,3-dihydro-isoindol-2-yl)-methanon |
| "A46" | {2-Amino-6-[2-(tert.-butylamino-methyl)-4,5-difluor-phenyl]-chinazolin-4-yl}-(1,3-dihydro-isoindol-2-yl)-methanon |
| "A47" | (2-Amino-6-{2-[(tert.-butyl-methyl-amino)-methyl]-4,5-difluor-phenyl)-chinazolin-4-yl)-(1,3-dihydro-isoindol-2-yl)-methanon |
| "A48" | [2-Amino-6-(4,5-difluor-2-pyrrolidin-1-ylmethyl-phenyl)-chinazolin-4-yl]-(1,3-dihydro-isoindol-2-yl)-methanon |
| "A49" | {2-Amino-6-[4,5-difluor-2-(2-methyl-pyrrolidin-1-ylmethyl)-phenyl]-chinazolin-4-yl}-(1,3-dihydro-isoindol-2-yl)-methanon |
| "A50" | {2-Amino-6-[2-(2,5-dimethyl-pyrrolidin-1-ylmethyl)-4,5-difluor-phenyl]-chinazolin-4-yl}-(1,3-dihydro-isoindol-2-yl)-methanon |
| "A52" | {2-Amino-6-[4,5-difluor-2-(4-methyl-piperazin-1-ylmethyl)-phenyl]-chinazolin-4-yl}-(1,3-dihydro-isoindol-2-yl)-methanon |
| "A53" | {2-[2-Amino-4-(1,3-dihydro-isoindol-2-carbonyl)-chinazolin-6-yl]-4,5-difluor-benzyl}-bis-carbaminsäure-tert.-butylester |
| "A54" | [2-Amino-6-(2-aminomethyl-4,5-difluor-phenyl)-chinazolin-4-yl]-(1,3-dihydro-isoindol-2-yl)-methanon |
| "A55" | 3-Amino-N-{2-[2-amino-4-(1,3-dihydro-isoindol-2-carbonyl)-chinazolin-6-yl]-4,5-difluor-benzyl}-propionamid |
| "A56" | N-{2-[2-Amino-4-(1,3-dihydro-isoindol-2-carbonyl)-chinazolin-6-yl]-4,5-difluor-benzyl}-3-dimethylamino-propionamid |
| "A59" | {2-Amino-6-[4,5-difluor-2-(2-hydroxy-ethoxymethyl)-phenyl]-chinazolin-4-yl}-(1,3-dihydro-isoindol-2-yl)-methanon |
| "A60" | [2-Amino-6-(4,5-difluor-2-{2-[2-(2-hydroxy-ethoxy)-ethoxy]-ethoxymethyl}-phenyl)-chinazolin-4-yl]-(1,3-dihydro-isoindol-2-yl)-methanon |
| "A61" | (2-Amino-6-{4,5-difluor-2-[2-(2-hydroxy-ethoxy)-ethoxymethyl]-phenyl}-chinazolin-4-yl)-(1,3-dihydro-isoindol-2-yl)-methanon |
| "A62" | {2-Amino-6-[4,5-difluor-2-(2-{2-[2-(2-hydroxy-ethoxy)-ethoxy]-ethoxy}-ethoxymethyl)-phenyl]-chinazolin-4-yl}-(1,3-dihydro-isoindol-2-yl)-methanon |
| "A63" | [2-Amino-6-(2-aminoxymethyl-4,5-difluor-phenyl)-chinazolin-4-yl]-(1,3-dihydro-isoindol-2-yl)-methanon |
| "A64" | [2-Amino-6-(4,5-difluor-2-hydroxymethyl-phenyl)-chinazolin-4-yl]-(1,3-dihydro-isoindol-2-yl)-methanon |
| "A65" | 3-Amino-propionsäure-2-[2-amino-4-(1,3-dihydro-isoindol-2-carbonyl)-chinazolin-6-yl]-4,5-difluor-benzylester |
| "A66" | (S)-2,5-Diamino-pentansäure-2-[2-amino-4-(1,3-dihydro-isoindol-2-carbonyl)-chinazolin-6-yl]-4,5-difluor-benzylester |
| "A67" | 3-Dimethylamino-propionsäure-2-[2-amino-4-(1,3-dihydro-isoindol-2-carbonyl)-chinazolin-6-yl]-4,5-difluor-benzylester |
| "A68" | (S)-2,6-Bis-tert.-butoxycarbonylamino-hexansäure-2-[2-amino-4-(1,3-dihydro-isoindol-2-carbonyl)-chinazolin-6-yl]-4,5-difluor-benzylester |
| "A70a" | (S)-2-tert.-Butoxycarbonylamino-6-dimethylamino-hexansäure-2-[2-amino-4-(1,3-dihydro-isoindol-2-carbonyl)-quinazolin-6-yl]-4,5-difluoro-benzylester |
| "A71" | (S)-2,6-Diamino-hexansäure-2-[2-amino-4-(1,3-dihydro-isoindol-2-carbonyl)-chinazolin-6-yl]-4,5-difluor-benzylester |
| "A73a" | (S)-2-Amino-6-dimethylamino-hexansäure-2-[2-amino-4-(1,3-dihydro-isoindol-2-carbonyl)-chinazolin-6-yl]-4,5-difluor-benzylester |
| "A74" | 2-Amino-4-methyl-pentansäure-2-[2-amino-4-(1,3-dihydro-isoindol-2-carbonyl)-chinazolin-6-yl]-4,5-difluorbenzylester |
| "A75" | (S)-2-tert.-Butoxycarbonylamino-propionsäure-2-[2-amino-4-(1,3-dihydro-isoindol-2-carbonyl)-chinazolin-6-yl]-4,5-difluor-benzylester |
| "A76" | (S)-2-Amino-propionsäure-2-[2-amino-4-(1,3-dihydro-isoindol-2-carbonyl)-chinazolin-6-yl]-4,5-difluor-benzylester |
| "A77" | 2,2-Dimethyl-propionsäure-2-[2-amino-4-(1,3-dihydro-isoindol-2-carbonyl)-chinazolin-6-yl]-4,5-difluor-benzylester |
| "A78" | Isobuttersäure-2-[2-amino-4-(1,3-dihydro-isoindol-2-carbonyl)-chinazolin-6-yl]-4,5-difluor-benzylester |
| "A79" | Propionsäure-2-[2-amino-4-(1,3-dihydro-isoindol-2-carbonyl)-chinazolin-6-yl]-4,5-difluor-benzylester |
| "A80" | 2-tert.-Butoxycarbonylamino-2-methyl-propionsäure-2-[2-amino-4-(1,3-dihydro-isoindol-2-carbonyl)-chinazolin-6-yl]-4,5-difluor-benzylester |
| "A81" | 2-Amino-2-methyl-propionsäure-2-[2-amino-4-(1,3-dihydro-isoindol-2-carbonyl)-chinazolin-6-yl]-4,5-difluor-benzylester |
| "A82" | Essigsäure-2-[2-amino-4-(1,3-dihydro-isoindol-2-carbonyl)-chinazolin-6-yl]-4,5-difluor-benzylester |
| "A83" | 6-tert.-Butoxycarbonylamino-hexansäure-2-[2-amino-4-(1,3-dihydro-isoindol-2-carbonyl)-chinazolin-6-yl]-4,5-difluor-benzylester |
| "A84" | 6-Amino-hexansäure-2-[2-amino-4-(1,3-dihydro-isoindol-2-carbonyl)-chinazolin-6-yl]-4,5-difluor-benzylester |
| "A85" | 5-tert.-Butoxycarbonylamino-pentansäure-2-[2-amino-4-(1,3-dihydro-isoindol-2-carbonyl)-chinazolin-6-yl]-4,5-difluor-benzylester |
| "A86" | 5-Amino-pentansäure-2-[2-amino-4-(1,3-dihydro-isoindol-2-carbonyl)-chinazolin-6-yl]-4,5-difluor-benzylester |
| "A87" | (S)-Pyrrolidin-1,2-dicarbonsäure-2-{2-[2-amino-4-(1,3-dihydro-isoindol-2-carbonyl)-chinazolin-6-yl]-4,5-difluor-benzyl}-ester-1-tert.-butylester |
| "A88" | (S)-Pyrrolidin-2-carbonsäure-2-[2-amino-4-(1,3-dihydro-isoindol-2-carbonyl)-chinazolin-6-yl]-4,5-difluor-benzylester |
| "A89" | 4-tert.-Butoxycarbonylamino-buttersäure-2-[2-amino-4-(1,3-dihydro-isoindol-2-carbonyl)-chinazolin-6-yl]-4,5-difluor-benzylester |
| "A90" | 4-Amino-buttersäure-2-[2-amino-4-(1,3-dihydro-isoindol-2-carbonyl)-chinazolin-6-yl]-4,5-difluor-benzylester Dihydrochlorid |
| "A91" | 3-(tert.-Butoxycarbonyl-methyl-amino)-propionsäure-2-[2-amino-4-(1,3-dihydro-isoindol-2-carbonyl)-chinazolin-6-yl]-4,5-difluor-benzylester |
| "A92" | 3-Methylamino-propionsäure-2-[2-amino-4-(1,3-dihydro-isoindol-2-carbonyl)-chinazolin-6-yl]-4,5-difluor-benzylester |
| "A93" | (R)-3-tert.-Butoxy-2-tert.-butoxycarbonylamino-propionsäure-2-[2-amino-4-(1,3-dihydro-isoindol-2-carbonyl)-chinazolin-6-yl]-4,5-difluor-benzylester |
| "A96" | (R)-2-Amino-3-hydroxy-propionsäure-2-[2-amino-4-(1,3-dihydro-isoindol-2-carbonyl)-chinazolin-6-yl]-4,5-difluor-benzylester |
| "A105" | (tert.-Butoxycarbonyl-methyl-amino)-essigsäure-2-[2-amino-4-(1,3-dihydro-isoindol-2-carbonyl)-chinazolin-6-yl]-4,5-difluor-benzylester |
| "A108" | Methyl-aminoessigsäure-2-[2-amino-4-(1,3-dihydro-isoindol-2-carbonyl)-chinazolin-6-yl]-4,5-difluor-benzylester |
| "A112" | Dimethyl-aminoessigsäure-2-[2-amino-4-(1,3-dihydro-isoindol-2-carbonyl)-chinazolin-6-yl]-4,5-difluor-benzylester |
| "A114" | Dimethyl-carbaminsäure-2-[2-amino-4-(1,3-dihydro-isoindol-2-carbonyl)-chinazolin-6-yl]-4,5-difluor-benzylester |
| "A115" | (2-Dimethylamino-ethyl)-carbaminsäure-2-[2-amino-4-(1,3-dihydro-isoindol-2-carbonyl)-chinazolin-6-yl]-4,5-difluor-benzylester |
| "A116" | 4-Methyl-piperazin-1-carbonsäure-2-[2-amino-4-(1,3-dihydro-isoindol-2-carbonyl)-chinazolin-6-yl]-4,5-difluor-benzylester |
| "A117" | (2-Amino-ethyl)-carbaminsäure-2-[2-amino-4-(1,3-dihydro-isoindol-2-carbonyl)-chinazolin-6-yl]-4,5-difluor-benzylester |
| "A118" | Carbonsäure-2-[2-amino-4-(1,3-dihydro-isoindol-2-carbonyl)-chinazolin-6-yl]-4,5-difluor-benzylester-2-tert.-butoxycarbonylamino-ethylester |
| "A119" | Carbonsäure-2-[2-amino-4-(1,3-dihydro-isoindol-2-carbonyl)-chinazolin-6-yl]-4,5-difluor-benzylester-2-amino-ethylester |
| "A120" | Carbonsäure-2-[2-amino-4-(1,3-dihydro-isoindol-2-carbonyl)-chinazolin-6-yl]-4,5-difluor-benzylester-2-dimethylamino-ethylester |
| "A121" | [2-[2-Amino-4-(isoindolin-2-carbonyl)chinazolin-6-yl]-4,5-difluor-phenyl]methyl-di-tert.-butylphosphat |
| "A122" | [2-[2-Amino-4-(isoindolin-2-carbonyl)chinazolin-6-yl]-4,5-difluor-phenyl]methyl dihydrogenphosphat |
| "A123" | 2-[2-Amino-4-(isoindolin-2-carbonyl)chinazolin-6-yl]-4,5-difluor-phenyl]methyl diethylphosphat |
sowie ihre pharmazeutisch verwendbaren Salze, Tautomere und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Tautomere und Stereoisomere, einschließlich deren Mischungen in allen Verhältnissen, sowie gegebenenfalls Träger- und/oder Hilfsstoffe.

3. Verbindungen gemäß Anspruch 1, sowie ihrer pharmazeutisch verwendbaren Salze, Tautomere und Stereoisomere, einschließlich deren Mischungen in allen Verhältnissen, zur Verwendung zur Behandlung oder Vorbeugung von Tumorerkrankungen, viralen Erkrankungen, zur Immunsuppression bei Transplantationen, entzündungsbedingten Erkrankungen, Zystische Fibrose, Erkrankungen im Zusammenhang mit Angiogenese, infektiösen Erkrankungen, Autoimmunerkrankungen, Ischämie, fibrogenetischen Erkrankungen, zur Förderung der Nervenregeneration,
zur Hemmung des Wachstums von Krebs, Tumorzellen und Tumormetastasen,
zum Schutz normaler Zellen gegen Toxizität, die durch Chemotherapie verursacht ist,
zur Behandlung von Krankheiten, wobei Proteinfehlfaltung oder Aggregation ein Hauptkausalfaktor ist.

4. Arzneimittel enthaltend mindestens eine Verbindung gemäß Anspruch 1 und/oder ihre pharmazeutisch verwendbaren Salze, Tautomere und Stereoisomere, einschließlich deren Mischungen in allen Verhältnissen, und mindestens einen weiteren Arzneimittelwirkstoff.

5. Set (Kit), bestehend aus getrennten Packungen von
(a) einer wirksamen Menge an einer Verbindung gemäß Anspruch 1 und/oder ihrer pharmazeutisch verwendbaren Salze, Tautomere und Stereoisomere, einschließlich deren Mischungen in allen Verhältnissen, und
(b) einer wirksamen Menge eines weiteren Arzneimittelswirkstoffs.

## Claims

1. Compounds selected from the group
| Compound No. | Name and/or structure |
|---|---|
| "A6" | [2-Amino-6-[4,5-difluoro-2-(hydroxymethyl)phenyl]-quinazolin-4-yl]isoindolin-2-ylmethanone |
| "A8" | [2-Amino-6-[2-(2-dimethylaminoethoxymethyl)-4,5-difluorophenyl]quinazolin-4-yl]isoindo]in-2-ylmethanone |
| "A9" | [2-Amino-6-[2-(3-dimethylaminopropoxymethyl)-4,5-difluorophenyl]quinazolin-4-yl]isoindolin-2-ylmethanone |
| "A44" | {2-Amino-6-[2-(methylethylaminomethyl)-4,5-difluoro-phenyl]quinazolin-4-yl}-(1,3-dihydroisoindol-2-yl)methanone |
| "A45" | {2-Amino-6-[2-(diethylaminomethyl)-4,5-difluorophenyl]-quinazolin-4-yl}-(1,3-dihydroisoindol-2-yl)methanone |
| "A46" | {2-Amino-6-[2-(tert-butylaminomethyl)-4,5-difluorophenyl]-quinazolin-4-yl}-(1,3-dihydroisoindol-2-yl)methanone |
| "A47" | (2-Amino-6-{2-[(tert-butylmethylamino)methyl]-4,5-difluoro-phenyl}quinazolin-4-yl)-(1,3-dihydroisoindol-2-yl)-methanone |
| "A48" | [2-Amino-6-(4,5-difluoro-2-pyrrolidin-1-ylmethylphenyl)-quinazolin-4-yl]-(1,3-dihydroisoindol-2-yl)methanone |
| "A49" | {2-Amino-6-[4,5-difluoro-2-(2-methylpyrrolidin-1-ylmethyl)-phenyl]quinazolin-4-yl}-(1,3-dihydroisoindol-2-yl)methanone |
| "A50" | {2-Amino-6-[2-(2,5-dimethylpyrrolidin-1-ylmethyl)-4,5-difluorophenyl]quinazolin-4-yl}-(1,3-dihydroisoindol-2-yl)-methanone |
| "A52" | {2-Amino-6-[4,5-difluoro-2-(4-methylpiperazin-1-ylmethyl)-phenyl]quinazolin-4-yl}-(1,3-dihydroisoindol-2-yl)methanone |
| "A53" | tert-Butyl {2-[2-amino-4-(1,3-dihydroisoindole-2-carbonyl)-quinazolin-6-yl]-4,5-difluorobenzyl}bis-carbamate |
| "A54" | [2-Amino-6-(2-aminomethyl-4,5-difluorophenyl)quinazolin-4-yl]-(1,3-dihydroisoindol-2-yl)methanone |
| "A55" | 3-Amino-N-{2-[2-amino-4-(1,3-dihydroisoindole-2-carbonyl)quinazolin-6-yl]-4,5-difluorobenzyl}propionamide |
| "A56" | N-{2-[2-Amino-4-(1,3-dihydroisoindole-2-carbonyl)-quinazolin-6-yl]-4,5-difluorobenzyl}-3-dimethylamino-propionamide |
| "A59" | {2-Amino-6-[4,5-difluoro-2-(2-hydroxyethoxymethyl)phenyl]-quinazolin-4-yl}-(1,3-dihydroisoindol-2-yl)methanone |
| "A60" | [2-Amino-6-(4,5-difluoro-2-{2-[2-(2-hydroxyethoxy)ethoxy]-ethoxymethyl}phenyl)quinazolin-4-yl]-(1,3-dihydroisoindol-2-yl)methanone |
| "A61" | (2-Amino-6-{4,5-difluoro-2-[2-(2-hydroxyethoxy)ethoxy-methyl]phenyl}quinazolin-4-yl)-(1,3-dihydroisoindol-2-yl)-methanone |
| "A62" | {2-Amino-6-[4,5-difluoro-2-(2-{2-[2-(2-hydroxyethoxy)-ethoxy]ethoxy}ethoxymethyl)phenyl]quinazolin-4-yl}-(1,3-dihydroisoindol-2-yl)methanone |
| "A63" | [2-Amino-6-(2-aminoxymethyl-4,5-difluorophenyl)-quinazolin-4-yl]-(1,3-dihydroisoindol-2-yl)methanone |
| "A64" | [2-Amino-6-(4,5-difluoro-2-hydroxymethylphenyl)quinazolin-4-yl]-(1,3-dihydroisoindol-2-yl)methanone |
| "A65" | 2-[2-Amino-4-(1,3-dihydroisoindole-2-carbonyl)quinazolin-6-yl]-4,5-difluorobenzyl 3-aminopropionate |
| "A66" | 2-[2-Amino-4-(1,3-dihydroisoindole-2-carbonyl)quinazolin-6-yl]-4,5-difluorobenzyl (S)-2,5-diaminopentanoate |
| "A67" | 2-[2-Amino-4-(1,3-dihydroisoindole-2-carbonyl)quinazolin-6-yl]-4,5-difluorobenzyl 3-dimethylaminopropionate |
| "A68" | 2-[2-Amino-4-(1,3-dihydroisoindole-2-carbonyl)quinazolin-6-yl]-4,5-difluorobenzyl (S)-2,6-bis-tert-butoxycarbonyl-aminohexanoate |
| "A70a" | 2-[2-Amino-4-(1,3-dihydroisoindole-2-carbonyl)quinazolin-6-yl]-4,5-difluorobenzyl (S)-2-tert-butoxycarbonylamino-6-dimethylaminohexanoate |
| "A71" | 2-[2-Amino-4-(1,3-dihydroisoindole-2-carbonyl)quinazolin-6-yl]-4,5-difluorobenzyl (S)-2,6-diaminohexanoate |
| "A73a" | 2-[2-Amino-4-(1,3-dihydroisoindole-2-carbonyl)quinazolin-6-yl]-4,5-difluorobenzyl (S)-2-amino-6-dimethylaminohexanoate |
| "A74" | 2-[2-Amino-4-(1,3-dihydroisoindole-2-carbonyl)quinazolin-6-yl]-4,5-difluorobenzyl 2-amino-4-methylpentanoate |
| "A75" | 2-[2-Amino-4-(1,3-dihydroisoindole-2-carbonyl)quinazolin-6-yl]-4,5-difluorobenzyl (S)-2-tert-butoxycarbonylamino-propionate |
| "A76" | 2-[2-Amino-4-(1,3-dihydroisoindole-2-carbonyl)quinazolin-6-yl]-4,5-difluorobenzyl (S)-2-aminopropionate |
| "A77" | 2-[2-Amino-4-(1,3-dihydroisoindole-2-carbonyl)quinazolin-6-yl]-4,5-difluorobenzyl 2,2-dimethylpropionate |
| "A78" | 2-[2-Amino-4-(1,3-dihydroisoindole-2-carbonyl)quinazolin-6-yl]-4,5-difluorobenzyl isobutyrate |
| "A79" | 2-[2-Amino-4-(1,3-dihydroisoindole-2-carbonyl)quinazolin-6-yl]-4,5-difluorobenzyl propionate |
| "A80" | 2-[2-Amino-4-(1,3-dihydroisoindole-2-carbonyl)quinazolin-6-yl]-4,5-difluorobenzyl 2-tert-butoxycarbonylamino-2-methylpropionate |
| "A81" | 2-[2-Amino-4-(1,3-dihydroisoindole-2-carbonyl)quinazolin-6-yl]-4,5-difluorobenzyl 2-amino-2-methylpropionate |
| "A82" | 2-[2-Amino-4-(1,3-dihydroisoindole-2-carbonyl)quinazolin-6-yl]-4,5-difluorobenzyl acetate |
| "A83" | 2-[2-Amino-4-(1,3-dihydroisoindole-2-carbonyl)quinazolin-6-yl]-4,5-difluorobenzyl 6-tert-butoxycarbonylamino-hexanoate |
| "A84" | 2-[2-Amino-4-(1,3-dihydroisoindole-2-carbonyl)quinazolin-6-yl]-4,5-difluorobenzyl 6-aminohexanoate |
| "A85" | 2-[2-Amino-4-(1,3-dihydroisoindole-2-carbonyl)quinazolin-6-yl]-4,5-difluorobenzyl 5-tert-butoxycarbonylamino-pentanoate |
| "A86" | 2-[2-Amino-4-(1,3-dihydroisoindole-2-carbonyl)quinazolin-6-yl]-4,5-difluorobenzyl 5-aminopentanoate |
| "A87" | 2-{2-[2-Amino-4-(1,3-dihydroisoindole-2-carbonyl)-quinazolin-6-yl]-4,5-difluorobenzyl} 1-tert-butyl (S)-pyrrolidine-1,2-dicarboxylate |
| "A88" | 2-[2-Amino-4-(1,3-dihydroisoindole-2-carbonyl)quinazolin-6-yl]-4,5-difluorobenzyl (S)-pyrrolidine-2-carboxylate |
| "A89" | 2-[2-Amino-4-(1,3-dihydroisoindole-2-carbonyl)quinazolin-6-yl]-4,5-difluorobenzyl 4-tert-butoxycarbonylaminobutyrate |
| "A90" | 2-[2-Amino-4-(1,3-dihydroisoindole-2-carbonyl)quinazolin-6-yl]-4,5-difluorobenzyl 4-aminobutyrate dihydrochloride |
| "A91" | 2-[2-Amino-4-(1,3-dihydroisoindole-2-carbonyl)quinazolin-6-yl]-4,5-difluorobenzyl 3-(tert-butoxycarbonylmethyl-amino)propionate |
| "A92" | 2-[2-Amino-4-(1,3-dihydroisoindole-2-carbonyl)quinazolin-6-yl]-4,5-difluorobenzyl 3-methylaminopropionate |
| "A93" | 2-[2-Amino-4-(1,3-dihydroisoindole-2-carbonyl)quinazolin-6-yl]-4,5-difluorobenzyl (R)-3-tert-butoxy-2-tert-butoxy-carbonylaminopropionate |
| "A96" | 2-[2-Amino-4-(1,3-dihydroisoindole-2-carbonyl)quinazolin-6-yl]-4,5-difluorobenzyl (R)-2-amino-3-hydroxypropionate |
| "A105" | 2-[2-Amino-4-(1,3-dihydroisoindole-2-carbonyl)quinazolin-6-yl]-4,5-difluorobenzyl (tert-butoxycarbonylmethyl-amino)acetate |
| "A108" | 2-[2-Amino-4-(1,3-dihydroisoindole-2-carbonyl)quinazolin-6-yl]-4,5-difluorobenzyl methylaminoacetate |
| "A112" | 2-[2-Amino-4-(1,3-dihydroisoindole-2-carbonyl)quinazolin-6-yl]-4,5-difluorobenzyl dimethylaminoacetate |
| "A114" | 2-[2-Amino-4-(1,3-dihydroisoindole-2-carbonyl)quinazolin-6-yl]-4,5-difluorobenzyl dimethylcarbamate |
| "A115" | 2-[2-Amino-4-(1,3-dihydroisoindole-2-carbonyl)quinazolin-6-yl]-4,5-difluorobenzyl (2-dimethylaminoethyl)carbamate |
| "A116" | 2-[2-Amino-4-(1,3-dihydroisoindole-2-carbonyl)quinazolin-6-yl]-4,5-difluorobenzyl 4-methylpiperazine-1-carboxylate |
| "A117" | 2-[2-Amino-4-(1,3-dihydroisoindole-2-carbonyl)quinazolin-6-yl]-4,5-difluorobenzyl (2-aminoethyl)carbamate |
| "A118" | 2-[2-Amino-4-(1,3-dihydroisoindole-2-carbonyl)quinazolin-6-yl]-4,5-difluorobenzyl 2-tert-butoxycarbonylaminoethyl carbonate |
| "A119" | 2-[2-Amino-4-(1,3-dihydroisoindole-2-carbonyl)quinazolin-6-yl]-4,5-difluorobenzyl 2-aminoethyl carbonate |
| "A120" | 2-[2-Amino-4-(1,3-dihydroisoindole-2-carbonyl)quinazolin-6-yl]-4,5-difluorobenzyl 2-dimethylaminoethyl carbonate |
| "A121" | [2-[2-Amino-4-(isoindoline-2-carbonyl)quinazolin-6-yl]-4,5-difluorophenyl]methyl di-tert-butyl phosphate |
| "A122" | [2-[2-Amino-4-(isoindoline-2-carbonyl)quinazolin-6-yl]-4,5-difluorophenyl]methyl dihydrogenphosphate |
| "A123" | [2-[2-Amino-4-(isoindoline-2-carbonyl)quinazolin-6-yl]-4,5-difluorophenyl]methyl diethyl phosphate |
and pharmaceutically usable salts, tautomers and stereoisomers thereof, including mixtures thereof in all ratios.

2. Medicaments comprising at least one compound according to Claim 1 and/or pharmaceutically usable salts, tautomers and stereoisomers thereof, including mixtures thereof in all ratios, and optionally excipients and/or adjuvants.

3. Compounds according to Claim 1, and pharmaceutically usable salts, tautomers and stereoisomers thereof, including mixtures thereof in all ratios, for use for the treatment or prevention of tumour diseases, viral diseases, for immunosuppression in transplants, inflammation-induced diseases, cystic fibrosis, diseases associated with angio-genesis, infectious diseases, autoimmune diseases, ischaemia, fibro-genetic diseases,
for the promotion of nerve regeneration,
for inhibiting the growth of cancer, tumour cells and tumour metastases,
for the protection of normal cells against toxicity caused by chemotherapy,
for the treatment of diseases in which incorrect protein folding or aggregation is a principal causal factor.

4. Medicaments comprising at least one compound according to Claim 1 and/or pharmaceutically usable salts, tautomers and stereoisomers thereof, including mixtures thereof in all ratios, and at least one further medicament active compound.

5. Set (kit) consisting of separate packs of
(a) an effective amount of a compound according to Claim 1 and/or pharmaceutically usable salts, tautomers and stereoisomers thereof, including mixtures thereof in all ratios,
and
(b) an effective amount of a further medicament active compound.

## Revendications

1. Composés choisis dans le groupe constitué par
| Composé n° | Nom et/ou structure |
|---|---|
| "A6" | [2-Amino-6-[4,5-difluoro-2-(hydroxyméthyl)phényl]-quinazolin-4-yl]isoindolin-2-ylméthanone |
| "A8" | [2-Amino-6-[2-(2-diméthylaminoéthoxyméthyl)-4,5-difluoro-phényl]quinazolin-4-yl]isoindolin-2-ylméthanone |
| "A9" | [2-Amino-6-[2-(3-diméthylaminopropoxyméthyl)-4,5-difluorophényl]quinazolin-4-yl]isoindolin-2-ylméthanone |
| "A44" | {2-Amino-6-[2-(méthyléthylaminométhyl)-4,5-difluoro-phényl]quinazolin-4-yl}-(1,3-dihydroisoindol-2-yl)méthanone |
| "A45" | {2-Amino-6-[2-(diéthylaminométhyl)-4,5-difluorophényl]-quinazolin-4-yl}-(1,3-dihydroisoindol-2-yl)méthanone |
| "A46" | {2-Amino-6-[2-(tertio-butylaminométhyl)-4,5-difluorophényl]-quinazolin-4-yl}-(1,3-dihydroisoindol-2-yl)méthanone |
| "A47" | (2-Amino-6-{2-[(tertio-butylméthylamino)méthyl]-4,5-difluorophényl}quinazolin-4-yl)-(1,3-dihydroisoindol-2-yl)-méthanone |
| "A48" | [2-Amino-6-(4,5-difluoro-2-pyrrolidin-1-ylméthylphényl)-quinazolin-4-yl]-(1,3-dihydroisoindol-2-yl)méthanone |
| "A49" | {2-Amino-6-[4,5-difluoro-2-(2-méthylpyrrolidin-1-ylméthyl)-phényl]quinazolin-4-yl}-(1,3-dihydroisoindol-2-yl)méthanone |
| "A50" | {2-Amino-6-[2-(2,5-diméthylpyrrolidin-1-ylméthyl)-4,5-difluorophényl]quinazolin-4-yl}-(1,3-dihydroisoindol-2-yl)-méthanone |
| "A52" | {2-Amino-6-[4,5-difluoro-2-(4-méthylpipérazin-1-ylméthyl)-phényl]quinazolin-4-yl}-(1,3-dihydroisoindol-2-yl)méthanone |
| "A53" | Acide {2-[2-amino-4-(1,3-dihydroisoindole-2-carbonyl)-quinazolin-6-yl]-4,5-difluorobenzyl}biscarbamique tertio-butylester |
| "A54" | [2-Amino-6-(2-aminométhyl-4,5-difluorophényl)quinazolin-4-yl]-(1,3-dihydroisoindol-2-yl)méthanone |
| "A55" | 3-Amino-N-{2-[2-amino-4-(1,3-dihydroisoindole-2-carbonyl)quinazolin-6-yl]-4,5-difluorobenzyl}propionamide. |
| "A56" | N-{2-[2-Amino-4-(1,3-dihydroisoindole-2-carbonyl)-quinazolin-6-yl]-4,5-difluorobenzyl}-3-diméthylamino-propionamide |
| "A59" | {2-Amino-6-[4,5-difluoro-2-(2-hydroxyéthoxyméthyl)phényl]-quinazolin-4-yl}-(1,3-dihydroisoindol-2-yl)méthanone |
| "A60" | [2-Amino-6-(4,5-difluoro-2-{2-[2-(2-hydroxyéthoxy)éthoxy]-éthoxyméthyl}phényl)quinazolin-4-yl]-(1,3-dihydroisoindol-2-yl)méthanone |
| "A61" | (2-Amino-6-{4,5-difluoro-2-[2-(2-hydroxyéthoxy)éthoxy-méthyl]phényl}quinazolin-4-yl)-(1,3-dihydroisoindol-2-yl)-méthanone |
| "A62" | {2-Amino-6-[4,5-difluoro-2-(2-{2-[2-(2-hydroxyéthoxy)-éthoxy]éthoxy}éthoxyméthyl)phényl]quinazolin-4-yl}-(1,3-dihydroisoindol-2-yl)méthanone |
| "A63" | [2-Amino-6-(2-aminoxyméthyl-4,5-difluorophényl)-quinazolin-4-yl]-(1,3-dihydroisoindol-2-yl)méthanone |
| "A64" | [2-Amino-6-(4,5-difluoro-2-hydroxyméthylphényl)quinazolin-4-yl]-(1,3-dihydroisoindol-2-yl)méthanone |
| "A65" | Acide 3-aminopropionique 2-[2-amino-4-(1,3-dihydroisoindole-2-carbonyl)quinazolin-6-yl]-4,5-difluorobenzylester |
| "A66" | Acide (S)-2,5-diaminopentanoique 2-[2-amino-4-(1,3-dihydroisoindole-2-carbonyl)quinazolin-6-yl]-4,5-difluorobenzylester |
| "A67" | Acide 3-diméthylaminopropionique 2-[2-amino-4-(1,3-dihydroisoindole-2-carbonyl)quinazolin-6-yl]-4,5-difluorobenzylester |
| "A68" | Acide (S)-2,6-bis-tertio-butoxycarbonylaminohexanoique 2-[2-amino-4-(1,3-dihydroisoindole-2-carbonyl)quinazolin-6-yl]-4,5-difluorobenzylester |
| "A70a" | Acide (S)-2-tertio-butoxycarbonylamino-6-diméthylaminohexanoique 2-[2-amino-4-(1,3-dihydroisoindole-2-carbonyl)quinazolin-6-yl]-4,5-difluorobenzylester |
| "A71" | Acide (S)-2,6-diaminohexanoique 2-[2-amino-4-(1,3-dihydroisoindole-2-carbonyl)quinazolin-6-yl]-4,5-difluorobenzylester |
| "A73a" | Acide (S)-2-amino-6-diméthylaminohexanoique 2-[2-amino-4-(1,3-dihydroisoindole-2-carbonyl)quinazolin-6-yl]-4,5-diftuorobenzylester |
| "A74" | Acide 2-amino-4-méthylpentanoique 2-[2-amino-4-(1,3-dihydroisoindole-2-carbonyl)quinazolin-6-yl]-4,5-difluorobenzylester |
| "A75" | Acide (S)-2-tertio-butoxycarbonylaminopropionique 2-[2-amino-4-(1,3-dihydroisoindole-2-carbonyl)quinazolin-6-yl]-4,5-difluorobenzylester |
| "A76" | Acide (S)-2-aminopropionique 2-[2-amino-4-(1,3-dihydroisoindole-2-carbonyl)quinazolin-6-yl]-4,5-difluorobenzylester |
| "A77" | Acide 2,2-diméthylpropionique 2-[2-amino-4-(1,3-dihydroisoindole-2-carbonyl)quinazolin-6-yl]-4,5-difluorobenzylester |
| "A78" | Acide isobutyrique 2-[2-amino-4-(1,3-dihydroisoindole-2-carbonyl)quinazolin-6-yl]-4,5-difluorobenzylester |
| "A79" | Acide propionique 2-[2-amino-4-(1,3-dihydroisoindole-2-carbonyl)quinazolin-6-yl]-4,5-difluorobenzylester |
| "A80" | Acide 2-tertio-butoxycarbonylamino-2-méthylpropionique 2-[2-amino-4-(1,3-dihydroisoindole-2-carbonyl)quinazolin-6-yl]-4,5-difluorobenzylester |
| "A81" | Acide 2-amino-2-méthylpropionique 2-[2-amino-4-(1,3-dihydroisoindole-2-carbonyl)quinazolin-6-yl]-4,5-difluorobenzylester |
| "A82" | Acide acétique 2-[2-amino-4-(1,3-dihydroisoindole-2-carbonyl)quinazolin-6-yl]-4,5-difluorobenzylester |
| "A83" | Acide 6-tertio-butoxycarbonylaminohexanoique 2-[2-amino-4-(1,3-dihydroisoindole-2-carbonyl)quinazolin-6-yl]-4,5-difluorobenzylester |
| "A84" | Acide 6-aminohexanoique 2-[2-amino-4-(1,3-dihydroisoindole-2-carbonyl)quinazolin-6-yl]-4,5-difluoro-benzylester |
| "A85" | Acide 5-tertio-butoxycarbonylaminopentanoique 2-[2-amino-4-(1,3-dihydroisoindole-2-carbonyl)quinazolin-6-yl]-4,5-difluorobenzylester |
| "A86" | Acide 5-aminopentanoique 2-[2-amino-4-(1,3-dihydroisoindole-2-carbonyl)quinazolin-6-yl]-4,5-difluorobenzylester |
| "A87" | Acide (S)-pyrrolidine-1,2-dicarboxylique 2-{2-[2-amino-4-(1,3-dihydroisoindole-2-carbonyl)quinazolin-6-yl]-4,5-difluorobenzylester} 1-tertio-butylester |
| "A88" | Acide (S)-pyrrolidine-2-carboxylique 2-[2-amino-4-(1,3-dihydroisoindole-2-carbonyl)quinazolin-6-yl]-4,5-difluorobenzylester |
| "A89" | Acide 4-tertio-butoxycarbonylaminobutyrique 2-[2-amino-4-(1,3-dihydroisoindole-2-carbonyl)quinazolin-6-yl]-4,5-difluorobenzylester |
| "A90" | Acide 4-aminobutyrique 2-[2-amino-4-(1,3-dihydroisoindole-2-carbonyl)quinazolin-6-yl]-4,5-difluorobenzylester, dichlorhydrate |
| "A91" | Acide 3-(tertio-butoxycarbonylméthylamino)propionique 2-[2-amino-4-(1,3-dihydroisoindole-2-carbonyl)quinazolin-6-yl]-4,5-difluorobenzylester |
| "A92" | Acide 3-méthylaminopropionique 2-[2-amino-4-(1,3-dihydroisoindole-2-carbonyl)quinazolin-6-yl]-4,5-difluorobenzylester |
| "A93" | Acide (R)-3-tertio-butoxy-2-tertio-butoxycarbonylaminopropionique 2-[2-amino-4-(1,3-dihydroisoindole-2-carbonyl)quinazolin-6-yl]-4,5-difluorobenzylester |
| "A96" | Acide (R)-2-amino-3-hydroxypropionique 2-[2-amino-4-(1,3-dihydroisoindole-2-carbonyl)quinazolin-6-yl]-4,5-difluorobenzylester |
| "A105" | Acide (tertio-butoxycarbonylméthylamino)acétique 2-[2-amino-4-(1,3-dihydroisoindole-2-carbonyl)quinazolin-6-yl]-4,5-difluorobenzylester |
| "A108" | Acide méthylaminoacétique 2-[2-amino-4-(1,3-dihydroisoindole-2-carbonyl)quinazolin-6-yl]-4,5-difluorobenzylester |
| "A112" | Acide diméthylaminoacétique 2-[2-Amino-4-(1,3-dihydroisoindole-2-carbonyl)quinazolin-6-yl]-4,5-difluorobenzylester |
| "A114" | Acide diméthylcarbamique 2-[2-amino-4-(1,3-dihydroisoindole-2-carbonyl)quinazolin-6-yl]-4,5-difluorobenzylester |
| "A115" | Acide (2-diméthylaminoéthyl)carbamique 2-[2-amino-4-(1,3-dihydroisoindole-2-carbonyl)quinazolin-6-yl]-4,5-difluorobenzylester |
| "A116" | Acide 4-méthylpipérazine-1-carboxylique 2 -[2-amino-4-(1,3-dihydroisoindole-2-carbonyl)quinazolin-6-yl]-4,5-difluorobenzylester |
| "A117" | Acide (2-aminoéthyl)carbamique 2-[2-amino-4-(1,3-dihydroisoindole-2-carbonyl)quinazolin-6-yl]-4,5-difluorobenzylester |
| "A118" | Acide carbonique 2-[2-amino-4-(1,3-dihydroisoindole-2-carbonyl)quinazolin-6-yl]-4,5-difluorobenzylester 2-tertiobutoxycarbonylaminoéthylester |
| "A119" | Acide carbonique 2-[2-amino-4-(1,3-dihydroisoindole-2-carbonyl)quinazolin-6-yl]-4,5-difluorobenzylester 2-aminoéthylester |
| "A120" | Acide carbonique 2-[2-amino-4-(1,3-dihydroisoindole-2-carbonyl)quinazolin-6-yl]-4,5-difluorobenzylester 2-diméthylaminoéthylester |
| "A121" | Phosphate de [2-[2-amino-4-(isoindoline-2-carbonyl)-quinazolin-6-yl]-4,5-difluorophényl]méthyle et de di-tertiobutyle |
| "A122" | Dihydrogénophosphate de [2-[2-Amino-4-(isoindoline-2-carbonyl)quinazolin-6-yl]-4,5-difluorophényl]méthyle |
| "A123" | Phosphate de [2-[2-amino-4-(isoindoline-2-carbonyl)-quinazolin-6-yl]-4,5-difluorophényl]méthyle et de diéthyle |
et les sels, tautomères et stéréoisomères pharmaceutiquement utilisables de ceux-ci, y compris des mélanges de ceux-ci en toutes proportions.

2. Médicaments comprenant au moins un composé selon la revendication 1 et/ou des sels, tautomères et stéréoisomères pharmaceutiquement utilisables de celui-ci, y compris des mélanges de ceux-ci en toutes proportions, et éventuellement des excipients et/ou des adjuvants.

3. Composés selon la revendication 1, et des sels, tautomères et stéréoisomères pharmaceutiquement utilisables de ceux-ci, y compris des mélanges de ceux-ci en toutes proportions, pour une utilisation dans le traitement ou la prévention de maladies tumorales, de maladies virales, pour la suppression immunitaire dans le cas des greffes, de maladies induites par des inflammations, de la fibrose kystique, de maladies associées à l'angiogenèse, de maladies infectieuses, de maladies auto-immunes, de l'ischémie, de maladies fibrosantes, pour la promotion de la régénération nerveuse,
pour l'inhibition de la croissance de cancers, de cellules tumorales et de métastases tumorales,
pour la protection de cellules normales contre une toxicité provoquée par une chimiothérapie,
pour le traitement de maladies dans lesquelles un repliement protéique incorrect ou une agrégation constitue un facteur causal principal.

4. Médicaments comprenant au moins un composé selon la revendication 1 et/ou des sels, tautomères et stéréoisomères pharmaceutiquement utilisables de celui-ci, y compris des mélanges de ceux-ci en toutes proportions, et au moins un autre composé actif médicamenteux.

5. Ensemble (kit) constitué de conditionnements séparés
(a) d'une quantité efficace d'un composé selon la revendication 1 et/ou de sels, tautomères et stéréoisomères pharmaceutiquement utilisables de celui-ci, y compris des mélanges de ceux-ci en toutes proportions,
et
(b) d'une quantité efficace d'un autre composé actif médicamenteux.
